# EUROPÄISCHE PATENTSCHRIFT

(11) **EP 1 562 935 B1**
(45) Veröffentlichungstag und Bekanntmachung des Hinweises auf die Patenterteilung: **06.09.2006**
(21) Anmeldenummer: 03769398.3
(22) Anmeldetag: 16.10.2003
(51) Int. Cl.: C07D 401/14, C07D 413/14, C07D 413/12, C07D 471/04, C07D 405/14, C07D 403/14, C07D 519/00, A61K 31/506, A61P 9/00

(54) **HETEROARYLOXY-SUBSTITUIERTE PHENYLAMINOPYRIMIDINE ALS RHO-KINASEINHIBITOREN**
HETEROARYLOXY-SUBSTITUTED PHENYLAMINOPYRIMIDINES AS RHO-KINASE INHIBITORS
PHENYLAMINOPYRIMIDINE SUBSTITUEE PAR HETEROARYLOXY ET UTILISEE EN TANT QU'INHIBITEUR DE KINASE

(30) Priorität: 28.10.2002 DE 10250113; 16.07.2003 DE 10332232
(43) Veröffentlichungstag der Anmeldung: 17.08.2005
(73) Patentinhaber: Bayer HealthCare AG, 51368 Leverkusen (DE)
(72) Erfinder: FEURER, Achim, 69259 Wilhelmsfeld (DE); BENNABI, Samir, 42103 Wuppertal (DE); HECKROTH, Heike, 42113 Wuppertal (DE); SCHIROK, Hartmut, 44329 Dortmund (DE); MITTENDORF, Joachim, 42113 Wuppertal (DE); KAST, Raimund, 42349 Wuppertal (DE); STASCH, Johannes-Peter, 42651 Solingen (DE); GNOTH, Mark, Jean, 42115 Wuppertal (DE); MÜNTER, Klaus, 42489 Wülfrath (DE); LANG, Dieter, 42553 Velbert (DE); FIGUEROA PEREZ, Santiago, 51373 Leverkusen (DE); EHMKE, Heimo, 22301 Hamburg (DE)
(86) Internationale Anmeldenummer: PCT/EP2003/011452
(87) Internationale Veröffentlichungsnummer: WO 2004/039796

(56) Entgegenhaltungen:
- WO-A-01/60816
- WO-A-01/64654
- FUKATA YUKO ET AL: "Rho-Rho-kinase pathway in smooth muscle contraction and cytoskeletal reorganization of non-muscle cells." TRENDS IN PHARMACOLOGICAL SCIENCES, Bd. 22, Nr. 1, Januar 2001 (2001-01), Seiten 32-39, XP002267445 & ISSN: 0165-6147 in der Anmeldung erwähnt

## Beschreibung

Die Erfindung betrifft heteroaryloxy-substituierte Phenylaminopyrimidine, ein Verfahren zu ihrer Herstellung sowie ihre Verwendung zur Herstellung von Arzneimitteln zur Behandlung und/oder Prophylaxe von Krankheiten bei Menschen und Tieren, insbesondere von kardiovaskulären Erkrankungen.

Ein Anstieg der intrazellulären Calcium-Konzentration ist ein Hauptauslöser für die Kontraktion der Gefäßmuskulatur (Somlyo, A.P. und Himpens, B. *FASEB J*. 1989, 3, 2266-2276). Dies geschieht in erster Linie durch Agonisten wie z.B. Phenylephrin oder Thromboxan A2, die nach Stimulierung der Phosphatidylinositolkaskade die Freisetzung von Calcium aus dem sarkoplasmatischen Retikulum bewirken. Die Erhöhung des intrazellulären Calciums aktiviert die MLC-Kinase (Myosin-Leichte-Ketten-Kinase), die die MLC-Untereinheiten des Myosinmoleküls phosphoryliert (Kamm, K.H. und Stull, J.T., *Annu. Rev. Pharmacol.Toxicol*. 1985, 25, 593-603). MLC-Phosphorylierung induziert die Glattmuskelkontraktion, MLC-Dephosphorylierung nach einer Reduktion der intrazellulären Calciumkonzentration resultiert in der Relaxation des Gefäßes.

Neben der Calcium-abhängigen MLC-Phosphorylierung existiert noch ein weiterer zentraler aber Calcium-unabhängiger Regulationsmechanismus des Gefäßtonus. Hierbei handelt es sich um den Rho/Rho-Kinase-Signalweg (Noda, M. et al., *FEBS Lett.* 1995, 367, 246-250; Uehata, M. et al., *Nature* 1997, 389, 990-994; Fukata, Y. et al., *Trends in Pharmacological Sciences* 2001, 22, 32-39). Binden Agonisten wie z.B. Phenylephrin oder Thromboxan A2 an ihre Rezeptoren, so führt dies zur Aktivierung der kleinen G-Proteine Rho, die dann mit der Rho-Kinase interagieren und diese aktivieren. Die aktivierte Rho-Kinase inhibiert die Myosin-Phosphatase, nachdem sie eine Untereinheit des Enzyms phosphoryliert hat. Gleichzeitig phosphoryliert Rho-Kinase MLC an der Stelle, die auch von der MLC-Kinase phosphoryliert wird. Eine Hemmung der Myosin-Phosphatase sowie der Phosphorylierung von MLC induziert die Kontraktion der Gefäßmuskulatur. Im Gegensatz dazu führt eine Hemmung der Rho-Kinase zu einer Gefäßrelaxation. Inhibitoren der Rho-Kinase bewirken daher eine Senkung des Blutdruckes und eine Steigerung des koronaren Blutflusses.

Darüber hinaus führen Inhibitoren der Rho-Kinase zu einer Hemmung des Wachstums von Tumorzellen und Metastasen (Itoh et al. *Nat. Med*. 1999, 5, 221; Somlyo et al. *Biochem. Biophys. Res. Commun*. 2000, 269, 652) und inhibieren die Angiogenese (Uchida et al. *Biochem. Biophys. Res. Commun*. 2000, 269, 633; Gingras et al. *Biochem. J*. 2000, 348 Bd. 2, 273).

Strukturell ähnliche Verbindungen sind in anderen Indikationen bzw. für andere Wirkmechanismen bekannt. So beschreiben beispielsweise US 3 478 030 und US 3 432 493 substituierte Aminopyrimidine, die den koronaren Blutfluss steigern können, dabei aber als Carboanhydrase-Inhibitoren wirken (*J. Chem. Inf. Comp. Sciences* 2002, 42, 94-102). Andere Pyrimidin-Derivate sind als Anti-Krebs- und Anti-HIV-Mittel (Debi, M.; *Indian J. Exp. Biol*. 1997, 35, 1208-1213) oder als cdk2-Inhibitoren (WO-A 01/64654) beschrieben.

Aufgabe der vorliegenden Erfindung ist die Bereitstellung von Arzneimitteln für die Behandlung von Erkrankungen, insbesondere von kardiovaskulären Erkrankungen.

Gegenstand der vorliegenden Erfindung sind Verbindungen der Formel (I) 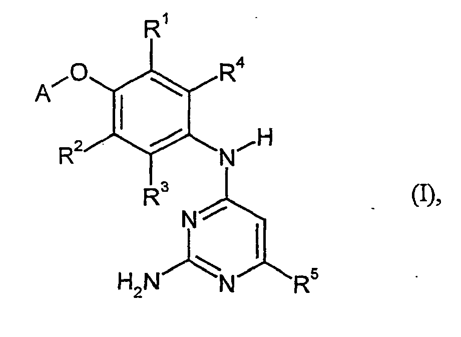 worin
- A: einen Rest 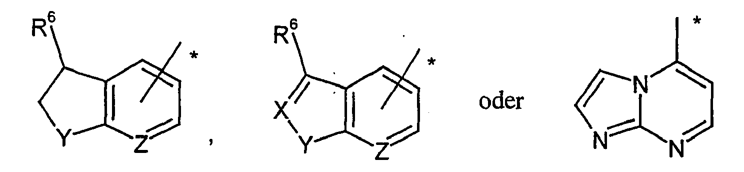 bedeutet,
worin
X für N oder C-H steht,
Y für N-R⁷, O oder S steht,
worin
R⁷ für Wasserstoff, Benzyl, Phenyl, (C₁-C₆)-Alkyl oder (C₃-C₈)-Cycloalkyl steht,
wobei Alkyl und Cycloalkyl ihrerseits durch Fluor, Hydroxy, Amino, Carboxyl, (C₁-C₆)-Alkoxy, (C₁-C₆)-Alkylamino oder Morpholinyl substituiert sein können,
Z für N oder C-H steht,
R⁶ für Wasserstoff, Halogen, Trifluormethyl, (C₁-C₆)-Alkylamino oder W-R⁷ steht,
worin
W für NH, O oder eine Bindung steht,
R⁷ die oben angegebene Bedeutung hat und
* für die Anknüpfstelle an den phenolischen Sauerstoff steht,
- R¹ und R²: unabhängig voneinander Wasserstoff, Halogen oder Cyano bedeuten,
- R³ und R⁴: unabhängig voneinander Wasserstoff, Fluor oder Chlor bedeuten,
- R⁵: einen Rest bedeutet, der ausgewählt ist aus der Gruppe von:
Wasserstoff, Hydroxy, Halogen, Trifluormethyl,
(C₃-C₈)-Cycloalkyl, (C₁-C₆)-Alkyl, (C₁-C₆)-Alkoxy,
   wobei Cycloalkyl, Alkyl und Alkoxy ihrerseits durch Hydroxy, Carboxyl, (C₁-C₆)-Alkoxy, (C₁-C₆)-Alkoxycarbonyl, (C₆-C₁₀)-Aryl, NR⁸R⁹ oder C(=O)NR⁸R⁹ substituiert sein können,
   worin
   R⁸ und R⁹ unabhängig voneinander für Wasserstoff, (C₁-C₈)-Alkyl, gegebenenfalls durch (C₁-C₆)-Alkyl substituiertes (C₃-C₆)-Cycloalkyl, gegebenenfalls durch Halogen substituiertes (C₆-C₁₀)-Aryl oder 5- bis 10-gliedriges Heteroaryl stehen oder
   R⁸ und R⁹ gemeinsam mit dem Stickstoffatom, an das sie gebunden sind, einen 5- oder 6-gliedrigen Heterocyclus bilden, der ein weiteres Heteroatom O oder N im Ring enthalten kann und der durch (C₁-C₆)-Alkyl, (C₁-C₆)-Alkanoyl oder (C₁-C₆)-Alkoxycarbonyl substituiert sein kann,
(C₆-C₁₀)-Aryl, (C₆-C₁₀)-Aryloxy, 5- bis 10-gliedriges Heteroaryl, 5- bis 10-gliedriges Heteroaryloxy, über ein Kohlenstoffatom gebundenes 5- bis 10-gliedriges Heterocyclyl,
   wobei Aryl, Aryloxy, Heteroaryl, Heteroaryloxy und Heterocyclyl ihrerseits durch Halogen, Cyano, Nitro, Carboxyl, Amino, Trifluor-methyl, gegebenenfalls durch Hydroxy substituiertes (C₁-C₆)-Alkyl, (C₁-C₆)-Alkoxy, (C₁-C₆)-Alkylamino, (C₁-C₆)-Alkanoyl, (C₁-C₆)-Alkoxycarbonyl, (C₁-C₆)-Alkanoylamino, (C₁-C₆)-Alkoxycarbonyl-amino oder 5- oder 6-gliedriges Heterocyclyl substituiert sein können,
NR¹⁰R¹¹,
   worin
   R¹⁰ und R¹¹ unabhängig voneinander für Wasserstoff, (C₁-C₆)-Alkyl, (C₃-C₈)-Cycloalkyl, (C₆-C₁₀)-Aryl oder 5- bis 10-gliedriges Heteroaryl stehen,
   wobei Alkyl und Cycloalkyl ihrerseits durch Hydroxy, (C₁-C₆)-Alkoxy, (C₆-C₁₀)-Aryl, 5- bis 10-gliedriges Heteroaryl oder NR¹⁵R¹⁶ substituiert sein können,
   worin
   R¹⁵ und R¹⁶ unabhängig voneinander für Wasserstoff, (C₁-C₆)-Alkyl, (C₃-C₆)-Cycloalkyl, (C₆-C₁₀)-Aryl oder 5- oder 6-gliedriges Heteroaryl stehen oder
   R¹⁵ und R¹⁶ gemeinsam mit dem Stickstoffatom, an das sie gebunden sind, einen 5- oder 6-gliedrigen Heterocyclus bilden, der ein weiteres Heteroatom O oder N im Ring enthalten kann und der durch (C₁-C₆)-Alkyl, (C₁-C₆)-Alkanoyl oder (C₁-C₆)-Alkoxycarbonyl substituiert sein kann, und
   Aryl und Heteroaryl ihrerseits durch Halogen, Hydroxy, Amino, Cyano, Trifluormethyl, (C₁-C₆)-Alkyl, (C₁-C₆)-Alkoxy, (C₁-C₆)-Alkylamino oder (C₁-C₆)-Alkanoylamino substituiert sein können, oder
   R¹⁰ und R¹¹ gemeinsam mit dem Stickstoffatom, an das sie gebunden sind, einen 4- bis 6-gliedrigen Heterocyclus bilden, der ein weiteres Heteroatom O oder N im Ring enthalten kann und der durch Fluor, Hydroxy, Carboxyl, 5- bis 7-gliedriges Heterocyclyl, das ein oder zwei weitere Heteroatome N und/oder O im Ring enthalten kann und das seinerseits durch (C₁-C₄)-Alkyl oder (C₁-C₄)-Alkoxycarbonyl substituiert sein kann, (C₁₋C₄)-Alkoxy, gegebenenfalls durch Hydroxy, (C₁-C₄)-Alkoxy oder NR¹⁷R¹⁸ substituiertes (C₁-C₄)-Alkyl, (C₁-C₄)-Alkanoyl, (C₁-C₄)-Alkoxycarbonyl oder NR¹²R¹³ substituiert sein kann,
   wobei
   R¹² und R¹³ unabhängig voneinander für Wasserstoff, (C₁-C₆)-Alkyl, (C₁-C₄)-Alkoxycarbonyl, (C₃-C₈)-Cycloalkyl oder (C₁-C₄)-Alkanoyl stehen oder
   R¹² und R¹³ gemeinsam mit dem Stickstoffatom, an das sie gebunden sind, einen 5- oder 6-gliedrigen Heterocyclus bilden, der ein weiteres Heteroatom O oder N im Ring enthalten kann und der durch (C₁-C₆)-Alkyl, (C₁-C₆)-Alkanoyl oder (C₁-C₆)-Alkoxycarbonyl substituiert sein kann, und
   R¹⁷ und R¹⁸ unabhängig voneinander für Wasserstoff, gegebenenfalls durch Hydroxy substituiertes (C₁-C₆)-Alkyl, (C₃-C₆)-Cycloalkyl, (C₆-C₁₀)-Aryl oder 5- oder 6-gliedriges Heteroaryl stehen oder
   R¹⁷ und R¹⁸ gemeinsam mit dem Stickstoffatom, an das sie gebunden sind, einen 5- oder 6-gliedrigen Heterocyclus bilden, der ein weiteres Heteroatom O oder N im Ring enthalten kann und der durch (C₁-C₆)-Alkyl, (C₁-C₆)-Alkanoyl oder (C₁-C₆)-Alkoxycarbonyl substituiert sein kann, oder
   R¹⁰ und R¹¹ gemeinsam mit dem Stickstoffatom, an das sie gebunden sind, einen 7- bis 12-gliedrigen bicyclischen oder tricyclischen Heterocyclus bilden, der anelliert oder spirocyclisch ist und ein oder zwei weitere Heteroatome aus der Reihe N und/oder O im Ring aufweisen kann und der durch Fluor, (C₁-C₄)-Alkyl, (C₁₋C₄)-Alkoxycarbonyl, (C₁-C₄)-Alkanoyl oder Benzyl substituiert sein kann,
und C(=O)R¹⁴,
   worin
   R¹⁴ für (C₁-C₆)-Alkoxy, (C₁-C₆)-Alkylamino oder einen über ein Stickstoffatom gebundenen 5- bis 10-gliedrigen mono- oder bicyclischen Heterocyclus, der anelliert oder spirocyclisch ist und ein oder zwei weitere Heteroatome aus der Reihe N und/oder O im Ring aufweisen kann, steht,
   wobei Alkylamino seinerseits durch einen 5- oder 6-gliedrigen Heterocyclyl substituiert sein kann,
und ihre Salze, Hydrate, Hydrate der Salze und Solvate.

Erfindungsgemäße Verbindungen sind die Verbindungen der Formel (I) und deren Salze, Solvate und Solvate der Salze; die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Die Erfindung betrifft in Abhängigkeit von der Struktur der Verbindungen auch Tautomere der Verbindungen.

Als Salze sind im Rahmen der Erfindung physiologisch unbedenkliche Salze der erfindungsgemäßen Verbindungen bevorzugt.

Physiologisch unbedenkliche Salze der Verbindungen (I) umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ehansulfonsäure, Toluolsulfonsäure, Benzolsulfonsäure, Naphthalindisulfonsäure, Essigsäure, Propionsäure, Milchsäure, Weinsäure, Äpfelsäure, Zitronensäure, Fumarsäure, Maleinsäure, Trifluoressigsäure und Benzoesäure.

Physiologisch unbedenkliche Salze der Verbindungen (I) umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclo-hexylamin, Dimethylaminoethanol, Prokain, Dibenzylamin, N-Methylmorpholin, Dihydroabiethylamin, Arginin, Lysin, Ethylendiamin und Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl per se und "Alk" und "Alkyl" in Alkoxy, Alkanoyl, Alkylamino, Alkoxycarbonyl, Alkoxycarbonylamino und Alkanoylamino stehen für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, tert.-Butyl, n-Pentyl und n-Hexyl.

Alkoxy steht beispielhaft und vorzugsweise für Methoxy, Ethoxy, n-Propoxy, Isopropoxy, tert.-Butoxy, n-Pentoxy und n-Hexoxy.

Alkanoyl steht beispielhaft und vorzugsweise für Acetyl und Propanoyl.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, tert.-Butylamino, n-Pentylamino, n-Hexylamino, *N,N*-Dimethylamino, *N,N*-Diethylamino, *N,N*-Diisopropylamino, N-Ethyl-*N*-methylamino, *N*-Methyl-*N*-n-propylamino, *N*-Isopropyl-*N*-n-propylamino, *N-t-*Butyl-*N*-methylamino, *N*-Ethyl-*N*-n-pentylamino und *N*-n-Hexyl-*N*-methylamino. C₁₋C₄-Alkylamino steht beispielsweise für einen Monoalkylaminorest mit 1 bis 4 Kohlenstoffatomen oder für einen Dialkylaminorest mit jeweils 1 bis 4 Kohlenstoffatomen pro Alkylsubstituent.

Alkoxycarbonyl steht beispielhaft und vorzugsweise für Methoxycarbonyl, Ethoxycarbonyl, n-Propoxycarbonyl, Isopropoxycarbonyl, tert.-Butoxycarbonyl, n-Pentoxycarbonyl und n-Hexoxycarbonyl.

Alkoxycarbonylamino steht beispielhaft und vorzugsweise für Methoxycarbonylamino, Ethoxycarbonylamino, n-Propoxycarbonylamino, Isopropoxycarbonylamino, tert.-Butoxycarbonylamino, n-Pentoxycarbonylamino und n-Hexoxycarbonylamino.

Alkanoylamino steht beispielhaft und vorzugsweise für Acetylamino und Ethylcarbonylamino.

Cycloalkyl steht für eine Cycloalkylgruppe mit in der Regel 3 bis 8, bevorzugt 5 bis 7 Kohlenstoffatomen, beispielhaft und vorzugsweise für Cyclopropyl, Cyclobutyl, Cyclopentyl, Cyclohexyl und Cycloheptyl.

Aryl per se und in Aryloxy steht für einen mono- bis tricyclischen aromatischen, carbocyclischen Rest mit in der Regel 6 bis 14 Kohlenstoffatomen; beispielhaft und vorzugsweise für Phenyl, Naphthyl und Phenanthrenyl.

Aryloxy steht beispielhaft und vorzugsweise für Phenyloxy und Naphthyloxy.

Heteroaryl per se und in Heteroaryloxy steht für einen aromatischen, mono- oder bicyclischen Rest mit in der Regel 5 bis 10, vorzugsweise 5 bis 6 Ringatomen und bis zu 5, vorzugsweise bis zu 4 Heteroatomen aus der Reihe S, O und N, beispielhaft und vorzugsweise für Thienyl, Furyl, Pyrrolyl, Thiazolyl, Oxazolyl, Imidazolyl, Pyridyl, Pyrimidyl, Pyridazinyl, Indolyl, Indazolyl, Benzofuranyl, Benzothiophenyl, Chinolinyl, Isochinolinyl.

Heteroaryloxy steht beispielhaft und vorzugsweise für Pyridyloxy, Pyrimidyloxy, Indolyloxy, Indazolyloxy.

Heterocyclyl und Heterocyclus steht für einen mono- oder polycyclischen, vorzugsweise mono- oder bicyclischen, nicht-aromatischen heterocyclischen Rest mit 4 bis 10, in der Regel 5 bis 8, vorzugsweise 5 oder 6 Ringatomen und bis zu 3, vorzugsweise bis zu 2 Heteroatomen und/oder Heterogruppen aus der Reihe N, O, S, SO, SO₂. Die Heterocyclyl-Reste können gesättigt oder teilweise ungesättigt sein. Bevorzugt sind 5- oder 6-gliedrige, monocyclische gesättigte Heterocyclylreste mit bis zu zwei Heteroatomen aus der Reihe O, N und S, wie beispielhaft und vorzugsweise Tetrahydrofuran-2-yl, Tetrahydrothienyl, Pyrrolidin-2-yl, Pyrrolidin-3-yl, Pyrrolinyl, Pyranyl, Piperidin-1-yl, Piperidin-2-yl, Piperidin-3-yl, Piperidin-4-yl, Thiopyranyl, Morpholin-1-yl, Morpholin-2-yl, Morpholin-3-yl, Perhydroazepinyl, Piperazin-1-yl, Piperazin-2-yl.

Halogen steht für Fluor, Chlor, Brom und Jod.

Wenn Reste in den erfindungsgemäßen Verbindungen substituiert sind, können die Reste, soweit nicht anders spezifiziert, ein- oder mehrfach gleich oder verschieden substituiert sein. Eine Substitution mit bis zu drei gleichen oder verschiedenen Substituenten ist bevorzugt. Ganz besonders bevorzugt ist die Substitution mit einem Substituenten.

Bevorzugt sind Verbindungen der Formel (I),
worin
- A: einen Rest 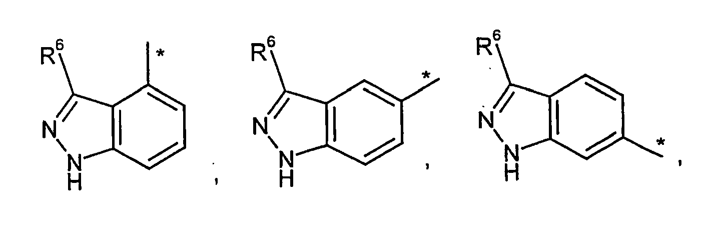 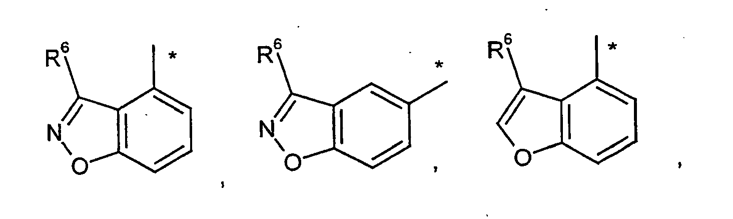 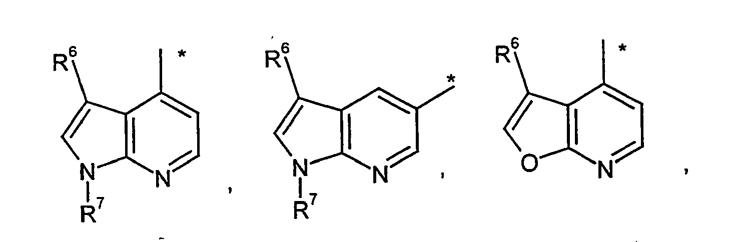 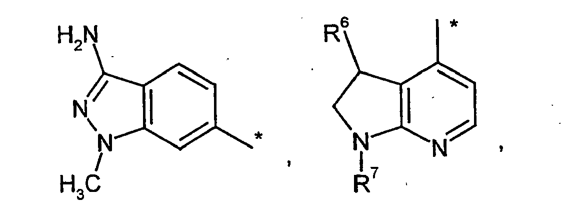 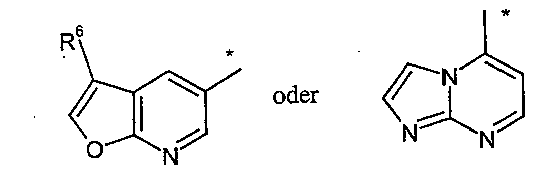 bedeutet,
worin
R⁶ für Wasserstoff, (C₁-C₄)-Alkyl oder NH-R⁷ steht,
R⁷ für Wasserstoff oder (C₁-C₄)-Alkyl steht und
* für die Anknüpfstelle an den phenolischen Sauerstoff steht,
- R¹ und R²: unabhängig voneinander Wasserstoff, Fluor oder Chlor bedeuten,
- R³ und R⁴: unabhängig voneinander Wasserstoff oder Fluor bedeuten,
- R⁵: einen Rest bedeutet, der ausgewählt ist aus der Gruppe von:
Wasserstoff, Chlor, (C₃-C₈)-Cycloalkyl, (C₁-C₆)-Alkyl, (C₁-C₆)-Alkoxy,
   wobei Alkyl und Alkoxy ihrerseits durch Hydroxy, Carboxyl, (C₁-C₄)-Alkoxy, (C₁-C₄)-Alkoxycarbonyl, NR⁸R⁹ oder C(=O)NR⁸R⁹ substituiert sein können,
   worin
   R⁸ und R⁹ unabhängig voneinander für Wasserstoff, (C₁-C₈)-Alkyl, gegebenenfalls durch (C₁-C₄)-Alkyl substituiertes (C₃-C₆)-Cycloalkyl, gegebenenfalls durch Halogen substituiertes Phenyl oder 5- oder 6-gliedriges Heteroaryl stehen oder
   R⁸ und R⁹ gemeinsam mit dem Stickstoffatom, an das sie gebunden sind, einen Morpholin-, Piperazin-, Piperidinoder Pyrrolidinring bilden, wobei die Ringe ihrerseits durch (C₁-C₄)-Alkyl substituiert sein können,
(C₆-C₁₀)-Aryl, 5- oder 6-gliedriges Heteroaryl, über ein Kohlenstoffatom gebundenes 5- oder 6-gliedriges Heterocyclyl,
   wobei Aryl, Heteroaryl und Heterocyclyl ihrerseits durch Halogen, Cyano, Nitro, Carboxyl, Amino, Trifluormethyl, gegebenenfalls durch Hydroxy substituiertes (C₁-C₄)-Alkyl, (C₁-C₄)-Alkoxy, (C₁-C₄)-Alkylamino, (C₁-C₄)-Alkanoyl, (C₁-C₄)-Alkoxycarbonyl, (C₁-C₄)-Alkanoylamino, (C₁-C₄)-Alkoxycarbonylamino oder 6-gliedriges Heterocyclyl substituiert sein können,
NR¹⁰R¹¹,
   worin
   R¹⁰ und R¹¹ unabhängig voneinander für Wasserstoff, (C₁-C₆)-Alkyl, (C₃-C₈)-Cycloalkyl, Phenyl oder 5- oder 6-gliedriges Heteroaryl stehen,
   wobei Alkyl und Cycloalkyl ihrerseits durch Hydroxy, (C₁-C₄)-Alkoxy, Phenyl, 5- oder 6-gliedriges Heteroaryl oder NR¹⁵R¹⁶ substituiert sein können,
   worin
   R¹⁵ und R¹⁶ unabhängig voneinander für Wasserstoff, (C₁-C₄)-Alkyl, (C₃-C₆)-Cycloalkyl, Phenyl oder 5- oder 6-gliedriges Heteroaryl stehen oder
   R¹⁵ und R¹⁶ gemeinsam mit dem Stickstoffatom, an das sie gebunden sind, einen Morpholin-, Piperazin-, Piperidin- oder Pyrrolidinring bilden, wobei die Ringe ihrerseits durch (C₁-C₄)-Alkyl substituiert sein können, und
   Phenyl und Heteroaryl ihrerseits durch Fluor, Chlor, Hydroxy, Amino, Cyano, Trifluormethyl, (C₁-C₄)-Alkyl, (C₁-C₄)-Alkoxy, (C₁-C₄)-Alkylamino oder (C₁-C₄)-Alkanoylamino substituiert sein können, oder
   R¹⁰ und R¹¹ gemeinsam mit dem Stickstoffatom, an das sie gebunden sind, einen 4- bis 6-gliedrigen Heterocyclus bilden, der ein weiteres Heteroatom O oder N im Ring enthalten kann und der durch Fluor, Hydroxy, Carboxyl, 5- bis 7-gliedriges Heterocyclyl, das ein oder zwei weitere Heteroatome N und/oder O im Ring enthalten kann, das seinerseits durch (C₁-C₄)-Alkyl oder (C₁-C₄)-Alkoxycarbonyl substituiert sein kann, (C₁-C₄)-Alkoxy, gegebenenfalls durch Hydroxy, (C₁-C₄)-Alkoxy oder NR¹⁷R¹⁸ substituiertes (C₁-C₄)-Alkyl, (C₁-C₄)-Alkanoyl, (C₁₋C₄)-Alkoxycarbonyl oder NR¹²R¹³ substituiert sein kann,
   wobei
   R¹² und R¹³ unabhängig voneinander für Wasserstoff oder (C₁₋C₄)-Alkyl stehen oder
   R¹² und R¹³ gemeinsam mit dem Stickstoffatom, an das sie gebunden sind, einen 5- oder 6-gliedrigen Heterocyclus bilden, der ein weiteres Heteroatom O oder N im Ring enthalten kann und der durch (C₁-C₆)-Alkyl, (C₁-C₆)-Alkanoyl oder (C₁-C₆)-Alkoxycarbonyl substituiert sein kann, und
   R¹⁷ und R¹⁸ unabhängig voneinander für Wasserstoff, gegebenenfalls durch Hydroxy substituiertes (C₁-C₄)-Alkyl oder Phenyl stehen oder
   R¹⁷ und R¹⁸ gemeinsam mit dem Stickstoffatom, an das sie gebunden sind, einen Pyrrolidinring bilden, oder
   R¹⁰ und R¹¹ gemeinsam mit dem Stickstoffatom, an das sie gebunden sind, einen 7- bis 12-gliedrigen bicyclischen oder tricyclischen Heterocyclus bilden, der anelliert oder spirocyclisch ist und ein oder zwei weitere Heteroatome aus der Reihe N und/oder O im Ring aufweisen kann und der durch (C₁-C₄)-Alkyl, (C₁-C₄)-Alkoxycarbonyl, (C₁-C₄)-Alkanoyl oder Benzyl substituiert sein kann,
und C(=O)R¹⁴,
   worin
   R¹⁴ für (C₁-C₆)-Alkoxy, (C₁-C₆)-Alkylamino oder einen über ein Stickstoffatom gebundenen 5- bis 10-gliedrigen mono- oder bicyclischen Heterocyclus, der anelliert oder spirocyclisch ist und ein oder zwei weitere Heteroatome aus der Reihe N und/oder O im Ring aufweisen kann, steht,
   wobei Alkylamino seinerseits durch einem 5- oder 6-gliedrigen Heterocyclyl substituiert sein kann,
und ihre Salze, Hydrate, Hydrate der Salze und Solvate.

Besonders bevorzugt sind Verbindungen der Formel (I),
worin
- A: einen Rest 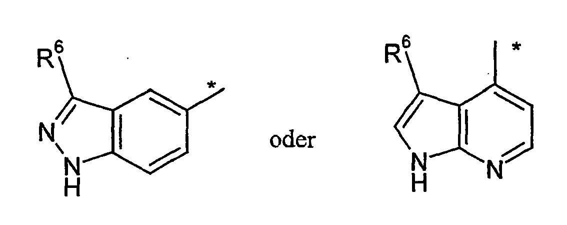 bedeutet,
worin
R⁶ für Wasserstoff oder Methyl steht und
* für die Anknüpfstelle an den phenolischen Sauerstoff steht,
- R¹ und R²: unabhängig voneinander Wasserstoff, Fluor oder Chlor bedeuten,
- R³ und R⁴: Wasserstoff bedeuten,
- R⁵: einen Rest bedeutet, der ausgewählt ist aus der Gruppe von:
Wasserstoff, Chlor, Cyclohexyl, (C₁-C₄)-Alkyl, (C₁-C₄)-Alkoxy,
   wobei Alkyl und Alkoxy ihrerseits durch Hydroxy, Carboxyl, (C₁-C₄)-Alkoxy; Methyloxycarbonyl, Ethyloxycarbonyl, NR⁸R⁹, oder C(=O)NR⁸R⁹ substituiert sein können,
   worin
   R⁸ und R⁹ unabhängig voneinander für Wasserstoff, (C₁-C₈)-Alkyl, Cyclopropyl, gegebenenfalls durch Methyl substituiertes Cyclopentyl oder gegebenenfalls durch Fluor substituiertes Phenyl stehen oder
   R⁸ und R⁹ gemeinsam mit dem Stickstoffatom, an das sie gebunden sind, einen Piperidin-, 2-Methylpiperidin-oder 2,6-Dimethylpiperidinring bilden,
Phenyl, Pyridyl, Pyrrolyl, Piperidin-3-yl, Piperidin-4-yl, Pyrrolidin-2-yl,
   wobei Phenyl, Pyridyl und Pyrrolyl ihrerseits durch Fluor, Chlor, Brom, Cyano, Nitro, Trifluormethyl, Methyl, Hydroxymethyl, Methoxy, Dimethylamino oder Morpholinyl substituiert sein können,
   und
   Piperidin-3-yl, Piperidin-4-yl und Pyrrolidin-2-yl ihrerseits durch Methyl, Ethyl, n-Propyl, iso-Propyl, Methylcarbonyl oder Ethyl-carbonyl substituiert sein können,
NR¹⁰R¹¹,
   worin
   R¹⁰ und R¹¹ unabhängig voneinander für Wasserstoff, (C₁-C₄)-Alkyl, 3-Hydroxypropyl, 2-Hydroxycyclohexyl, 2-Aminocyclohexyl, Phenyl, Pyridyl oder Pyrazolyl stehen,
   wobei Phenyl und Pyridyl ihrerseits durch Chlor, Hydroxy, Amino, Cyano, Methyl oder Methoxy substituiert sein können, oder
   R¹⁰ und R¹¹ gemeinsam mit dem Stickstoffatom, an das sie gebunden sind, einen Piperazin-, 3-Methylpiperazin-, 3,5-Dimethyl-piperazin-, 4-Isobutylpiperazin-, Morpholin-, Pyrrolidin-, 3-Aminopyrrolidin-, 3-Methylaminopyrrolidin-, 3-(*N,N-*Dimethylamino)-pyrrolidin-, 2-Aminomethylpyrrolidin-, 3-Hydroxypyrrolidin-, 2-Hydroxymethylpyrrolidin- oder 2-Methoxymethylpyrrolidinring oder einen Rest 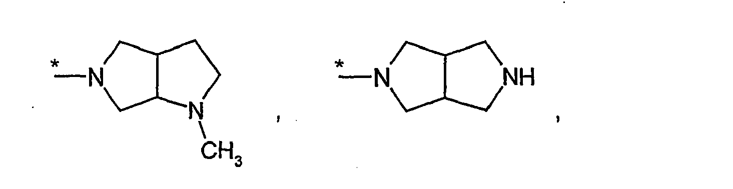 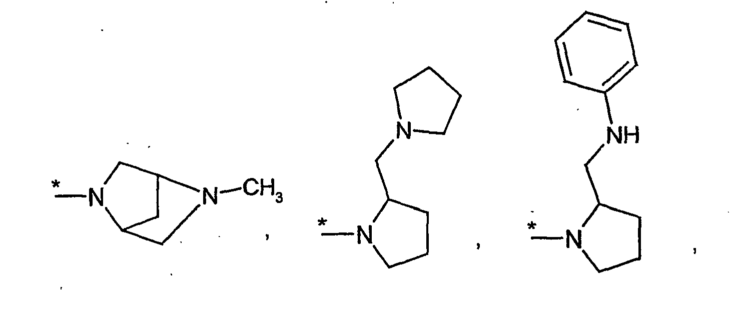 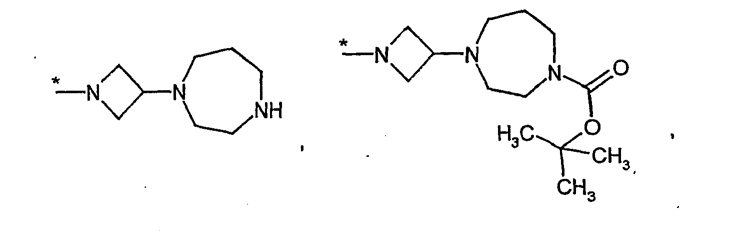 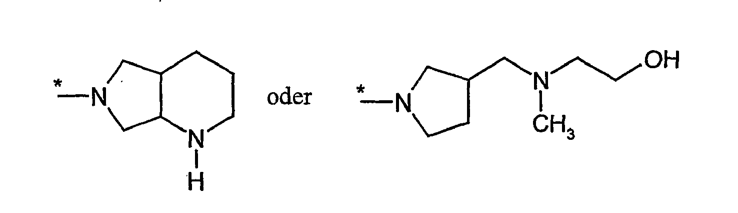 bilden,
   worin
   * für die Anknüpfstelle an den Pyrimidinring steht,
und C(=O)R¹⁴,
   worin
   R¹⁴ für Methoxy, Piperidinyl-N-ethylamino, Piperidinyl oder Piperazinyl steht,
und ihre Salze, Hydrate, Hydrate der Salze und Solvate.

Ebenfalls besonders bevorzugt sind Verbindungen der Formel (I), worin A einen Rest 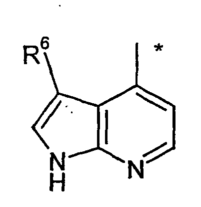 bedeutet,
worin
- R⁶: für Wasserstoff oder Methyl steht
und
- *: für die Anknüpfstelle an den phenolischen Sauerstoff steht.

Ebenfalls besonders bevorzugt sind Verbindungen der Formel (I), worin R¹ für Fluor steht.

Ebenfalls besonders bevorzugt sind Verbindungen der Formel (I), worin R² für Fluor oder Wasserstoff steht.

Ebenfalls besonders bevorzugt sind Verbindungen der Formel (I), worin R³ und R⁴ für Wasserstoff stehen.

Ganz besonders bevorzugt sind Kombinationen von zwei oder mehreren der oben genannten Vorzugsbereiche.

Gegenstand der vorliegenden Erfindung sind Verbindungen der Formel (I),
worin
- A: einen Rest 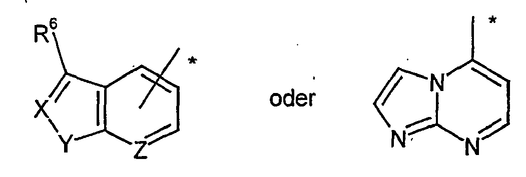 bedeutet
worin
X für N oder C-H steht,
Y für N-R⁷, O oder S steht,
worin
R⁷ für Wasserstoff, Benzyl, Phenyl, (C₁-C₆)-Alkyl oder (C₃-C₈)-Cycloalkyl steht,
wobei Alkyl und Cycloalkyl ihrerseits durch Fluor, Hydroxy, Amino, Carboxyl, (C₁-C₆)-Alkoxy, (C₁-C₆)-Alkylamino oder Morpholinyl substituiert sein können,
Z für N oder C-H steht,
R⁶ für Wasserstoff, Halogen, Trifluormethyl, (C₁-C₆)-Alkylamino oder W-R⁷ steht,
worin
W für NH, O oder eine Bindung steht,
R⁷ die oben angegebene Bedeutung hat und
* für die Anknüpfstelle an den phenolischen Sauerstoff steht,
- R¹ und R²: unabhängig voneinander Wasserstoff, Halogen oder Cyano bedeuten,
- R³ und R⁴: unabhängig voneinander Wasserstoff, Fluor oder Chlor bedeuten,
- R⁵: einen Rest bedeutet, der ausgewählt ist aus der Gruppe von:
Hydroxy, Halogen, Trifluormethyl,
(C₁-C₆)-Alkyl, (C₁-C₆)-Alkoxy, (C₃-C₈)-Cycloalkyl,
   wobei Alkyl, Alkoxy und Cycloalkyl ihrerseits durch Hydroxy, (C₁-C₆)-Alkoxy, (C₆-C₁₀)-Aryl oder NR⁸R⁹ substituiert sein können,
   worin
   R⁸ und R⁹ unabhängig voneinander für Wasserstoff, (C₁-C₆)-Alkyl oder 5- bis 10-gliedriges Heteroaryl stehen oder
   R⁸ und R⁹ gemeinsam mit dem Stickstoffatom, an das sie gebunden sind, einen 5- oder 6-gliedrigen Heterocyclus bilden, der ein weiteres Heteroatom O oder N im Ring enthalten kann und der durch (C₁-C₆)-Alkyl, (C₁-C₆)-Alkanoyl oder (C₁-C₆)-Alkoxy-carbonyl substituiert sein kann,
(C₆-C₁₀)-Aryl, (C₆-C₁₀)-Aryloxy, 5- bis 10-gliedriges Heteroaryl, 5- bis 10-gliedriges Heteroaryloxy,
   wobei Aryl, Aryloxy, Heteroaryl und Heteroaryloxy ihrerseits durch Halogen, Nitro, Carboxyl, Amino, Trifluormethyl, (C₁-C₆)-Alkyl, (C₁-C₆)-Alkoxy, (C₁-C₆)-Alkylamino, (C₁-C₆)-Alkanoyl, (C₁-C₆)-Alkoxycarbonyl, (C₁-C₆)-Alkanoylamino oder (C₁-C₆)-Alkoxy-carbonylamino substituiert sein können,
und NR¹⁰R¹¹,
   worin
   R¹⁰ und R¹¹ unabhängig voneinander für Wasserstoff, (C₁-C₆)-Alkyl, (C₃-C₈)-Cycloalkyl, (C₆-C₁₀)-Aryl oder 5- bis 10-gliedriges Heteroaryl stehen,
   wobei Alkyl und Cycloalkyl ihrerseits durch Hydroxy, (C₁-C₆)-Alkoxy, (C₆-C₁₀)-Aryl, 5- bis 10-gliedriges Heteroaryl oder NR⁸R⁹ substituiert sein können
   worin
   R⁸ und R⁹ die oben angegebene Bedeutung haben
   und
   Aryl und Heteroaryl ihrerseits durch Fluor, Chlor, Amino, Trifluormethyl, (C₁-C₆)-Alkyl (C₁-C₆)-Alkylamino oder (C₁-C₆)-Alkanoylamino substituiert sein können, oder
   R¹⁰ und R¹¹ gemeinsam mit dem Stickstoffatom, an das sie gebunden sind, einen 5- oder 6-gliedrigen Heterocyclus bilden, der durch Fluor, Carboxyl, 1,1-Dioxyethylen, 5- oder 6-gliedriges Heterocyclyl mit einem oder zwei Heteroatomen N und/oder O, das seinerseits durch (C₁-C₄)-Alkyl substituiert sein kann, (C₁-C₄)-Alkoxy, durch Hydroxy oder (C₁-C₄)-Alkoxy substituiertes (C₁-C₄)-Alkyl, (C₁-C₄)-Alkanoyl oder NR¹²R¹³ substituiert ist,
   worin
   R¹² und R¹³ unabhängig voneinander Wasserstoff, (C₁-C₆)-Alkyl, (C₁-C₄)-Alkoxycarbonyl, (C₃-C₈)-Cycloalkyl oder (C₁-C₄)-Alkanoyl bedeuten oder
   R¹² und R¹³ gemeinsam mit dem Stickstoffatom, an das sie gebunden sind, einen 5- oder 6-gliedrigen Heterocyclus bilden, der ein weiteres Heteroatom O oder N im Ring enthalten kann und der durch (C₁-C₆)-Alkyl, (C₁-C₆)-Alkanoyl oder (C₁-C₆)-Alkoxycarbonyl substituiert sein kann, oder
   R¹⁰ und R¹¹ gemeinsam mit dem Stickstoffatom, an das sie gebunden sind, einen 7- bis 12-gliedrigen bicyclischen Heterocyclus bilden, der anneliert oder spirocyclisch ist und ein oder zwei weitere Heteroatome aus der Reihe N und/oder O im Ring aufweisen kann und der durch Fluor, (C₁-C₄)-Alkyl, (C₁-C₄)-Alkoxycarbonyl, (C₁-C₄)-Alkanoyl oder Benzyl substituiert sein kann,
und ihre Salze, Hydrate, Hydrate der Salze und Solvate.

Bevorzugt sind Verbindungen der Formel (I),
worin
- A: einen Rest 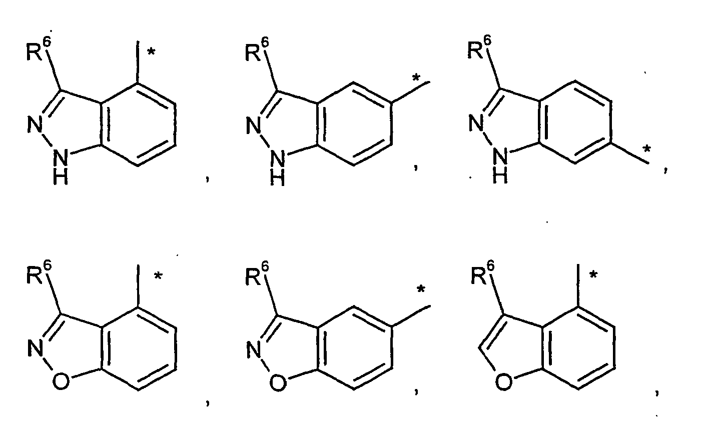 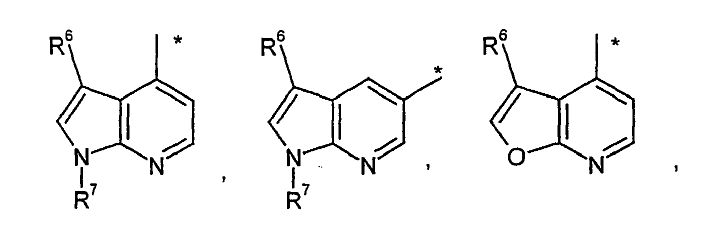 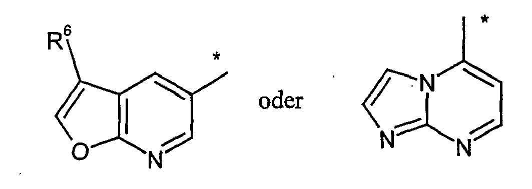 bedeutet,
worin
R⁶ für Wasserstoff, (C₁-C₄)-Alkyl, Hydroxy, Fluor, Chlor oder NH-R⁷ steht,
R⁷ für Wasserstoff oder (C₁-C₄)-Alkyl, das durch Fluor, Hydroxy, Methoxy, (C₁-C₄)-Alkylamino oder Morpholinyl substituiert sein kann, steht und
* für die Anknüpfstelle an den phenolischen Sauerstoff steht,
- R¹ und R²: unabhängig voneinander Wasserstoff, Fluor oder Chlor bedeuten,
- R³ und R⁴: Wasserstoff bedeuten,
- R⁵: einen Rest bedeutet, der ausgewählt ist aus der Gruppe von:
Hydroxy, Chlor, Trifluormethyl,
(C₁-C₆)-Alkyl, (C₁-C₆)-Alkoxy, (C₃-C₈)-Cycloalkyl,
   wobei Alkyl, Alkoxy und Cycloalkyl ihrerseits durch NR⁸R⁹ substituiert sein können,
   worin
   R⁸ und R⁹ unabhängig voneinander für Wasserstoff, (C₁-C₄)-Alkyl oder 5- oder 6-gliedriges Heteroaryl stehen oder
   R⁸ und R⁹ gemeinsam mit dem Stickstoffatom, an das sie gebunden sind, einen Morpholin-, Piperazin- oder N-Methylpiperazinring bilden,
(C₆-C₁₀)-Aryl, (C₆-C₁₀)-Aryloxy, 5- bis 10-gliedriges Heteroaryl, 5- bis 10-gliedriges Heteroaryloxy,
   wobei Aryl, Aryloxy, Heteroaryl und Heteroaryloxy ihrerseits durch Fluor, Chlor, Nitro, Carboxyl, Amino, Trifluormethyl, (C₁-C₄)-Alkyl, (C₁-C₄)-Alkoxy, (C₁-C₄)-Alkylamino, (C₁-C₄)-Alkoxycarbonyl, (C₁-C₄)-Alkanoylamino oder (C₁-C₄)-Alkoxycarbonylamino substituiert sein können,
und NR¹⁰R¹¹,
   worin
   R¹⁰ und R¹¹ unabhängig von einander für Wasserstoff, (C₁-C₆)-Alkyl, (C₃-C₈)-Cycloalkyl, (C₆-C₁₀)-Aryl oder 5- bis 10-gliedriges Heteroaryl stehen,
   wobei Alkyl und Cycloalkyl ihrerseits durch Hydroxy, (C₁-C₆)-Alkoxy, (C₆-C₁₀)-Aryl, 5- bis 10-gliedriges Heteroaryl oder NR⁸R⁹ substituiert sein können
   worin
   R⁸ und R⁹ die oben angegebene Bedeutung haben
   und
   Aryl und Heteroaryl ihrerseits durch Fluor, Chlor, Amino, Trifluormethyl, (C₁-C₆)-Alkyl (C₁-C₆)-Alkylamino oder (C₁₋C₆)-Alkanoylamino substituiert sein können, oder
   R¹⁰ und R¹¹ gemeinsam mit dem Stickstoffatom, an das sie gebunden sind, einen 5- oder 6-gliedrigen Heterocyclus bilden, der durch Fluor, Carboxyl, 1,1-Dioxyethylen, 5- oder 6-gliedriges Heterocyclyl mit einem oder zwei Heteroatomen N und/oder O, das seinerseits durch (C₁-C₄)-Alkyl substituiert sein kann, (C₁-C₄)-Alkoxy, durch Hydroxy oder (C₁-C₄)-Alkoxy substituiertes (C₁-C₄)-Alkyl, (C₁-C₄)-Alkanoyl oder NR¹²R¹³ substituiert ist,
   worin
   R¹² und R¹³ unabhängig voneinander Wasserstoff, (C₁-C₆)-Alkyl, (C₁-C₄)-Alkoxycarbonyl, (C₃-C₈)-Cycloalkyl oder (C₁-C₄)-Alkanoyl bedeuten oder
   R¹² und R¹³ gemeinsam mit dem Stickstoffatom, an das sie gebunden sind, einen 5- oder 6-gliedrigen Heterocyclus bilden, der ein weiteres Heteroatom O oder N im Ring enthalten kann und der durch (C₁-C₆)-Alkyl, (C₁-C₆)-Alkanoyl oder (C₁-C₆)-Alkoxycarbonyl substituiert sein kann, oder
   R¹⁰ und R¹¹ gemeinsam mit dem Stickstoffatom, an das sie gebunden sind, einen 7- bis 12-gliedrigen bicyclischen Heterocyclus bilden, der anneliert oder spirocyclisch ist und ein oder zwei weitere Heteroatome aus der Reihe N und/oder O im Ring aufweisen kann und der durch Fluor, (C₁-C₄)-Alkyl, (C₁-C₄)-Alkoxycarbonyl, (C₁-C₄)-Alkanoyl oder Benzyl substituiert sein kann,
und ihre Salze, Hydrate, Hydrate der Salze und Solvate.

Besonders bevorzugt sind Verbindungen der Formel (I),
worin
- A: einen Rest 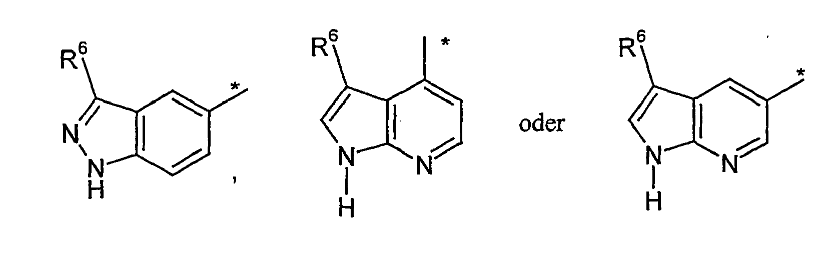 bedeutet,
worin
R⁶ für Wasserstoff oder Amino steht und
* für die Anknüpfstelle an den phenolischen Sauerstoff steht,
- R¹ und R²: unabhängig voneinander Wasserstoff, Fluor oder Chlor bedeuten,
- R³ und R⁴: Wasserstoff bedeuten,
- R⁵: einen Rest bedeutet, der ausgewählt ist aus der Gruppe von:
(C₁-C₄)-Alkyl, (C₁-C₄)-Alkoxy,
   wobei Alkyl und Alkoxy ihrerseits durch Amino oder Morpholinyl substituiert sein können,
Phenyl, Phenoxy, Pyridyl, Pyridyloxy,
   wobei Phenyl, Phenoxy, Pyridyl und Pyridyloxy ihrerseits durch Fluor, Chlor, Amino, Methyl oder NH-CO-CH₃ substituiert sein können,
und NR¹⁰R¹¹,
   worin
   R¹⁰ und R¹¹ unabhängig voneinander für Wasserstoff, 2-Hydroxyethyl, 3-Hydroxypropyl, 2-Aminoethyl, 3-Aminopropyl, 2-Morpholinoethyl, 3-Morpholinopropyl, 2-Aminocyclohexyl, Pyridyl oder Aminopyridyl stehen oder
   R¹⁰ und R¹¹ gemeinsam mit dem Stickstoffatom, an das sie gebunden sind, einen Piperazin-, N-Methylpiperazin- oder N-Isopropylpiperazinring oder einen Rest 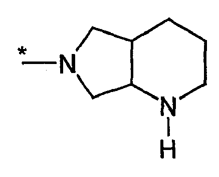 bilden,
   worin
   * für die Anknüpfstelle an den Pyrimidinring steht,
und ihre Salze, Hydrate, Hydrate der Salze und Solvate.

Weiterer Gegenstand der vorliegenden Erfindung ist ein Verfahren zur Herstellung der Verbindungen der Formel (I), das dadurch gekennzeichnet ist, dass man entweder
- [A]: Verbindungen der Formel (II) 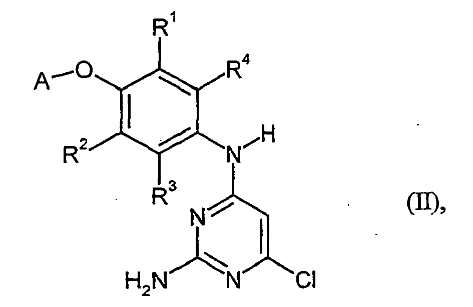 worin
A, R¹, R², R³ und R⁴ die oben angegebene Bedeutung aufweisen,
mit Verbindungen der Formel (III)

R⁵―X¹ (III),

worin
R⁵ die oben angegebene Bedeutung aufweist und
X¹ für Wasserstoff, B(OH)₂ oder einen Boronsäureester wie 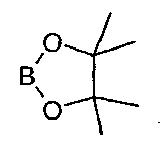 steht, oder
- **[B]**: Verbindungen der Formel (IV) 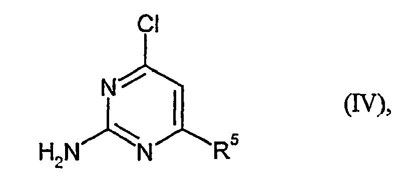 worin
R⁵ die oben angegebene Bedeutung aufweist, mit Verbindungen der Formel (V) 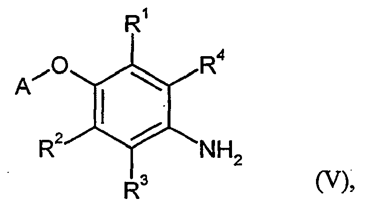 worin
A, R¹, R², R³ und R⁴ die oben angegebene Bedeutung aufweisen,
zu Verbindungen der Formel (I) umsetzt.

Im Verfahrensschritt [A] für den Fall, dass X¹ für Wasserstoff steht, erfolgt die Umsetzung, gegebenenfalls in Gegenwart einer Base, entweder in inerten Lösungsmitteln bei Normaldruck in einem Temperaturbereich von 20°C bis zum Rückfluss der Lösungsmittel oder bei erhöhtem Druck im Autoklaven bei Temperaturen oberhalb des Siedepunktes des Lösungsmittels bis 250°C oder alternativ in Substanz in der Schmelze.

Inerte Lösungsmittel sind beispielsweise Alkohole wie Methanol, Ethanol, Propanol, Isopropanol, Butanol oder 2-Ethylhexanol, *N*-alkylierte Carbonsäureamide wie Dimethylformamid, Dimethylacetamid oder N-Methylpyrrolidon, Alkylsulfoxide wie Dimethylsulfoxid, oder andere Lösungsmittel wie Acetonitril oder Pyridin. Bevorzugt sind Ethanol, Butanol, 2-Ethylhexanol, N-Methylpyrrolidon oder Dimethylformamid.

Basen sind beispielsweise Alkalihydroxide wie Natrium- oder Kaliumhydroxid, oder Alkalialkoholate wie Natrium- oder Kalium-tert.-butanolat, oder Alkalicarbonate wie Cäsiumcarbonat, Natrium- oder Kaliumcarbonat, oder Amide wie Lithiumdiisopropylamid, oder andere Basen wie DBU, Triethylamin oder Diisopropylethylamin, bevorzugt Diisopropylethylamin oder Triethylamin.

Im Verfahrensschritt [A] für den Fall, dass X¹ für B(OH)₂ oder für eine äquivalente Gruppe wie beispielsweise einen Boronsäureester steht, erfolgt die Umsetzung zu Verbindungen der Formel (I) im Allgemeinen in inerten Lösungsmitteln in Gegenwart eines Übergangsmetallkatalysators in Gegenwart einer Base bevorzugt in einem Temperaturbereich von 70°C bis 150°C bei Normaldruck.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, Nitroaromaten wie Nitrobenzol, gegebenenfalls *N*-alkylierte Carbonsäureamide wie Dimethylformamid, Dimethylacetamid, Alhylsulfoxide wie Dimethylsulfoxid oder cyclische Lactame wie *N*-Methylpyrrolidon. Die Lösungsmittel finden gegebenenfalls unter Zusatz von Ethanol oder Wasser Verwendung. Bevorzugte Lösungsmittel sind Dimethylformamid, 1,2-Dimethoxyethan und Toluol/Ethanol.

Als Übergangsmetallkatalysatoren werden bevorzugt Palladium(0)- oder Palladium-(II)-verbindungen, insbesondere Bis-(diphenylphosphanferrocenyl)-palladium(II)-chlorid, Dichlorbis(triphenylphosphin)-palladium oder Tetrakis(triphenylphosphin)-palladium(0) verwendet.

Als Basen werden Natrium- oder Kalium-tert.-butylat, oder Alkalihydroxide oder -salze wie Kaliumacetat, Natriumhydroxid, Natriumhydrogencarbonat, Natriumcarbonat oder Kaliumcarbonat, gegebenenfalls in Form ihrer wässrigen Lösungen, bevorzugt.

Im Verfahrensschritt [B] erfolgt die Umsetzung zu Verbindungen der Formel (I) in wässriger salzsaurer Lösung, bevorzugt in einem Temperaturbereich von 70°C bis 110°C bei Normaldruck.

Zur Herstellung der Verbindungen der Formel (II) aus Verfahrensschritt [A] setzt man Verbindungen der Formel (V) mit der Verbindung der Formel (VI) 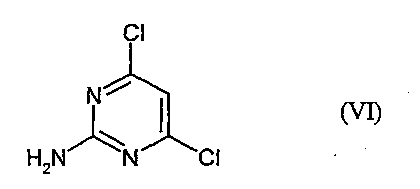 unter Reaktionsbedingungen, wie für den Verfahrensschritt [B] beschrieben, um.

Zur Herstellung der Verbindungen der Formel (IV) aus Verfahrensschritt [B] setzt man Verbindungen der Formel (VII) 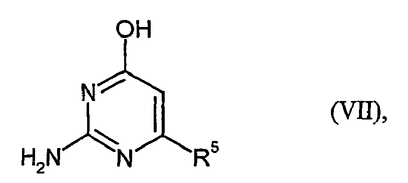 worin
- R⁵: die oben angegebene Bedeutung aufweist,
in Phosphorylchlorid unter Zugabe von 0,01 bis einem Äquivalent Dimethylformamid oder N,N-Dimethylanilin oder N,N-Diethylanilin, bevorzugt in einem Temperaturbereich von 50°C bis zur Rückflusstemperatur des Lösungsmittels bei Normaldruck um.

In einer anderen Verfahrensvariante setzt man zur Herstellung der Verbindungen der Formel (IV) Verbindungen der Formel (VI) mit Verbindungen der Formel (III) unter Reaktionsbedingungen, wie für den Verfahrensschritt [A] beschrieben, um.

Zur Herstellung der Verbindungen der Formel (VII) setzt man Verbindungen der Formel (VIII) 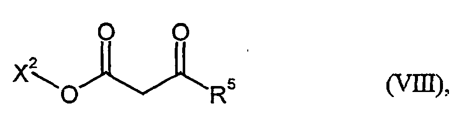 worin
- R⁵: die oben angegebene Bedeutung aufweist und
- X²: für Alkyl, bevorzugt für Methyl oder Ethyl, steht,
mit der Verbindung der Formel (IX) 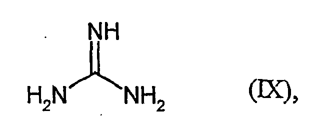 oder deren Salzen, vorzugsweise deren Carbonat, um.

Die Umsetzung der Verbindungen der Formel (VIII) und (IX) erfolgt beispielsweise zweistufig zunächst mit konzentrierter Salzsäure in Ethanol, bevorzugt in einem Temperaturbereich von 50°C bis zum Rückfluss der Lösungsmittel bei Normaldruck, und anschließend mit wässriger Natronlauge, bevorzugt in einem Temperaturbereich von 50°C bis zum Rückfluss der Lösungsmittel bei Normaldruck.

Zur Herstellung der Verbindungen der Formel (V) aus Verfahrensschritt [B] setzt man entweder Verbindungen der Formel (X) 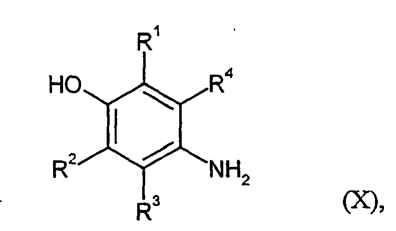 worin
R¹, R², R³ und R⁴ die oben angegebene Bedeutung aufweisen,
mit Verbindungen der Formel (XI)

A―X³ (XI),

worin
- A: die oben angegebene Bedeutung aufweist und
- X³: für Halogen, bevorzugt Fluor oder Chlor steht,
um.

Im Falle, dass X³ an einen Pyridinring gebunden ist, kann X³ für eine Nitrogruppe stehen.

Die Umsetzung erfolgt vorzugsweise in Substanz in Gegenwart von Kaliumhydroxid als Base in der Schmelze bei einer Temperatur von 200°C bis 280°C oder in einem inerten Lösungsmittel wie beispielsweise N,N-Dimethylformamid, N-Methylpyrrolidon oder Nitrobenzol in Gegenwart einer Base wie beispielsweise Kaliumhydroxid, Kalium-tert.-butylat oder Natriumhydrid bei einer Temperatur von 120°C bis 280°C.

Alternativ können Verbindungen der Formel (V) hergestellt werden, indem Verbindungen der Formel (XII), 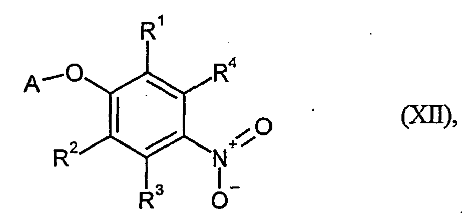 worin
A, R¹, R², R³ und R⁴ die oben angegebene Bedeutung aufweisen,
mit Reduktionsmitteln umgesetzt werden.

Die Umsetzung erfolgt im Allgemeinen in inerten Lösungsmitteln, bevorzugt in einem Temperaturbereich von Raumtemperatur bis zum Rückfluss der Lösungsmittel bei Normaldruck bis 3 bar.

Reduktionsmittel sind beispielsweise Palladium auf Aktivkohle und Wasserstoff, Platinoxid auf Aktivkohle und Wasserstoff, Zinndichlorid oder Titantrichlorid, bevorzugt ist Palladium auf Aktivkohle und Wasserstoff in Gegenwart von Hydrazinhydrat oder Platinoxid auf Aktivkohle und Wasserstoff.

Inerte Lösungsmittel sind beispielsweise Ether wie Diethylether, Methyl-tert.-butylether, 1,2-Dimethoxyethan, Dioxan, Tetrahydrofuran, Glykoldimethylether oder Diethylenglykoldimethylether, Alkohole wie Methanol, Ethanol, n-Propanol, isoPropanol, n-Butanol, tert.-Butanol oder 2-Ethylhexanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Pyridin, als Lösungsmittel sind bevorzugt Ethanol, n-Butanol oder 2-Ethylhexanol.

Zur Herstellung der Verbindungen der Formel (XII) setzt man Verbindungen der Formel (XIII), 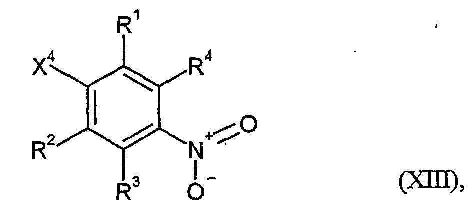 worin
- R¹, R², R³ und R⁴: die oben angegebene Bedeutung aufweisen und
- x⁴: für Halogen, bevorzugt Fluor oder Chlor, steht,
mit Verbindungen der Formel (XIV)

A―OH (XIV),

worin
- A: die oben angegebene Bedeutung aufweist,
um.

Die Umsetzung erfolgt im Allgemeinen in inerten Lösungsmitteln, gegebenenfalls in Gegenwart einer Base, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Methylenchlorid, Trichlormethan oder 1,2-Dichlorethan, Ether wie Dioxan, Tetrahydrofuran oder 1,2-Dimethoxyethan, oder andere Lösemittel wie Aceton, Dimethylformamid, Dimethylacetamid, 2-Butanon oder Acetonitril, bevorzugt Acetonitril, Dimethylformamid oder 1,2-Dimethoxyethan.

Basen sind beispielsweise Alkalicarbonate wie Cäsiumcarbonat, Natrium- oder Kaliumcarbonat, oder Natrium- oder Kaliummethanolat, oder Natrium- oder Kaliumethanolat oder Kalium-tert.-butylat, oder Amide wie Natriumamid, Lithiumbis-(trimethylsilyl)amid oder Lithiumdiisopropylamid, oder metallorganische Verbindungen wie Butyllithium oder Phenyllithium, oder andere Basen wie Natriumhydrid, DBU, bevorzugt Kalium-tert.-butylat, Cäsiumcarbonat, Kaliumcarbonat oder Natriumcarbonat.

Die Verbindungen der Formel (III), (VI), (VIII), (IX), (X), (XI), (XIII) und (XIV) sind dem Fachmann an sich bekannt oder lassen sich nach üblichen literaturbekannten Verfahren herstellen.

Die Verbindungen der Formel (I) lassen sich beispielsweise durch Umsetzung mit Oxidationsmitteln weiter derivatisieren.

Die Herstellung der erfindungsgemäßen Verbindungen kann durch folgende Syntheseschemata verdeutlicht werden.

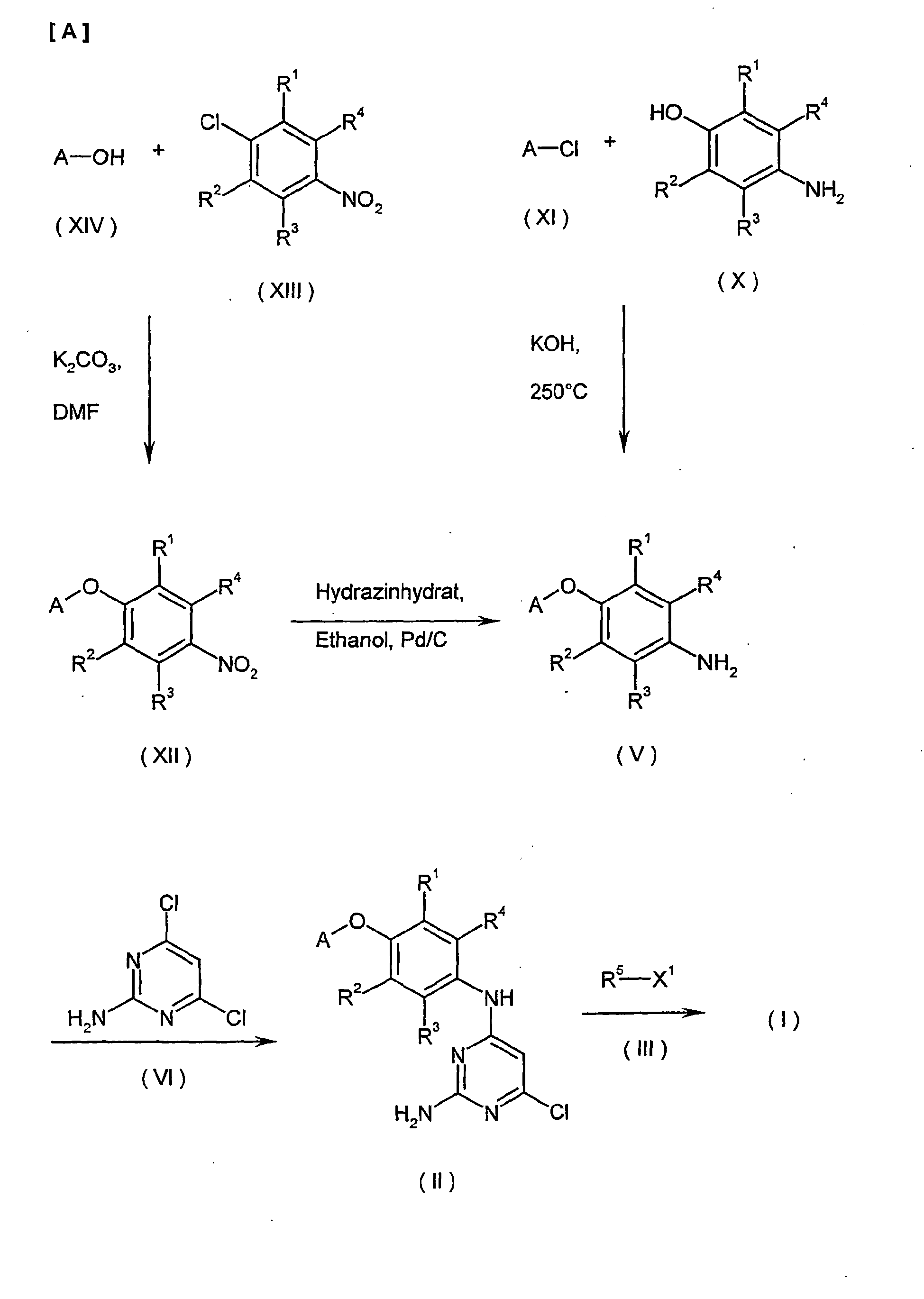

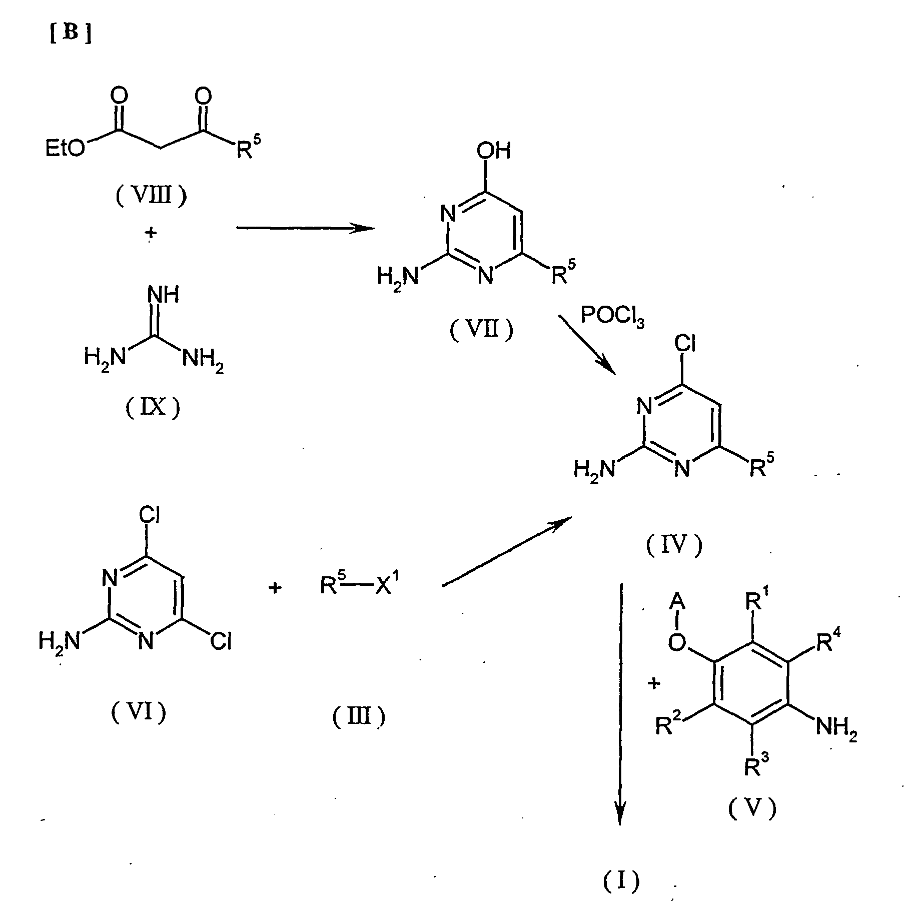

Die erfindungsgemäßen Verbindungen zeigen ein nicht vorhersehbares, wertvolles pharmakologisches und pharmakokinetisches Wirkspektrum.

Sie eignen sich daher zur Verwendung als Arzneimittel zur Behandlung und/oder Prophylaxe von Krankheiten bei Menschen und Tieren.

Die pharmazeutische Wirksamkeit der erfindungsgemäßen Verbindungen lässt sich durch ihre Wirkung als Rho-Kinase-Inhibitoren erklären.

Weiterer Gegenstand der vorliegenden Erfindung ist der Einsatz der erfindungs-gemäßen Verbindungen Herstellung von Medikamenten zur Behandlung und/oder Prophylaxe von Erkrankungen, vorzugsweise von kardiovaskulären Erkrankungen.

Die erfindungsgemäßen Verbindungen sind geeignet für die Prophylaxe und/oder Behandlung von kardiovaskulären Erkrankungen wie beispielsweise Bluthochdruck und Herzinsuffizienz, stabiler und instabiler Angina pectoris, peripheren und kardialen Gefäßerkrankungen, von Arrhythmien, von thromboembolischen Erkrankungen und Ischämien wie Myokardinfarkt, Hirnschlag, transitorischen und ischämischen Attacken, peripheren Durchblutungsstörungen, Subarachnoidalblutungen, Verhinderung von Restenosen wie beispielsweise nach Thrombolysetherapien, percutanen transluminalen Angioplastien (PTA), percutanen transluminalen Koronarangioplastien (PTCA), Bypass sowie zur Prophylaxe und/oder Behandlung von Arteriosklerose, asthmatischen Erkrankungen, COPD und Krankheiten des Urogenitalsystems wie beispielsweise Prostatahypertrophie, erektiler Dysfunktion, weiblicher sexueller Dysfunktion, Osteoporose, Gastroparese und Inkontinenz.

Weiterhin können die erfindungsgemäßen Verbindungen zur Prophylaxe und/oder Behandlung von Krebserkrankungen, insbesondere von Tumoren eingesetzt werden.

Im Rahmen der vorliegenden Erfindung umfasst die Definition von Tumoren sowohl benigne, wie auch maligne Tumore und damit beispielsweise auch benigne Neoplasien, Dysplasien, Hyperplasien, wie auch Neoplasien mit Metastasenbildung. Weitere Beispiele für Tumore sind Karzinome, Sarkome, Karzinosarkome, Tumore der blutbildenden Organe, Tumore des Nervengewebes z.B. des Gehirns oder Tumore von Hautzellen. Bei der Tumorbildung kommt es zur unkontrollierten oder unzureichend kontrollierten Zellteilung. Der Tumor kann örtlich begrenzt sein, er kann aber auch das umliegende Gewebe infiltrieren und sich dann durch das lymphatische System oder durch den Blutstrom an einem neuen Ort festsetzen. Somit gibt es primäre und sekundäre Tumore. Primäre Tumore sind ursprünglich in dem Organ entstanden, in dem sie gefunden werden. Sekundäre Tumore haben sich durch Metastasenbildung in einem anderen Organ festgesetzt und sich dann an ihrem neuen Ort ausgebreitet.

Weiterer Gegenstand der vorliegenden Erfindung ist die Verwendung der erfindungsgemäßen Verbindungen Hestellung von Medikamenten zur Prophylaxe und/oder Behandlung von Erkrankungen, insbesondere der zuvor genannten Krankheitsbilder.

Weiterer Gegenstand der vorliegenden Erfindung ist die Verwendung der erfindungsgemäßen Verbindungen zur Herstellung eines Arzneimitteln zur Prophylaxe und/oder Behandlung von Erkrankungen, insbesondere der zuvor genannten Krankheitsbilder.

Weiterer Gegenstand der vorliegenden Erfindung ist ein Verfahren zur Prophylaxe und/oder Behandlung von Erkrankungen, insbesondere der zuvor genannten Erkrankungen, unter Verwendung einer kardiovaskulär wirksamen Menge der erfindungsgemäßen Verbindung.

Weiterer Gegenstand der vorliegenden Erfindung sind Arzneimittel, enthaltend eine erfindungsgemäße Verbindung und einen oder mehrere weitere Wirkstoffe, insbesondere zur Prophylaxe und/oder Behandlung der zuvor genannten Erkrankungen.

Die erfindungsgemäße Verbindung kann systemisch und/oder lokal wirken. Zu diesem Zweck kann sie auf geeignete Weise appliziert werden, wie z.B. oral, parenteral, pulmonal, nasal, sublingual, lingual, buccal, rectal, transdermal, conjunctival, otisch, als Stents oder als Implantat.

Für diese Applikationswege kann die erfindungsgemäße Verbindung in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten, sich verzögert auflösenden oder unlöslichen Überzügen, die die Freisetzung der erfindungsgemäßen Verbindungen kontrollieren), in der Mundhöhle schnell zerfallende Tabletten oder Filme/Oblaten, Kapseln, Dragees, Granulate, Pellets, Pulver, Emulsionen, Suspensionen, Aerosole oder Lösungen.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan, oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Für die sonstigen Applikationswege eignen sich z.B. Inhalationsarzneiformen (u.a. Pulverinhalatoren, Nebulizer), Nasentropfen / -lösungen, Sprays; lingual, sublingual oder buccal zu applizierende Tabletten oder Kapseln, Suppositorien, Ohren- und Augenpräparationen, Vaginalkapseln, wässrige Suspensionen (Lotionen, Schüttelmixturen), lipophile Suspensionen, Salben, Cremes, Milch, Pasten, Streupuder oder Implantate.

Die erfindungsgemäßen Verbindungen können in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und/oder Geruchskorrigentien.

Weiterer Gegenstand der vorliegenden Erfindung sind Arzneimittel, die mindestens eine erfindungsgemäße Verbindung, vorzugsweise zusammen mit einem oder mehreren inerten nichttoxischen, pharmazeutisch geeigneten Hilfsstoff enthalten, sowie deren Verwendung zu den zuvor genannten Zwecken.

Im Allgemeinen hat es sich sowohl in der Human- als auch in der Veterinärmedizin als vorteilhaft erwiesen, die erfindungsgemäße Verbindung in Gesamtmengen von etwa 0,01 bis etwa 700, vorzugsweise 0,01 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält die erfindungsgemäße Verbindung vorzugsweise in Mengen von etwa 0,1 bis etwa 80, insbesondere 0,1 bis 30 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- DC: Dünnschichtchromatographie
- DCI: direkte chemische Ionisation (bei MS)
- DCM: Dichlormethan
- DIEA: *N,N*-Diisopropylethylamin
- DMA: Dimethylacetamid
- DMF: *N,N*-Dimethylformamid
- DMSO: Dimethylsulfoxid
- d. Th.: der Theorie
- EE: Ethylacetat (Essigsäureethylester)
- EI: Elektronenstoß-Ionisation (bei MS)
- ESI: Elektrospray-Ionisation (bei MS)
- Fp.: Schmelzpunkt
- ges.: gesättigt
- h: Stunde
- HATU: *O*-(7-Azabenzotriazol-1-yl)-*N,N,N',N'*-tetramethyluronium-Hexafluorphosphat
- HOAT: 3H-[1,2,3]-Triazol[4,5-b]pyridin-3-ol
- HOBt: 1-Hydroxy-1H-benzotriazol x H₂O
- HPLC: Hochdruck-, Hochleistungsflüssigchromatographie
- konz.: konzentriert
- LC-MS: Flüssigchromatographie-gekoppelte Massenspektroskopie
- LDA: Lithium-Diisopropylamid
- min.: Minuten
- MPLC: Mitteldruck-, Mittelleistungsflüssigchromatographie
- MS: Massenspektroskopie
- NMR: Kernresonanzspektroskopie
- org.: organisch
- proz.: prozentig
- RF: Rückfluss
- R_{f}: Retentionsfaktor (bei DC)
- RP-HPLC: Reverse Phase HPLC
- RT: Raumtemperatur
- Rt: Retentionszeit (bei HPLC)
- TFA: Trifluoressigsäure
- THF: Tetrahydrofuran

### HPLC-, LCMS- und GCMS-Methoden:

### Methode 1 (LC/MS)

Instrument: Micromass Platform LCZ, HP1100; Säule: Symmetry C18, 50 mm x 2.1 mm, 3.5 µm; Eluent A: Wasser + 0.05% Ameisensäure, Eluent B: Acetonitril + 0.05% Ameisensäure; Gradient: 0.0 min 90% A → 4.0 min 10% A → 6.0 min 10% A; Ofen: 40°C; Fluss: 0.5 ml/min; UV-Detektion: 208-400 nm.

### Methode 2 (LC/MS)

Instrument: Micromass Platform LCZ mit HPLC Agilent Serie 1100; Säule: Grom-SIL120 ODS-4 HE, 50 mm x 2.0 mm, 3 µm; Eluent A: 1 1 Wasser + 1 ml 50%ige Ameisensäure, Eluent B: 1 1 Acetonitril + 1 ml 50%ige Ameisensäure; Gradient: 0.0 min 100% A → 0.2 min 100% A → 2.9 min 30% A → 3.1 min 10% A → 4.5 min 10% A; Ofen: 55°C, Fluss: 0.8 ml/min, UV-Detektion: 2.08-400 nm.

### Methode 3 (LC/MS)

Instrument: Finnigan MAT 900S, TSP: P4000, AS3000, UV3000HR; Säule: Symmetry C 18, 150 mm x 2.1 mm, 5.0 µm; Eluent C: Wasser, Eluent B: Wasser + 0.3 g 35%ige Salzsäure, Eluent A: Acetonitril; Gradient: 0.0 min 2% A → 2.5 min 95% A → 5 min 95 % A; Ofen: 70°C; Fluss: 1.2 ml/min; UV-Detektion: 210 nm.

### Methode 4 (LC/MS)

Instrument: Micromass Quattro LCZ, HP1100; Säule: Symmetry C18, 50 mm x 2.1 mm, 3.5 µm; Eluent A: Acetonitril + 0.1 % Ameisensäure, Eluent B: Wasser + 0.1 % Ameisensäure; Gradient: 0.0 min 10% A → 4.0 min 90% A → 6.0 min 90% A; Ofen: 40°C; Fluss: 0.5 ml/min; UV-Detektion: 208-400 nm.

### Methode 5 (LC/MS)

Instrument: Micromass Quattro LCZ mit HPLC Agilent Serie 1100; Säule: UPTISPHERE HDO, 50 mm x 2.0 mm, 3 µm; Eluent A: 1 1 Wasser + 1 ml 50%ige Ameisensäure, Eluent B: 1 1 Acetonitril + 1 ml 50%ige Ameisensäure; Gradient: 0.0 min 100% A → 0.2 min 100% A → 2.9 min 30% A → 3.1 min 10% A → 4.5 min 10% A; Ofen: 55°C, Fluss: 0.8 ml/min, UV-Detektion: 208-400 nm.

### Methode 6 (LC/MS)

Gerätetyp MS: Micromass ZQ; Gerätetyp HPLC: Waters Alliance 2790; Säule: Uptisphere C 18, 50 mm x 2.0 mm, 3.0 µm; Eluent B: Acetonitril + 0.05% Ameisensäure, Eluent A: Wasser + 0.05% Ameisensäure; Gradient: 0.0 min 5% B → 2.0 min 40% B → 4.5 min 90% B→ 5.5 min 90% B; Ofen: 45°C; Fluss: 0.0 min 0.75 ml/min → 4.5 min 0.75 ml/min→ 5.5 min 1.25 ml/min; UV-Detektion: 210 nm.

### Methode 7 (HPLC)

Instrument: HP 1100 mit DAD-Detektion; Säule: Kromasil RP-18, 60 mm x 2 mm, 3.5 µm; Eluent A: 5 ml HClO₄/l Wasser, Eluent B: Acetonitril; Gradient: 0 min 2% B, 0.5 min 2% B, 4.5 min 90% B, 6.5 min 90% B; Fluss: 0.75 ml/min; Temp.: 30°C; UV-Detektion: 210 nm.

### Methode 8 (LC/MS)

Gerätetyp MS: Micromass ZQ; Gerätetyp HPLC: Waters Alliance 2790; Säule: Grom-Sil 120 ODS-4 HE 50 mm x 2 mm, 3.0 µm; Eluent B: Acetonitril + 0.05% Ameisensäure, Eluent A: Wasser + 0.05% Ameisensäure; Gradient: 0.0 min 5% B → 2.0 min 40% B → 4.5 min 90% B→ 5.5 min 90% B; Ofen: 45°C; Fluss: 0.0 min 0.75 ml/min → 4.5 min 0.75 ml/min → 5.5 min 1.25 ml/min; UV-Detektion: 210 nm.

### Methode 9 (LC/MS)

Instrument: Micromass Quattro LCZ, mit HPLC Agilent Serie 1100 ; Säule: Grom-SIL120 ODS-4 HE, 50 mm x 2.0 mm, 3 µm; Eluent A: 1 1 Wasser + 1 ml 50%ige Ameisensäure, Eluent B: 1 1 Acetonitril + 1 ml 50%ige Ameisensäure; Gradient: 0.0 min 100% A → 0.2 min 100% A → 2.9 min 30% A → 3.1 min 10% A → 4.5 min 10% A; Ofen: 55°C, Fluss: 0.8 ml/min, UV-Detektion: 208-400 nm.

### Methode 10 (LC/MS)

Gerätetyp MS: Micromass ZQ; Gerätetyp HPLC: HP 1100 Series; UV DAD; Säule: Grom-Sil 120 ODS-4 HE 50 mm x 2 mm, 3.0 µm; Eluent A: Wasser + 500 µl 50%ige Ameisensäure/l, Eluent B: Acetonitril + 500 µl 50%ige Ameisensäure/l; Gradient: 0.0 min 0% B → 2.9 min 70% B → 3.1 min 90% B → 4.5 min 90% B; Ofen: 50°C, Fluss:0.8 ml/min, UV-Detektion: 210 nm.

### Methode 11 (HPLC)

Instrument: HP 1100 mit DAD-Detektion; Säule: Kromasil RP-18, 60 mm x 2 mm, 3.5 µm; Eluent A: 5 ml HClO₄/l Wasser, Eluent B: Acetonitril; Gradient: 0 min 2% B, 0.5 min 2% B, 4.5 min 90% B, 9 min 90% B; Fluss: 0.75 ml/min, Temp.: 30°C, UV-Detektion: 210 nm.

### Methode 12 (LC/MS)

Instrument: Micromass Platform LCZ mit HPLC Agilent Serie 1100; Säule: Grom-SIL120 ODS-4 HE, 50 mm x 2.0 mm, 3 µm; Eluent A: 1 1 Wasser + 1 ml 50%ige Ameisensäure, Eluent B: 1 1 Acetonitril + 1 ml 50%ige Ameisensäure; Gradient: 0.0 min 100% A → 0.2 min 100% A → 2.9 min 30% A → 3.1 min 10% A → 4.5 min 10% A; Ofen: 55°C, Fluss: 0.8 ml/min, UV-Detektion: 210 nm.

### Methode 13 (LC/MS)

Gerätetyp MS: Micromass ZQ; Gerätetyp HPLC: HP 1100 Series; UV DAD; Säule: Phenomenex Synergi 2µ Hydro- RP Mercury 20 mm x 4 mm; Eluent A: 11 Wasser + 0.5 ml 50%ige Ameisensäure, Eluent B: 1 1 Acetonitril + 0.5 ml 50%ige Ameisensäure; Gradient: 0.0 min 90% A Fluss 1 ml/min → 2.5 min 30% A Fluss 2 ml/min→ 3.0 min 5% A Fluss 2 ml/min → 4.5min 5% A Fluss 2 ml/min; Ofen: 50°C; UV-Detektion: 210 nm.

### Methode 14 (LC/MS)

Instrument: Micromass Quattro LCZ mit HPLC Agilent Serie 1100; Säule: Phenomenex Synergi 2µ Hydro- RP Mercury 20 mm x 4 mm; Eluent A: 11 Wasser + 0.5 ml 50%ige Ameisensäure, Eluent B: 1 1 Acetonitril + 0.5 ml 50%ige Ameisensäure; Gradient: 0.0 min 90% A Fluss 1 ml/min → 2.5 min 30% A Fluss 2 ml/min→ 3.0 min 5% A Fluss 2 ml/min → 4.5 min 5% A Fluss 2 ml/min; Ofen: 50°C; UV-Detektion: 208-400 nm.

### Methode 15 (LC/MS)

Gerätetyp MS: Micromass ZQ; Gerätetyp HPLC: Waters Alliance 2795; Säule: Phenomenex Synergi 2µ Hydro- RP Mercury 20 mm x 4 mm; Eluent A: 11 Wasser + 0.5 ml 50%ige Ameisensäure, Eluent B: 1 1 Acetonitril + 0.5 ml 50%ige Ameisensäure; Gradient: 0.0 min 90% A Fluss 1 ml/min → 2.5 min 30% A Fluss 2 ml/min → 3.0 min 5% A Fluss 2 ml/min → 4.5 min 5% A Fluss 2 ml/min; Ofen: 50°C; UV-Detektion: 210 nm.

### Methode 16 (LC/MS)

Gerätetyp MS: Micromass ZQ; Gerätetyp HPLC: Waters Alliance 2795; Säule: Merck Chromolith SpeedROD RP-18e 50 mm x 4.6 mm; Eluent A: Wasser + 500 µl 50%ige Ameisensäure/l; Eluent B: Acetonitril + 500 µl 50%ige Ameisensäure/l; Gradient: 0.0 min 10% B → 3.0 min 95% B → 4.0 min 95% B; Ofen: 35 °C; Fluss: 0.0 min 1.0 ml/min, 3.0 min 3.0 ml/min, 4.0 min 3.0 ml/min; UV-Detektion: 210 nm.

### Methode 17 (LC/MS)

Instrument: Micromass Platform LCZ mit HPLC Agilent Serie 1100; Säule: Phenomenex Synergi 2µ Hydro- RP Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90% A Fluss 1 ml/min → 2.5 min 30% A Fluss 2 ml/min → 3.0 min 5% A Fluss 2 ml/min → 4.5min 5% A Fluss 2 ml/min; Ofen: 50°C; UV-Detektion: 210 nm.

### Methode 18 (LC/MS)

Gerätetyp MS: Micromass ZQ; Gerätetyp HPLC: HP 1100 Series; UV DAD; Säule: Grom-Sil 120 ODS-4 HE 50 mm x 2 mm, 3.0 µm; Eluent A: Wasser + 500 µl 50%ige Ameisensäure/l, Eluent B: Acetonitril + 500 µl 50%ige Ameisensäure/l; Gradient: 0.0 min 70% B → 4.5 min 90% B; Ofen: 50°C, Fluss: 0.8 ml/min, UV-Detektion: 210 nm.

### Präparative HPLC

Säule: YMC Gel ODS-AQ S-5 / 15 µM, 250 mm x 30 mm, Eluent A: Wasser, Eluent B: Acetonitril; Gradient: 0.00 min 30% B → 3.00 min 30% B → 34.0 min 95% B → 38.0 min 30% B; Temp.: Raumtemperatur; Fluss: 50 ml/min; UV-Detektion.

### Ausgangsverbindungen

### Beispiel I

### 3-Methyl-1H-indazol-4-ol

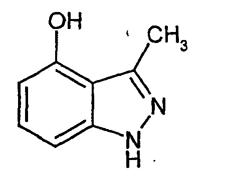

800 mg (5.26 mmol) 2,6-Dihydroxyacetophenon werden mit 526 mg (10.5 mmol) Hydrazinhydrat und 1 ml Eisessig versetzt. Nach 15-minütigem Rühren bei 110°C wird die Reaktionsmischung auf Raumtemperatur abgekühlt. Nach Zugabe von 6 ml Polyphosphorsäure wird 20 min auf 120°C erhitzt. Nach Abkühlen auf Raumtemperatur werden 0.8 g (7.89 mmol) Essigsäureanhydrid zugetropft. Man erhitzt nochmals für 20 min auf 120°C. Anschließend wird die auf Raumtemperatur abgekühlte Reaktionsmischung auf Eis gegossen. Man neutralisiert mit 1N Natriumhydroxid-Lösung und extrahiert dreimal mit Ethylacetat. Die vereinigten organischen Phasen werden zweimal mit Wasser gewaschen. Nach Trocknen über Natriumsulfat wird das Lösungsmittel im Vakuum entfernt. Das Produkt wird mittels Säulenchromatographie gereinigt. Zum Eluieren wird ein Gemisch aus Cyclohexan und Ethylacetat (1:1) verwendet.
Ausbeute: 429 mg (55 %)
LC-MS (Methode 1): Rₜ = 1.8 min.
MS (ESI pos.): m/z = 149 (M+H)⁺
¹H-NMR (DMSO-d₆, 300 MHz): δ = 2.54 (s, 3H), 6.31 (d, 1H), 6.79 (d, 1H), 7.02 (t, 1H), 9.84 (s, 1H), 12.3 (s, 1H).

### Beispiel II

### 4-(2-Fluor-4-nitrophenoxy)-3-methyl-1H-indazol

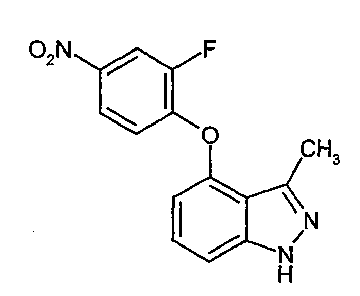

100 mg (0.63 mmol) 3,4-Difluornitrobenzol werden mit 93.1 mg (0.63 mmol) 3-Methyl-1H-indazol-4-ol (aus Beispiel I) und 95.6 mg (0.69 mmol) Kaliumcarbonat in 5 ml wasserfreiem Dimethylformamid suspendiert und fünf Stunden bei 50°C gerührt. Anschließend wird die Reaktionslösung mit Wasser verdünnt und zweimal mit Ethylacetat extrahiert. Die vereinten organischen Phasen werden über Natriumsulfat getrocknet. Das Lösungsmittel wird im Vakuum entfernt und der Rückstand mittels Säulenchromatographie gereinigt. Zum Eluieren wird ein Gemisch aus Cyclohexan und Ethylacetat (1:1) verwendet.
Ausbeute: 93 mg (51.5 %)
LC-MS (Methode 2): Rₜ = 3.7 min.
MS (ESI pos.): m/z = 288 (M+H)⁺
¹H-NMR (DMSO-d₆, 300 MHz): δ = 2.36 (s, 3H), 6.73 (dd, 1H), 7.07 (t, 1H), 7.37 (m, 2H), 8.06 (m, 1H), 8.37 (dd, 1H), 13.06 (s, 1H).

### Beispiel III

### 3-Fluor-4-[(3-methyl-1H-indazol-4-yl)oxy]phenylamin

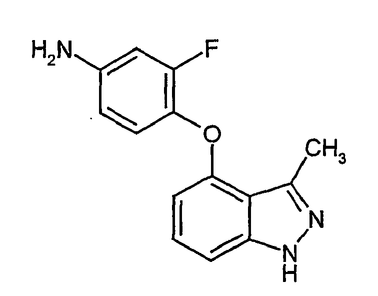

75 mg (0.26 mmol) 4-(2-Fluor-4-nitrophenoxy)-3-methyl-1H-indazol (aus Beispiel II) werden in 3 ml Ethanol gelöst und mit 261 mg (5.22 mmol) Hydrazinhydrat sowie mit 10 mg 10 %igem Palladium auf Aktivkohle versetzt. Man erhitzt zwei Stunden auf 80°C. Anschließend wird über Kieselgur filtriert. Das Filtrat wird im Vakuum eingeengt.
Ausbeute: 65 mg (96.8 %)
LC-MS (Methode 5): Rₜ = 3.23 min.
MS (ESI pos.): m/z = 258 (M+H)⁺
¹H-NMR (DMSO-d₆, 300 MHz): δ = 2.61 (s, 3H), 5.32 (s, 2H), 6.06 (d, 1H), 6.41 (dd, 1H), 6.52 (dd, 1H), 6.96 (t, 1H), 7.08 (m, 2H), 12.63 (s, 1H).

### Beispiel IV

### 4-Methoxy-1,2-benzisoxazol-3-amin

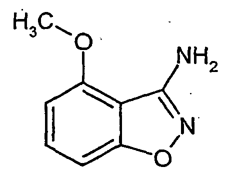

709.5 mg (9.45 mmol) Acetohydroxamsäure werden in 8 ml wasserfreiem Dimethylformamid vorgelegt und bei Raumtemperatur mit 1.06 g (9.45 mmol) Kalium-*tert-*butylat versetzt. Nach 40-minütigem Rühren wird 1 g (6.62 mmol) 2-Fluor-6-methoxybenzonitril zugesetzt. Man erwärmt 16 Stunden auf 60°C. Anschließend wird die Reaktionsmischung mit 20 ml gesättigter Natriumchlorid-Lösung versetzt. Die dabei ausfallenden Kristalle werden abgesaugt und mit Wasser gewaschen.
Ausbeute: 358 mg (23 %)
LC-MS (Methode 6): Rₜ = 2.59 min.
MS (ESI pos.): m/z = 165 (M+H)⁺
¹H-NMR (DMSO-d₆, 300 MHz): δ = 3.91 (s, 3H), 5.91 (s, 2H), 6.71 (d, 1H), 6.98 (d, 1H), 7.43 (t,1H).

### Beispiel V

### 3-Amino-1,2-benzisoxazol-4-ol

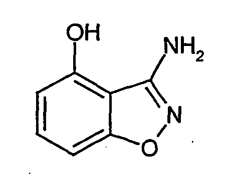

250 mg (1.52 mmol) 4-Methoxy-1,2-benzisoxazol-3-amin (aus Beispiel IV) werden in 5 ml absolutem Methylenchlorid gelöst und unter Argon mit 7.6 ml (7.6 mmol) 1M-Bortribromid-Lösung in Methylenchlorid versetzt. Man rührt über Nacht bei Raumtemperatur und hydrolysiert dann vorsichtig mit Wasser. Der ausgefallene Niederschlag wird abfiltriert, mit Wasser gewaschen und im Hochvakuum getrocknet.
Ausbeute: 195 mg (85.3 %)
LC-MS (Methode 6): Rₜ = 1.78 min.
MS (ESI pos.): m/z = 151 (M+H)⁺
¹H-NMR (DMSO-d₆, 300 MHz): δ = 5.90 (s, 2H), 6.70 (d, 1H), 6.97 (d, 1H), 7.42 (t, 1H).

### Beispiel VI

### 4-(2-Fluor-4-nitrophenoxy)-1,2-benzisoxazol-3-amin

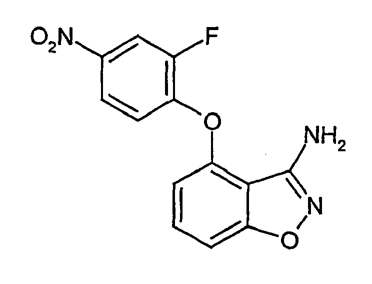

94.37 mg (0.63 mmol) 3-Amino-1,2-benzisoxazol-4-ol (aus Beispiel V) werden mit 100 mg (0.63 mmol) 3,4-Difluornitrobenzol und 95.56 mg (0.69 mmol) Kaliumcarbonat in 3 ml wasserfreiem Dimethylformamid suspendiert und 16 Stunden bei Raumtemperatur gerührt. Anschließend wird die Reaktionslösung mit Wasser verdünnt und der ausgefallene Niederschlag abfiltriert. Das Produkt wird im Hochvakuum getrocknet.
Ausbeute: 151 mg (83.1 %)
LC-MS (Methode 2): Rₜ = 3.5 min.
MS (ESI pos.): m/z = 290 (M+H)⁺
¹H-NMR (DMSO-d₆, 300 MHz): δ = 6.15 (s, 2H), 6.64 (d, 1H), 7.28 (d, 1H), 7.49 (m, 2H), 8.14 (m, 1H), 8.39 (dd, 1H).

### Beispiel VII

### 4-(4-Amino-2-fluorphenoxy)-1,2-benzisoxazol-3-amin

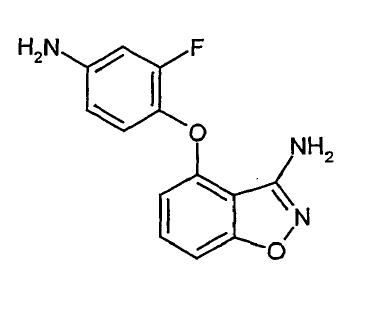

120 mg (0.41 mmol) 4-(2-Fluor-4-nitrophenoxy)-1,2-benzisoxazol-3-amin (aus Beispiel VI) werden mit 468 mg (2.07 mmol) Zinn(II)chlorid-Dihydrat in 8 ml Ethylacetat gelöst und 4 Stunden auf 70°C erhitzt. Nach Abkühlen auf Raumtemperatur wird die Reaktionslösung mit gesättigter Natriumhydrogencarbonat-Lösung auf pH 9 eingestellt, wobei ein farbloser Niederschlag entsteht. Die Suspension wird mit 5 g Kieselgur versetzt und filtriert. Das Filtrat wird mehrfach mit Ethylacetat extrahiert. Die vereinigten organischen Phasen werden mit Wasser gewaschen und über Natriumsulfat getrocknet. Nach Entfernen des Lösungsmittels im Vakuum wird das Produkt erhalten.
Ausbeute: 102 mg (94.8 %)
LC-MS (Methode 2): Rₜ = 2.95 min.
MS (ESI pos.): m/z = 260 (M+H)⁺
¹H-NMR (DMSO-d₆, 300 MHz): δ = 5.40 (s, 2H), 6.03 (s, 2H), 6.24 (d, 1H), 6.43 (m, 1H), 6.46 (dd, 1H), 7.04 (m, 2H), 7.34 (t, 1H).

### Beispiel VIII

### 1H-Indazol-6-ol

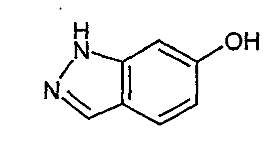

8.00 g (60.1 mmol) 6-Aminoindazol werden mit verdünnter Schwefelsäure (8 ml konzentrierte Schwefelsäure in 52.8 ml H₂O) versetzt und auf 0°C gekühlt. Dazu tropft man langsam eine wässrige Natriumnitrit-Lösung (4.48 g Natriumnitrit in 12.8 ml Wasser). Die Mischung wird 1 Stunde bei 0°C gerührt. Nach Zugabe von 5.60 g (90.6 mmol) Borsäure und weiteren 8 ml verdünnter Schwefelsäure wird 15 min unter Rückfluss erhitzt. Die Reaktionslösung wird abgekühlt und mit 25%iger Ammoniak-Lösung neutralisiert. Der hierbei ausgefallene Feststoff wird abgesaugt und in Wasser aufgekocht. Es wird heiß filtriert. Das Filtrat wird mehrmals mit Ethylacetat extrahiert. Die vereinigten organischen Phasen werden über Magnesiumsulfat getrocknet und eingeengt.
Ausbeute: 5.15 g (58 %)
LC-MS (Methode 2): Rₜ = 2.25 min.
MS (ESI pos.): m/z = 134 (M+H)⁺
¹H-NMR (DMSO-d₆, 200 MHz): δ = 6.62 (dd, 1H), 6.77 (d, 1H), 7.51 (d, 1H), 7.88 (s, 1H), 9.63 (s, 1H), 12.58 (s, 1H).

### Beispiel IX

### 5-(2-Fluor-4-nitrophenoxy)-1H-indazol

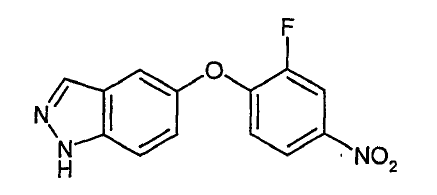

5.00 g (37.3 mmol) 5-Hydroxyindazol werden in 40 ml wasserfreiem DMF gelöst. Dazu gibt man 5.93 g (37.3 mmol) 3,4-Difluornitrobenzol und 5.15 g (37.3 mmol) Kaliumcarbonat und lässt über Nacht bei 40°C rühren. Zur Aufarbeitung saugt man über Kieselgur ab, verdünnt mit Wasser und extrahiert dreimal mit Ethylacetat: Die vereinigten organischen Phasen werden 1x mit gesättigter wässriger Natriumchlorid-Lösung gewaschen, über Natriumsulfat getrocknet und am Rotationsverdampfer eingeengt. Der Rückstand wird zweimal über Kieselgel mit Cyclohexan/ Ethylacetat 3:1 → 1:1 → 1:2 chromatographiert und anschließend mittels präparativer HPLC gereinigt.
Ausbeute: 4.02 g (39 %)
MS (ESI pos.): m/z = 274 (M+H)⁺
¹H-NMR (DMSO-d₆, 300 MHz): δ = 6.98 (t, 1H), 7.22 (dd, 1H), 7.58 - 7.69 (m, 2H), 7.95 - 8.12 (m, 2H), 8.33 (dd, 1H), 13.20 - 13.36, br. s, 1H).

In analoger Weise werden hergestellt:

| **Bsp.-Nr.** | **Struktur** | **MS (ESI pos.)** | **HPLC** |
|---|---|---|---|
| **X** | 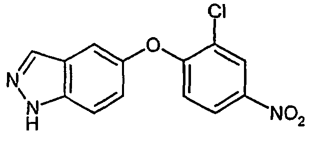 | m/z = 290 (M+H)⁺ | Methode 7: Rₜ = 4.56 min. |
| **XI** | 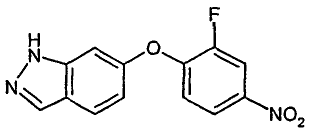 | m/z = 274 (M+H)⁺ | Methode 7: Rₜ = 4.40 min. |

### Beispiel XII

### 5-(4-Amino-2-fluorphenoxy)-1H-indazol

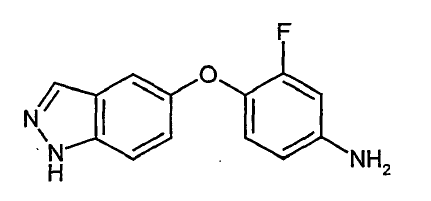

1.96 g (7.17 mmol) 5-(2-Fluor-4-nitrophenoxy)-1H-indazol (aus Beispiel IX) werden in 50 ml Ethanol gelöst. Dazu gibt man 0.16 g (0.71 mmol) Platin(IV)oxid und hydriert bei Raumtemperatur 2 Stunden unter Atmosphärendruck. Zur Aufarbeitung wird über Kieselgur abgesaugt, mit Ethanol gewaschen und das Filtrat eingeengt.
Ausbeute: 1.66 g (95 %)
MS (ESI pos.): m/z = 244 (M+H)⁺
¹H-NMR (DMSO-d₆, 300 MHz): δ = 5.26 (s, 2H), 6.39 (dd, 1H), 6.49 (dd, 1H), 6.88 (t, 1H), 7.00 (d, 1H), 7.08 (dd, 1H), 7.48 (d, 1H), 7.90 (s, 1H), 12.83 - 13.04, br. s, 1H).

In analoger Weise werden hergestellt:

| **Bsp.-Nr.** | **Struktur** | **MS (ESI pos.)** | **HPLC/LC-MS** |
|---|---|---|---|
| **XIII** | 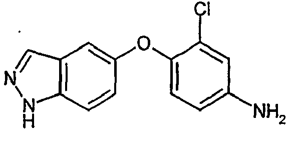 | m/z = 260 (M+H)⁺ | HPLC: Methode 7: Rₜ = 3.45 min. |
| **XIV** | 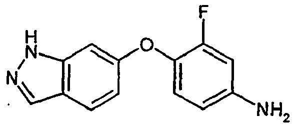 | m/z = 243 (M+H)⁺ | LC-MS: Methode 8: Rₜ = 2.87 min. |

### Beispiel XV

### 1H-Pyrrolo[2,3-b]pyridin-7-oxid

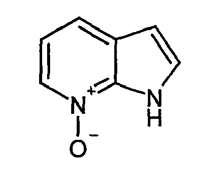

539.7 g (2.35 mol) 3-Chlorperbenzoesäure werden in 6.11 1 Dichlormethan gelöst und abgeschiedenes Wasser wird abgetrennt. Die organische Phase wird über Natriumsulfat getrocknet und auf 0°C gekühlt. Dann gibt man eine Lösung aus 163 g (1.38mol) 1H-Pyrrolo[2,3-b]pyridin in 1.00 1 Dichlormethan zu und lässt die Temperatur auf Raumtemperatur ansteigen. Nach 2 Stunden gibt man soviel Methanol zu, dass sich der gebildete Niederschlag wieder auflöst. Die Mischung wird über Kieselgel filtriert (Eluens: Dichlormethan/Methanol 95:5) und die Produktfraktionen nach Einengen am Hochvakuum getrocknet.
Ausbeute: 145 g (75 %)
HPLC (Methode 7): Rₜ = 2.02 min.
MS (ESI pos.): m/z = 135 (M+H)⁺, 152 (M+NH4)⁺, 269 (2M+H)⁺
¹H-NMR (DMSO-d₆, 200 MHz): δ= 6.58 (d, 1H), 7.07 (dd, 1H), 7.48 (d, 1H), 7.65 (d, 1H), 8.17 (d, 1H), 12.42 - 12.64 (br. s, 1H).

### Beispiel XVI

### 4-Nitro-1H-pyrrolo[2,3-b]pyridin-7-oxid

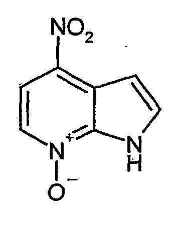

Eine Durchführung größerer Ansätze als der im folgenden beschriebene ist aufgrund der Ergebnisse der Differentialthermoanalyse nicht empfehlenswert.

Eine Lösung von 20.0 g (149 mmol) 1H-Pyrrolo[2,3-b]pyridin-7-oxid (aus Beispiel XV) in 160 ml Trifluoressigsäure wird auf Raumtemperatur gekühlt. Anschließend tropft man langsam 69.3 ml 65%ige Salpetersäure zu und lässt über Nacht bei Raumtemperatur rühren. Man gießt auf Eis und stellt mit 45%iger Natriumhydroxid-Lösung einen pH-Wert von 8-9 ein. Der Niederschlag wird abgesaugt und mit Wasser gewaschen. Es werden die Rohprodukte aus 4 Ansätzen der beschriebenen Größe und einem analog durchgeführten 13 g-Ansatz vereinigt und gemeinsam gereinigt. Die Rohprodukte werden in Wasser aufgeschlämmt und mit 2N Natriumhydroxidlösung auf pH 8-9 eingestellt. Nach 10 min Rühren wird der Niederschlag abgesaugt und im Hochvakuum getrocknet.
Ausbeute: 29.7 g (24 %)
HPLC (Methode 7): Rₜ = 3.02 min.
MS (ESI pos.): m/z = 180 (M+H)⁺, 197 (M+NH4)⁺, 359 (2M+H)⁺
¹H-NMR (DMSO-d₆, 200 MHz): δ = 7.03 (d, 1H), 7.80 (d, 1H), 8.03 (d, 1H), 8.31 (d, 1H), 13.22 - 13.41 (br. s, 1H).

### Beispiel XVII

### 4-Amino-1H-pyrrolo[2,3-b]pyridin

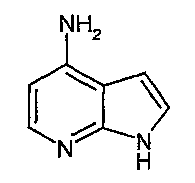

Zu einer Lösung aus 3.00 g (16.8 mmol) 4-Nitro-1H-pyrrolo[2,3-b]pyridin-7-oxid (aus Beispiel XVI) in 225 ml Essigsäure wird 9.35 g (167 mmol) Eisenpulver zugegeben und 2 Stunden unter Rückfluss erhitzt. Die Rohmischungen zweier solcher Ansätze werden vereinigt und gemeinsam weiter aufgearbeitet. Der Feststoff wird abgetrennt und mit 50 ml Essigsäure und 100 ml Tetrahydrofuran gewaschen. Das Filtrat wird am Rotationsverdampfer zur Trockne eingeengt. Der Rückstand wird mit 50 ml Wasser verdünnt und die Lösung mit 45%iger Natriumhydroxid-Lösung alkalisch gestellt. Anschließend wird mit Dichlormethan versetzt und die Mischung über Kieselgur abgesaugt. Das Filtrat wird sechsmal mit je 100 ml Dichlormethan extrahiert, die organische Phase über Natriumsulfat getrocknet und am Rotationsverdampfer zur Trockne eingeengt. Der Rückstand wird im Hochvakuum getrocknet. Zur weiteren Reinigung wird mit Tetrahydrofuran verrieben und der Feststoff abgesaugt. Das Filtrat wird eingeengt, der Rückstand in Dichlormethan aufgenommen, getrocknet und eingeengt.
Ausbeute: 3.5 g (78 %)
MS (ESI pos.): m/z = 134 (M+H)⁺, 267 (2M+H)⁺
¹H-NMR (DMSO-d₆, 200 MHz): δ = 6.05 (s, 2H), 6.09 (d, 1H), 6.47 (d, 1H), 7.02 (d, 1H), 7.70 (d, 1H), 10.90 - 11.28 (br. s, 1H).

### Beispiel XVIII

### 4-Chlor-1H-pyrrolo[2,3-b]pyridin

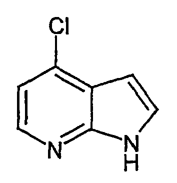

750 mg (5.63 mmol) 4-Amino-1H-pyrrolo[2,3-b]pyridin (aus Beispiel XVII) werden in einer Mischung aus 67.5 ml Eisessig und 0.67 ml konzentrierter Schwefelsäure gelöst. Die Mischung wird auf 12°C gekühlt. Dazu tropft man langsam 3.67 ml (3.20 g, 117 mmol) Isopentylnitrit und lässt 3 Stunden bei 12°C rühren. Nun gibt man diese Lösung zu einer 50°C warmen Suspension aus 6.07 g (61.4 mmol) Kupfer(I)chlorid in 34 ml konzentrierter Salzsäure und erhitzt anschließend 30 min auf 80-90°C. Man lässt auf RT abkühlen und über Nacht rühren. Zur Aufarbeitung engt man die Reaktionslösung ein, stellt mit 1N wässriger Natriumhydroxid-Lösung alkalisch, saugt über Kieselgur die ausgefallenen Kupfersalze ab und extrahiert 3x mit Ethylacetat. Die organische Phase wird über Magnesiumsulfat getrocknet und am Rotationsverdampfer eingeengt.
Ausbeute: 548 mg (64 %)
LC-MS (Methode 5): Rₜ = 3.21 min.
MS (ESI pos.): m/z = 153 (M+H)⁺
¹H-NMR (DMSO-d₆, 200 MHz): δ = 6.44 - 6.55 (m, 1H), 7.20 (d, 1H), 7.60 (t, 1H), 8.10 - 8.27 (m, 1H), 12.05 (br. s, 1H).

Die zuvor beschriebene Verbindung (Beispiel XVIII) kann alternativ auch nach folgendem Verfahren hergestellt werden:

### Beispiel XVIII (Alternative Herstellungsmethode)

### 4-Chlor-1H-pyrrolo[2,3-b]pyridin

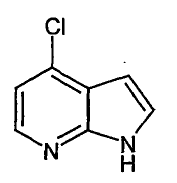

100 mg (0.76 mmol) 1H-Pyrrolo[2,3-b]pyridin-7-oxid (aus Beispiel XV) werden in 3 ml Phosphoroxychlorid so lange zum Rückfluss erhitzt, bis sich eine klare Lösung bildet. Nach Abkühlen auf Raumtemperatur wird die Reaktionsmischung vorsichtig mit Eis hydrolysiert. Man macht mit Ammoniak alkalisch und extrahiert mehrfach mit Ethylacetat. Die organische Phase wird mit Wasser und gesättigter Natriumchlorid-Lösung gewaschen. Nach Trocknen über Magnesiumsulfat wird das Lösungsmittel im Vakuum entfernt. Man erhält ein gelbes Öl, welches mittels präparativer HPLC gereinigt wird.
Ausbeute: 210 mg (73 %)
LC-MS (Methode 8): Rₜ = 2.9 min.
MS (ESI pos.): m/z = 153 (M+H)⁺
¹H-NMR (DMSO-d₆, 300 MHz):δ = 6.51 (dd, 1H), 7.20 (d, 1H), 7.60 (t, 1H), 8.17 (d, 1H), 12.05 (s, 1H).

### Beispiel XIX

### 3-Fluor-4-(1H-pyrrolo[2,3-b]pyridin-4-yloxy)anilin

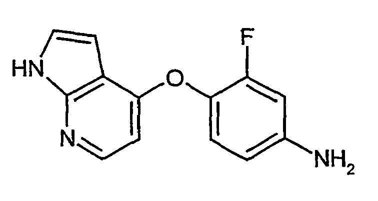

543 mg (3.56 mmol) 4-Chlor-1H-pyrrolo[2,3-b]pyridin (aus Beispiel XVIII, 905 mg (7.12 mmol) 3-Fluor-4-hydroxyanilin und 399 mg (7.12 mmol) gepulvertes Kaliumhydroxid werden 8 Stunden auf 260°C erhitzt. Man gibt nochmals 905 mg (7.12 mmol) 3-Fluor-4-hydroxyanilin und 200 mg (3.56 mmol) gepulvertes Kaliumhydroxid zu und lässt weitere 8 h bei 260°C reagieren. Zur Aufarbeitung kühlt man auf Raumtemperatur ab, verdünnt mit Wasser und extrahiert dreimal mit Ethylacetat. Die organische Phase wird über Magnesiumsulfat getrocknet und am Rotationsverdampfer eingeengt. Der Rückstand wird über präparative HPLC gereinigt. Die laut LC-MS 39% Produkt enthaltenden Fraktionen werden ohne weitere Reinigung in der nächsten Reaktion eingesetzt.

Die zuvor beschriebene Verbindung (Beispiel XIX) kann alternativ auch nach folgendem Verfahren hergestellt werden:

### Beispiel XIX (Alternative Herstellungsmethode)

### 3-Fluor-4-(1H-pyrrolo[2,3-b]pyridin-4-yloxy)anilin

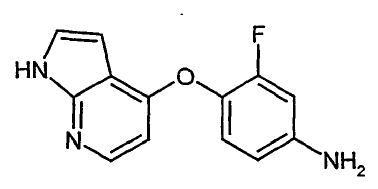

3.2 g (11.5 mmol) {4-[(6-Chlor-1H-pyrrolo[2,3-b]pyridin-4-yl)oxy]-3-fluor-phenyl}-amin (aus Beispiel XXXI) werden in Ethanol bei 50°C gelöst. Dann läßt man die Lösung auf RT abkühlen und gibt 2.45 g (2.30 mmol) 10% Palladium auf Aktivkohle hinzu. Das Gemisch wird über Nacht unter 2 bar Wasserstoff-Druck hydriert. Das Palladium wird anschließend über Kieselgur abgesaugt, mit Ethanol nachgewaschen und das Filtrat eingeengt.
Ausbeute: 2.5 g (89 %)
LC-MS (Methode 8): Rₜ = 2.43 min.
MS (ESI pos.): m/z = 244 (M+H)⁺
¹H-NMR (DMSO-d₆, 200 MHz): δ = 5.45 (mc, 2H), 6.25 (mc, 2H), 6.40-6.55 (br. 2H), 7.05 (t, 1H), 7.33 (mc, 1H), 8.25 (d, 1H), 11.69 (s, 1H).

### Beispiel XX

### 2-Fluor-4-(1H-pyrrolo[2,3-b]pyridin-4-yloxy)anilin

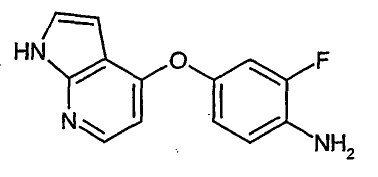

100 mg (660 µmol) 4-Chlor-1H-pyrrolo[2,3-b]pyridin (aus Beispiel XVIII), 166 mg (1.31 mmol) 2-Fluor-4-hydroxyanilin und 73.5 mg (1.31 mmol) gepulvertes Kaliumhydroxid werden 8 Stunden auf 260°C erhitzt. Zur Aufarbeitung kühlt man auf Raumtemperatur ab, verdünnt mit Wasser und extrahiert dreimal mit Ethylacetat. Die organische Phase wird über Magnesiumsulfat getrocknet und am Rotationsverdampfer eingeengt. Der Rückstand wird durch präparative HPLC gereinigt.
Ausbeute: 11 mg (6.7 %)
LC-MS (Methode 6): Rₜ = 2.37 min.

### Beispiel XXI

### 1-Benzofuran-4-ol

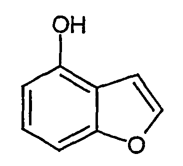

3.76 g (27.6 mmol) 6,7-Dihydro-1-benzofuran-4(5H)-on (hergestellt nach *Tetrahedron Lett.* 1994, 35, 6231) werden mit 10% Palladium auf Aktivkohle in 34 ml Decalin und 6 ml Dodecen über Nacht auf 200°C im Metallbad erhitzt. Es wird auf 80°C abgekühlt, mit Ethanol versetzt und über Celite abfiltriert. Das Celite wird zweimal mit Ethanol gewaschen und das Filtrat eingeengt. Der noch Decalin- und Dodecen-haltige Rückstand wird mit Petrolether versetzt und im Eisbad abgekühlt. Es setzt sich ein öliger Niederschlag ab. Das Lösungsmittel wird abdekantiert und das Öl über eine präparative HPLC gereinigt.
Ausbeute: 180 mg (5 %)
LC-MS (Methode 5): Rₜ = 3.1 min.

### Beispiel XXII

### 4-(2-Fluor-4-nitrophenoxy)-1-benzofuran

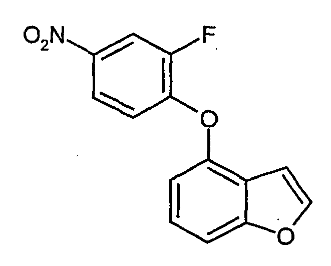

2.40 g (5.37 mmol) 1-Benzofuran-4-ol (aus Beispiel XXI), werden mit 0.90 g (5.64 mmol) 3,4-Difluornitrobenzol und 1.48 g (10.7 mmol) Kaliumcarbonat in 20 ml wasserfreiem Dimethylformamid suspendiert und fünf Stunden bei 50°C gerührt. Anschließend wird die Reaktionslösung mit Wasser verdünnt und zweimal mit Ethylacetat extrahiert. Die vereinten organischen Phasen werden über Natriumsulfat getrocknet. Das Lösungsmittel wird im Vakuum entfernt. Das Rohprodukt wird über eine Kieselgelsäule aufgereinigt (Fließmittel: Cyclohexan:Ethylacetat (10:1)).
Ausbeute: 254 mg (38 %)
LC-MS (Methode 6): Rₜ = 4.14 min.
MS (ESI pos.): m/z = 291 (M+NH₄)⁺

### Beispiel XXIII

### 4-(1-Benzofuran-4-yloxy)-3-fluorphenylamin

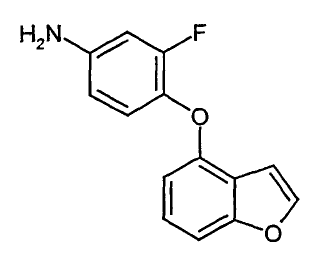

150 mg (0.55 mmol) 4-(2-Fluor-4-nitrophenoxy)-1-benzofuran (aus Beispiel XXII) werden in 5 ml Ethanol/Tetrahydrofuran (1:1) unter Argon vorgelegt, es wird Platin(IV)oxid zugegeben und 2 Stunden bei Normaldruck hydriert. Die Suspension wird über Celite abfiltriert, mit Ethanol gewaschen und einrotiert.
Ausbeute: 33 mg (25 %)
LC-MS (Methode 2): Rₜ = 3.7 min.
MS (ESI pos.): m/z = 261 (M+NH₄)⁺
¹H-NMR (DMSO-d₆, 200 MHz): δ = 6.49 (m, 2H), 6.60 (d, 1H), 6.84 (dd, 1H), 7.00 (t, 1H), 7.24 (m, 2H), 7.95 (d, 1H).

### Beispiel XXIV

### 1H-Indazol-4-ol

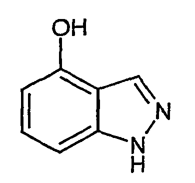

500 mg (375 mmol) 1H-Indazol-4-ylamin (hergestellt gemäß *J. Chem. Soc.* 1955, 2412, 2419) werden in 10%iger Schwefelsäure bei 180°C im Autoklaven bei Eigendruck über Nacht gerührt. Die Reaktionslösung wird auf Raumtemperatur abgekühlt, mit 1N Natriumhydroxid-Lösung neutralisiert und von den Salzen abfiltriert. Das Filtrat wird zur Trockne eingeengt.
Ausbeute: 480 mg (95%)
LC-MS (Methode 6): Rₜ = 1.22 min.
MS (ESI pos.): m/z = 13 5 (M+H)⁺
¹H-NMR (DMSO-d₆, 300 MHz): δ =6.38 (d, 1H), 6.92 (d, 1H), 7.11 (t, 1H), 8.0 (s, 1H), 9.8 (s, 1H), 12.8 (s, 1H).

### Beispiel XXV

### 4-(2-Fluor-4-nitrophenoxy)-1H-indazol

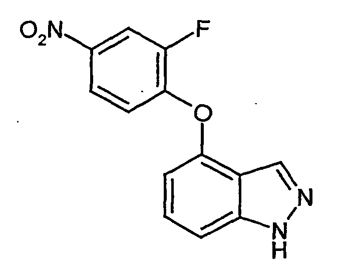

100 mg (0.75 mmol) 1H-Indazol-4-ol (aus Beispiel XXIV), werden mit 94 mg (0.64 mmol) 3,4-Difluornitrobenzol und 103 mg (0.75 mmol) Kaliumcarbonat in 2 ml wasserfreiem Dimethylformamid suspendiert und 5 Stunden bei 50°C gerührt. Anschließend wird die Reaktionslösung mit Wasser verdünnt und 2x mit Ethylacetat extrahiert. Die vereinigten organischen Phasen werden über Natriumsulfat getrocknet. Das Lösungsmittel wird im Vakuum entfernt. Das Rohprodukt wird mittels präparativer HPLC gereinigt.
Ausbeute: 78 mg (38 %)
LC-MS (Methode 6): Rₜ = 3.5 min.
MS (ESI pos.): m/z = 274 (M+H)⁺
¹H-NMR (DMSO-d₆, 300 MHz):δ = 6.79 (d, 1H), 7.20 (t, 1H), 7.41 (m, 2H), 7.94 (s, 1H), 8.07 (m, 1H), 8.36 (dd, 1H), 13.39 (s, 1H).

### Beispiel XXVI

### 3-Fluor-4-(1H-indazol-4-yloxy)anilin

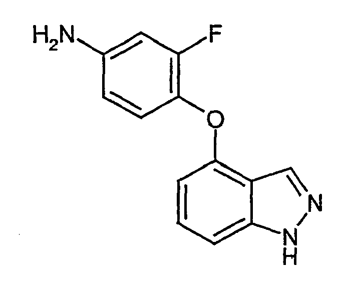

60 mg (0.22 mmol) 4-(2-Fluor-4-nitrophenoxy)-1H-indazol (aus Beispiel XXV) werden in 5 ml Ethanol/Tetrahydrofuran (1:1) unter Argon vorlegt, 9.97 mg (0.04 mmol) Platin(IV)oxid werden zugeben und 2 Stunden bei Normaldruck hydriert. Die Suspension wird über Celite abfiltriert, mit Ethanol gewaschen und im Vakuum eingeengt.
Ausbeute: 50 mg (94 %)
LC-MS (Methode 6): Rₜ = 2.9 min.
MS (ESI pos.): m/z = 244 (M+H)⁺
¹H-NMR (DMSO-d₆, 300 MHz):δ = 5.36 (s, 2H), 6.24 (d, 1H), 6.42 (dd, 1H), 6.51 (dd, 1H), 7.0 (t, 1H), 7.17 (m, 2H), 7.88 (s, 1H), 13.13 (s, 1H).

### Beispiel XXVII

### 3-Oxo-3-(4-pyridinyl)propansäureethylester

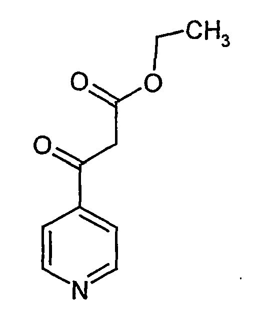

25 g (203 mmol) Isonicotinsäure, 35.12 g (243.7 mmol) 2,2-Dimethyl-1,3-dioxolan-4,6-dion und 49.6 g (406 mmol) 4-Dimethylaminopyridin werden in 300 ml Dichlormethan vorgelegt und auf 0°C gekühlt. Es wird eine 1N Lösung von 46.1 g (223.4 mmol) 1,3-Dicydohexylcarbodiimid in Dichlormethan zugetropft. Es wird 2 Stunden bei Raumtemperatur nachgerührt. Der entstandene Niederschlag wird abfiltriert und mit Dichlormethan nachgewaschen. Das Filtrat wird im Vakuum eingeengt. Der Rückstand wird in 1200 ml Ethanol gelöst und mit einer Lösung aus 96.6 g (507.7 mmol) p-Toluolsulfonsäure-monohydrat in 300 ml Ethanol versetzt und eine Stunde unter Rückfluss gerührt. Nach dem Abkühlen wird das Ethanol im Vakuum abgezogen. Der Rückstand wird in 1000 ml Ethylacetat und 900 ml Wasser aufgenommen und in der Hitze gelöst. Die organische Phase wird abgetrennt, mit 600 ml gesättigter Natriumhydrogencarbonatlösung und gesättigter Natriumchlorid-lösung gewaschen und über Natriumsulfat getrocknet. Es wird im Vakuum eingeengt. Das Rohprodukt wird über eine Kieselgel-Fritte mit Dichlormethan-Methanol 10:1 filtriert. Da die wässrige Phase noch Produkt enthält, wird diese mit Dichlormethan extrahiert, über Natriumsulfat getrocknet und im Vakuum eingeengt. Das Rohprodukt wird über eine Kieselgel-Fritte mit Dichlormethan-Methanol 10:1 filtriert.
Insgesamt erhält man 25.9 g (42 % d. Th.) Produkt.
LC-MS (Methode 3): Rₜ = 2.40 min.
MS (ESIpos): m/z = 194 (M+H)⁺
¹H-NMR (300 MHz, DMSO-d₆): δ = 1.17 (t, 3H), 4.12 (q, 2H), 4.25 (s, 2H), 7.82 (dd, 2H), 8.83 (dd, 2H).

### Beispiel XXVIII

### 2-Amino-6-(4-pyridinyl)-4-pyrimidinol

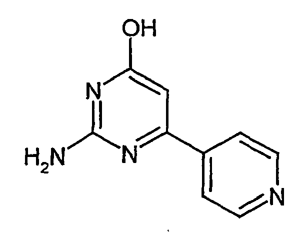

25 g (81.52 mmol) der Verbindung (aus Beispiel XXVII) und 13.22 g (73.37 mmol) Guanidiniumcarbonat werden in 250 ml Ethanol gelöst, mit 2.5 ml (29.76 mmol) konzentrierter Salzsäure versetzt und über Nacht unter Rückfluss gerührt. Nach dem Abkühlen wird der Niederschlag abgesaugt, mit Ethanol nachgewaschen und im Hochvakuum getrocknet. Der Feststoff wird mit 250 ml 1N Natriumhydroxidlösung versetzt und 2 Stunden unter Rückfluss gerührt. Nach dem Abkühlen wird mit konzentrierter Essigsäure sauer gestellt, das ausgefallene Produkt abgesaugt und mit Diethylether nachgewaschen. Nach dem Trocknen im Hochvakuum erhält man 12.52 g (82 % d. Th.) Produkt.
LC-MS (Methode 4): Rₜ = 0.30 min.
MS (ESIpos): m/z =189 (M+H)⁺
¹H-NMR (300 MHz, DMSO-d₆): δ = 6.23 (s, 1H), 6.89 (br.s, 2H), 7.86 (dd, 2H), 8.64 (dd, 2H).

### Beispiel XXIX

### 4-Chlor-6-(4-pyridinyl)-2-pyrimidinamin

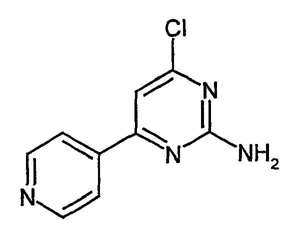

32 g (170.04 mmol) der Verbindung (aus Beispiel XXVIII) werden in 87.1 ml (0.93 mmol) Phosphorylchlorid gelöst. Es werden 2.80 ml (22.11 mmol) N,N-Dimethylanilin langsam zugetropft und eine Stunde bei 100°C gerührt. Anschließend wird die Reaktionslösung noch 2 Stunden bei Raumtemperatur gerührt. Das Phosphorylchlorid wird im Vakuum abrotiert. Der Rückstand wird mit Wasser-Dichlormethan 9:1 versetzt und für 5 Minuten aufgekocht. Dann wird mit gesättigter Natriumhydrogencarbonatlösung neutralisiert, das Produkt abgesaugt und im Hochvakuum getrocknet.
Ausbeute: 18.8 g (48 %)
LC-MS (Methode 4): Rₜ = 1.08 min.
MS (ESIpos): m/z = 207 (M+H)⁺
¹H-NMR (300 MHz, DMSO-d₆): δ = 7.31 (br.s, 2H), 7.38 (s, 1H), 8.00 (dd, 2H), 8.74 (dd, 2H).

### Beispiel XXX

### 6-Chlor-4-nitro-1H-pyrrolo[2,3-b]pyridin

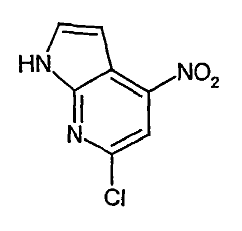

5.00 g (27.9 mmol) 4-Nitro-1H-pyrrolo[2,3-b]pyridin-7-oxid (aus Beispiel XVI) und 11.8 ml (55.8 mmol) Hexamethyldisilazan werden in 290 ml THF unter ArgonAtmosphäre vorgelegt. 10.8 ml (139.6 mmol) Chlorameisensäuremethylester werden bei RT zugegeben. Die Lösung wird über Nacht unter RT gerührt. Die Reaktionslösung wird über eine Kieselgelkartusche filtriert und mit DCM/Methanol 10:1 nachgewaschen.
Ausbeute: 2.8 g (70 %)
LC-MS (Methode 8): Rₜ = 2.74 min.
MS (ESI pos.): m/z = 198 (M+H)⁺
¹H-NMR (DMSO-d₆, 200 MHz): δ = 7.00 (mc, 1H), 7.97 (s, 1H), 8.00 (t, 1H), 12.79 (s, 1H).

### Beispiel XXXI

### {4-[(6-Chlor-1H-pyrrolo[2,3-b]pyridin-4-yl)oxy]-3-fluorphenyl}amin

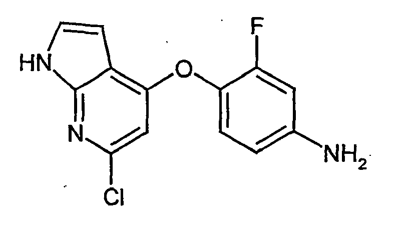

4-Amino-2-fluorphenol (0.77 g, 6.07 mmol) wird in DMF gelöst. Kalium-tert-butylat (0.68 g, 6.07 mmol) wird zugegeben und das Gemisch wird 30 Minuten bei Raumtemperatur gerührt. Dann werden nacheinander gepulvertes Kaliumcarbonat (0.35 g, 2.53 mmol) und 1 g (5.06 mmol) 6-Chlor-4-nitro-1H-pyrrolo[2,3-b]pyridin (aus Beispiel XXX) dazugegeben. Das Gemisch wird 12 Stunden bei 120°C gerührt. Nach dem Abkühlen wird es mit Essigsäureethylester (200 ml) verdünnt. Die Suspension wird über Celite® abgesaugt und mit Essigsäureethylester nachgewaschen. Die Lösung wird nacheinander mit wässriger Natriumhydrogencarbonatlösung und Natriumchloridlösung ausgeschüttelt. Die organische Phase wird über wasserfreies Magnesiumsulfat getrocknet und eingeengt. Der Rückstand wird durch Säulenchromatographie gereinigt (Kieselgel 60, Eluens: DCM : Aceton = 5 : 1). Man erhält 0.95 g (67 % d. Th.) Produkt.
LC-MS (Methode 16): Rₜ = 1.99 min.
MS (ESIpos): m/z = 278 (M+H)⁺

### Beispiel XXXII

### 4-Amino-2,6-difluorphenol

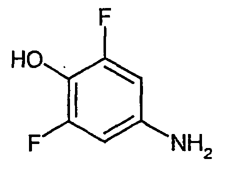

1.00 g (5.71 mmol) 2,6-Difluor-4-nitrophenol (hergestellt durch Nitrierung von 2,6-Difluorphenol nach Kirk, K.L.; *J. Heterocycl. Chem*. **1976**, *13*, 1253-1256) werden in 35 ml Ethanol vorgelegt. Man gibt 80 mg Palladium auf Kohle (10%) hinzu und hydriert 1.5 Stunden unter normalem Druck. Unter Eisbadkühlung werden 2 ml konz. Salzsäure zugetropft. Man filtriert vom Katalysator ab und entfernt das Lösungsmittel im Vakuum. Der Rückstand wird mit Diethylether und wenig Methanol suspendiert. Man saugt ab und wäscht mit Diethylether nach und erhält die Titelverbindung (Qiu, Jian; Stevenson, Scott H.; O'Beirne, Michael J.; Silverman, Richard B.; *J. Med. Chem.* **1999,** *42* (2), 329 - 332) als Hydrochlorid.
Ausbeute: 939 mg (91 %)
LC-MS (Methode 9): Rₜ = 0.29 min.
MS (ESI pos.): m/z = 146 (M+H)⁺
¹H-NMR (DMSO-d₆, 300 MHz): δ = 6.89 (mc, 2H), 4.5-9.0 (br. s, 3H), 10.1 (br. s, 1H).

### Beispiel XXXIII

### {4-[(6-Chlor-1H-pyrrolo[2,3-b]pyridin-4-yl)oxy]-3,5-difluorphenyl}amin

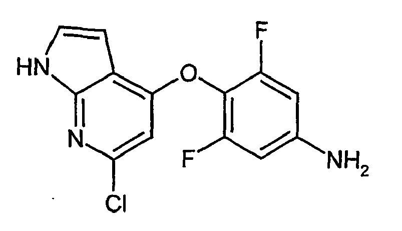

664 mg (3.36 mmol) 6-Chlor-4-nitro-1H-pyrrolo[2,3-b]pyridin (aus Beispiel XXX), 1.39 g (10.1 mmol) pulverisiertes Kaliumcarbonat und 877 mg (5.04 mmol) Natriumdithionit werden in 10 ml DMSO suspendiert. Die Mischung wird entgast, und 915 mg (5.04 mmol) 4-Amino-2,6-difluorphenol-Hydrochlorid (aus Beispiel XXXII) werden zugesetzt. Man erhitzt 4 Stunden auf 120°C. Nach Zugabe von Essigsäureethylester wird über Celite abgesaugt und mit Essigsäureethylester nachgewaschen. Das Filtrat wird dreimal mit ges. Natriumhydrogencarbonatlösung und mit ges. Natriumchloridlösung geschüttelt. Man trocknet über Natriumsulfat und entfernt das Lösungsmittel im Vakuum. Der Rückstand wird durch Säulenchromatographie gereinigt (Kieselgel 60, Eluens: DCM : Methanol = 50 : 1).
Ausbeute: 356 mg (36 %)
HPLC (Methode 7): Rₜ = 4.12 min.
MS (ESI pos.): m/z = 296 (M+H)⁺
¹H-NMR (DMSO-d₆, 400 MHz): δ = 5.84 (s, 2H), 6.28 (mc, 1H), 6.38 - 6.41 (m, 3H), 7.42 (mc, 1H), 12.02 (s, 1H).

### Beispiel XXXIV

### [3,5-Difluor-4-(1H-pyrrolo[2,3-b]pyridin-4-yloxy)phenyl]amin

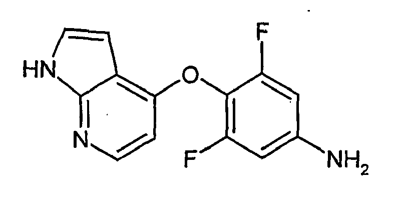

Analog Beispiel XIX (Alternativmethode) wird die Titelverbindung durch katalytische Hydrierung von 408 mg (1.38 mmol) {4-[(6-Chlor-1H-pyrrolo[2,3-b]pyridin-4-yl)oxy]-3,5-difluorphenyl}amin (aus Beispiel XXXIII) gewonnen.
Ausbeute: 360 mg (100 %)
LC-MS (Methode 10): Rₜ = 2.19 min.
MS (ESI pos.): m/z = 262 (M+H)⁺

### Beispiel XXXV

### 2-(4-Amino-2-chlorphenoxy)-6-fluorbenzonitril

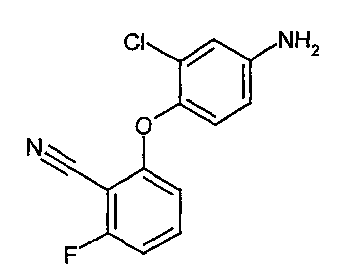

1.00 g (7.19 mmol) 2,6-Difluorbenzonitril werden in 10 ml Acetonitril vorgelegt. Man setzt 1.99 g (14.4 mmol) Kaliumcarbonat und 1.03 g (7.19 mmol) 3-Chlor-4-hydroxyanilin hinzu und erwärmt 45 min. auf Rückfluss. Man filtriert und wäscht mit Essigsäureethylester und DCM nach. Das Lösungsmittel wird im Vakuum entfernt und der Rückstand durch Säulenchromatographie (Kieselgel 60, Gradientensäule:
Eluens: DCM : Petrolether = 2 : 1, dann DCM).
Ausbeute: 1.13 g (60 %)
HPLC (Methode 7): Rₜ = 4.04 min.
MS (DCI): m/z = 280 (M+NH₄)⁺
¹H-NMR (DMSO-d₆, 200 MHz): δ = 5.53 (br. s, 2H), 6.51 (d, 1H), 6.59 (dd, 1H), 6.76 (d; 1H), 7.10 (d, 1H), 7.17 (t, 1H), 7.65 (dt, 1H).

### Beispiel XXXVI

### 4-(4-Amino-2-chlorphenoxy)-1H-indazol-3-amin

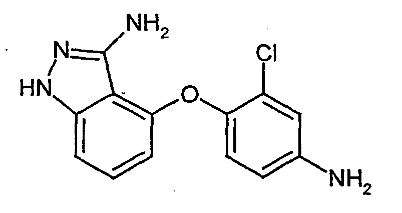

60 mg (0.23 mmol) 2-(4-Amino-2-chlorphenoxy)-6-fluorbenzonitril (aus Beispiel XXXV) werden mit 17 mg (0.34 mmol) Hydrazinhydrat in 0.2 ml 1-Butanol im Druckgefäß über Nacht auf 120°C erhitzt. Dann wird das Lösungsmittel im Vakuum entfernt, mit DCM suspendiert und abgesaugt.
Ausbeute: 47 mg (73 %)
HPLC (Methode 7): Rₜ = 3.05 min.
MS (ESI pos.): m/z = 275 (M+H)⁺
¹H-NMR (DMSO-d₆, 200 MHz): δ = 5.03 (br. s, 2H), 5.37 (br. s, 2H), 5.78 (d, 1H), 6.58 (dd, 1H), 6.74 (d, 1H), 6.82 (d, 1H), 6.96 - 7.05 (m, 2H), 11. 52 (br. s, 1H).

### Beispiel XXXVII

### 3-Methyl-1H-pyrrolo[2,3-b]pyridin-7-oxid

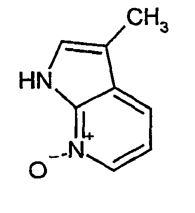

Analog zu Beispiel XV wird die Titelverbindung durch Oxidation von 11 g (54.1 mmol) 3-Methyl-1H-pyrrolo[2,3-b]pyridin (Hands, D.; Bishop, B.; Cameron, M.; Edwards, T.S.; Cottrell, I.F.; Wright, S.H.B.; *Synthesis* 1996 (7), 877-882) mit 24.2 g (108.2 mmol) 3-Chlorperbenzoesäure gewonnen.
Ausbeute: 5.4 g (67 %)
LC-MS (Methode 13): Rₜ = 1.19 min.
MS (ESI pos.): m/z = 149 (M+H)⁺
¹H-NMR (DMSO-d₆, 300 MHz): δ = 2.25 (m, 3H), 7.05 (m, 1H), 7.21 (s, 1H), 7.59 (m, 1H), 8.10 (s, 1H), 12.06 (s, 1H).

### Beispiel XXXVIII

### 4-Chlor-3-methyl-1H-pyrrolo[2,3-b]pyridin

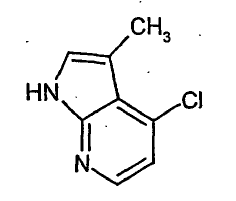

1.00 g (6.75 mmol) 3-Methyl-1H-pyrrolo[2,3-b]pyridin-7-oxid (aus Beispiel XXXVII) werden in 5 ml Phosphorylchlorid suspendiert. Dann werden 2 ml Chloroform dazugegeben und das Gemisch wird über Nacht unter Rückfluss erhitzt. Man lässt auf RT abkühlen und gießt auf Essigsäureethylester/Eiswasser. Anschließend gibt man festes Natriumcarbonat dazu. Die Phasen werden getrennt und die wässrige Phase mit Essigsäureethylester gewaschen. Die organischen Phasen werden über Natriumsulfat getrocknet und eingeengt. Der Rückstand wird durch Säulenchromatographie gereinigt (Kieselgel 60, Eluens: Cyclohexan : Methanol = 4: 1).
Ausbeute: 200 mg (18 %)
LC-MS (Methode 13): Rₜ = 2.05 min.
¹H-NMR (DMSO-d₆, 200 MHz): δ = 2.41 (m, 3H), 7.10 (d, 1H), 7.31 (s, 1H), 8.07 (d, 1H), 12.44 (s, 1H).

### Beispiel XXXIX

### 4-Chlor-3-methyl-1H-pyrrolo[2,3-b]pyridin-7-oxid

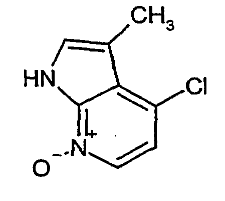

Analog zu Beispiel XV wird die Titelverbindung durch Oxidation von 898 mg (5.39 mmol) 4-Chlor-3-methyl-1H-pyrrolo[2,3-b]pyridin (aus Beispiel XXXVIII) mit 2.42 g (10.78 mmol) 3-Chlorperbenzoesäure gewonnen.
Ausbeute: 688 mg (70 %)
LC-MS (Methode 13): Rₜ = 1.75 min.
MS (ESI pos.): m/z = 183 (M+H)⁺
¹H-NMR (DMSO-d₆, 200 MHz): δ = 2.41 (m, 3H), 7.10 (d, 1H), 7.30 (s, 1H), 8.07 (d, 1H), 12.44 (s, 1H).

### Beispiel XL

### 1-Acetyl-4,6-dichlor-3-methyl-1H-pyrrolo[2,3-b]pyridin

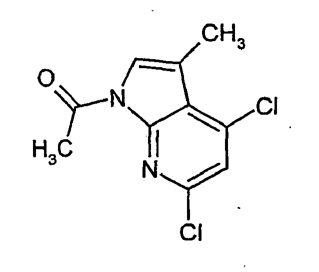

Analog zu Beispiel XXX wird die Titelverbindung aus 688 mg (3.77 mmol) 4-Chlor-3-methyl-1H-pyrrolo[2,3-b]pyridin-7-oxid (aus Beispiel XXXIX) und 1.78 g (18.84 mmol) Chlorameisensäuremethylester und 0.61 g (3.77 mmol) Hexamethyldisilazan gewonnen.
Ausbeute: 420 mg (22 %)
LC-MS (Methode 13): Rₜ = 2.44 min.
MS (ESI pos.): m/z = 259 (M+H)⁺
¹H-NMR (DMSO-d₆, 200 MHz): δ = 3.99 (s, 3H), 2.40 (m, 3H), 7.61 (s, 1H), 7.77 (d, 1H).

### Beispiel XLI

### {4-[(6-Chlor-3-methyl-1H-pyrrolo[2,3-b]pyridin-4-yl)oxy]-3-fluorphenyl}amin

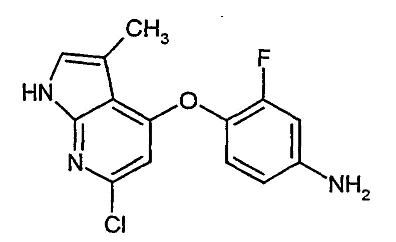

300 mg (1.16 mmol) 1-Acetyl-4,6-dichlor-3-methyl-1H-pyrrolo[2,3-b]pyridin (aus Beispiel XL) und 320 mg (2.32 mmol) pulverisiertes Kaliumcarbonat werden in 9 ml DMSO suspendiert. Die Mischung wird entgast und 442 mg (3.48 mmol) 4-Amino-2-fluorphenol werden zugesetzt. Man erhitzt 4 Stunden auf 120°C. Nach Zugabe von Essigsäureethylester wird über Celite abgesaugt und mit Essigsäureethylester nachgewaschen. Das Filtrat wird dreimal mit ges. Natriumhydrogencarbonatlösung und mit ges. Natriumchloridlösung geschüttelt. Man trocknet über Natriumsulfat und entfernt das Lösungsmittel im Vakuum. Der Rückstand wird durch Säulenchromatographie gereinigt (Kieselgel 60, Eluens: DCM : Methanol = 50 : 1).
Ausbeute: 142 mg (42 %)
LC-MS (Methode 13): Rₜ = 2.32 min.
MS (ESI pos.): m/z = 292 (M+H)⁺

### Beispiel XLII

### {3-Fluor-4-[(3-methyl-1H-pyrrolo[2,3-b]pyridin-4-yl)oxy]phenyl}amin

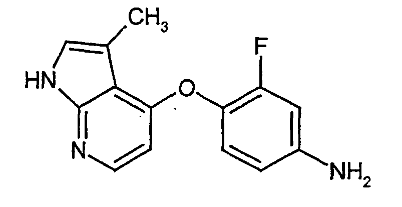

Analog zu Beispiel XIX (Alternativmethode) wird die Titelverbindung durch katalytische Hydrierung von 142 mg (0.49 mmol) {4-[(6-Chlor-3-methyl-1H-pyrrolo[2,3-b]pyridin-4-yl)oxy]-3-fluorphenyl}amin (aus Beispiel XLI) gewonnen.
Ausbeute: 125 mg (100 %)
LC-MS (Methode 13): Rₜ = 1.58 min.

Analog zu Beispiel XLVII wird hergestellt:

| **Bsp.-Nr.** | **Struktur** | **MS** | **HPLC, LC-MS** |
|---|---|---|---|
| **XLIII** | 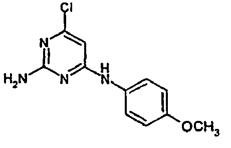 | MS (ESI pos.): m/z = 251 (M+H)⁺ | LC-MS (Methode 9): Rₜ = 2.92 min. |

### Beispiel XLIV

### N-[3-Fluor-4-({1-[(4-methylphenyl)sulfonyl]-1H-pyrrolo[2,3-b]pyridin-4-yl}oxy)phenyl]-4-methylphenylsulfonamid

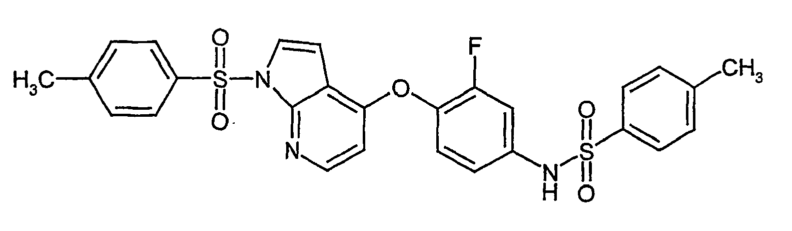

147 mg (0.60 mmol) der Verbindung aus Beispiel XIX werden in 10 ml THF gelöst, mit 48 mg (1.21 mmol) Natriumhydrid (in THF) versetzt und anschließend eine Stunde bei RT gerührt. Dann werden 242 mg (1.27 mmol) p-Toluolsulfonsäurechlorid dazugegeben und die Reaktionslösung für eine Stunde bei 60°C weitergerührt. Die Suspension wird über Celite filtriert und mit THF und etwas DCM/Methanol 10:1 nachgewaschen und einrotiert. Der Kolbenrückstand wird in DMSO aufgenommen und mittels einer RP-HPLC-Chromatographie (Gradient: Acetonitril/Wasser) gereinigt.
Ausbeute: 94 mg (28 %)
LC-MS (Methode 15): Rₜ = 2.72 min.
¹H-NMR (DMSO-d₆, 300 MHz): δ = 2.35 (s, 3H), 2.37 (s, 3H), 6.49-6.52 (m, 2H), 6.95 (m, 1H), 7.10 (m, 1H), 7.31 (t, 1H), 7.41 (m, 4H), 7.69 (m, 2H), 7.79 (d, 1H), 7.98 (d, 2H), 8.19 (d, 1H), 10.55 (s, 1H).

### Beispiel XLV

### N-[3-Fluor-4-({1-[(4-methylphenyl)sulfonyl]-2,3-dihydro-1H-pyrrolo[2,3-b]pyridin-4-yl}oxy)phenyl]-4-methylphenylsulfonamid

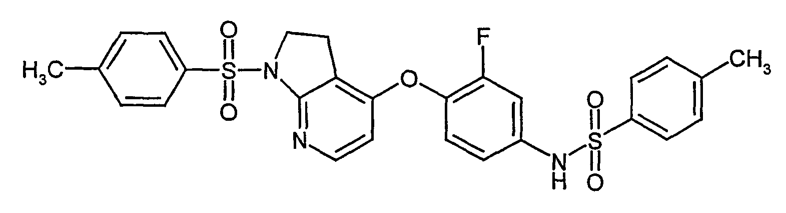

50 mg (0.09 mmol) N-[3-Fluor-4-({1-[(4-methylphenyl)sulfonyl]-1H-pyrrolo[2,3-b]pyridin-4-yl}oxy)phenyl]-4-methylphenylsulfonamid (aus Beispiel XLIV) werden in 30 ml Methanol gelöst und 48.2 mg (0.05 mmol) 10% Palladium auf Aktivkohle hinzugefügt. Das Gemisch wird über Nacht unter 3.5 bar Wasserstoff-Druck hydriert. Das Palladium wird anschließend über Kieselgur abgesaugt, mit Ethanol nachgewaschen und das Filtrat eingeengt.
Ausbeute: 48 mg (95 %)
LC-MS (Methode 9): Rₜ = 3.35 min.

### Beispiel XLVI

### [4-(2,3-Dihydro-1H-pyrrolo[2,3-b]pyridin-4-yloxy)-3-fluorphenyl]amin

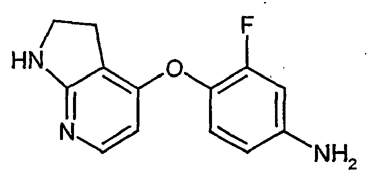

50 mg (0.09 mmol) N-[3-Fluor-4-({1-[(4-methylphenyl)sulfonyl]-2,3-dihydro-1H-pyrrolo[2,3-b]pyridin-4-yl}oxy)phenyl]-4-methylphenylsulfonamid (aus Beispiel XLV) werden in 1.00 ml Schwefelsäure (95%) gelöst und über Nacht bei RT gerührt. Die Reaktionslösung wird unter Eiskühlung mit 20%iger Natronlauge-Lösung neutralisiert und dreimal mit Essigsäureethylester extrahiert. Die organische Phase wird über wasserfreiem Magnesiumsulfat getrocknet und eingeengt.
Ausbeute: 20 mg (91 %)
LC-MS (Methode 8): Rₜ = 2.22 min.

### Beispiel XLVII

### 4-[(2-Amino-6-chlorpyrimidin-4-yl)amino]benzonitril

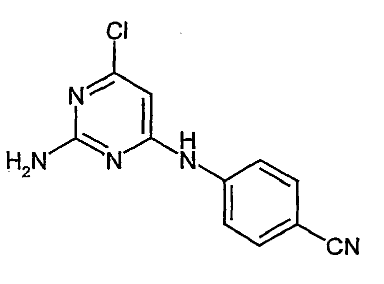

300 mg (1.83 mmol) 2-Amino-4,6-dichlorpyrimidin und 216 mg (1.83 mmol) 4-Aminobenzonitril werden in 6 ml Wasser suspendiert. Dazu gibt man 180 µl konzentrierte Salzsäure und lässt über Nacht bei 100°C rühren. Der Niederschlag wird abgesaugt.
Ausbeute: 450 mg (96 %)
LC-MS (Methode 9): Rₜ = 2.39 min.
MS (ESI pos.): m/z = 246 (M+H)⁺
¹H-NMR (DMSO-d₆, 200 MHz): δ = 4.98 (m, 2H), 6.14 (s, 1H), 7.69 - 7.99 (m, 4H), 9.99 (s, 1H).

Analog zu Beispiel XLVII werden hergestellt:

| **Bsp.-Nr.** | **Struktur** | **MS** | **HPLC, LC-MS** |
|---|---|---|---|
| **XLVIII** | 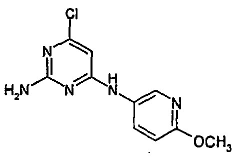 | | LC-MS (Methode 9): Rₜ = 2.09 min. |
| **XLIX** | 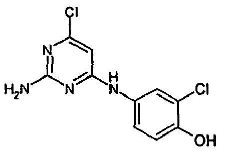 | MS (ESI pos.): m/z = 272 (M+H)⁺ | LC-MS (Methode 16): Rₜ = 1.66 min. |
| **L** | 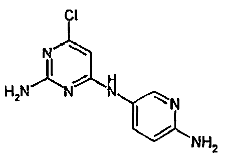 | MS (ESI pos.): m/z = 238 (M+H)⁺ | LC-MS (Methode 16): Rₜ = 0.95 min. |
| **LI** | 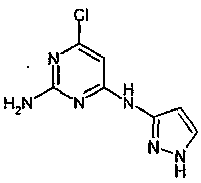 | MS (ESI pos.): m/z = 211 (M+H)⁺ | LC-MS (Methode 10): Rₜ = 2.10 min |

### Beispiel LII

### 4-(4-Amino-2-chlorphenoxy)-2-fluorbenzonitril

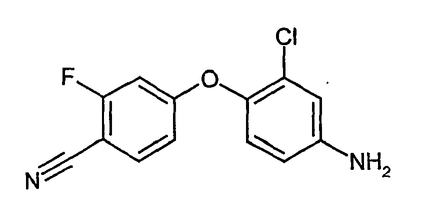

2.00 g (14.4 mmol) 2,4-Difluorbenzonitril, 2.06 g (14.4 mmol) 3-Chlor-4-hydroxyanilin und 3.97 g (28.8 mmol) Kaliumcarbonat werden in 20 ml Acetonitril eine Stunde auf RF erhitzt. Man setzt Essigsäureethylester zu, filtriert und entfernt das Lösungsmittel im Vakuum. Man reinigt durch Säulenchromatographie (Kieselgel 60) und erhält das Produkt, bei dem es sich um eine Regioisomerenmischung im Verhältnis 62:38 handelt.
Ausbeute: 2.55 g (68 %)
HPLC (Methode 7): Rₜ = 3.90 min (Hauptisomer), 4.01 min (Minderisomer).
MS (ESI pos.): m/z = 263 (M+H)⁺

### Beispiel LIII

### 6-(4-Amino-2-chlorphenoacy)-1-methyl-1H-indazol-3-amin

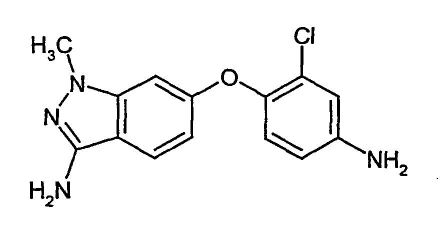

1.00 g (3.81 mmol) 4-(4-Amino-2-chlorphenoxy)-2-fluorbenzonitril (verunreinigt mit seinem Regioisomer; aus Beispiel LII) werden mit 1.05 g (22.8 mmol) Methylhydrazin in 4 ml 1-Butanol 5 Stunden auf RF erhitzt. Man entfernt flüchtige Bestandteile im Vakuum und reinigt den Rückstand durch präparative HPLC.
Ausbeute: 496 mg (45 %)
HPLC (Methode 7): Rₜ = 3.26 min.
MS (ESI pos.): m/z = 289 (M+H)⁺
¹H-NMR (DMSO-d₆, 200 MHz): δ = 3.57 (s, 3H), 5.33 (s, 2H), 5.37 (s, 2H), 6.47 - 6.59 (m, 3H), 6.72 (d, 1H), 6.91 (d, 1H), 7.58 (m, 1H).

### Beispiel LIV

### 6-(4-Amino-2-chlorphenoxy)-1H-indazol-3-amin

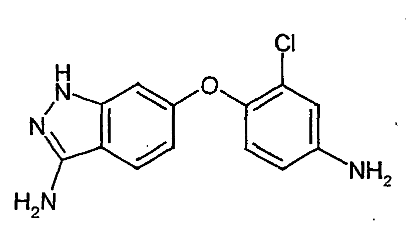

Die Titelverbindung wird analog zu Beispiel LIII aus 2.3 g (8.76 mmol) 4-(4-Amino-2-chlorphenoxy)-2-fluorbenzonitril (aus Beispiel LII) und Hydrazinhydrat synthetisiert.
Ausbeute: 750 mg (31 %)
HPLC (Methode 7): Rₜ = 3.24 min.
MS (ESI pos.): m/z = 275 (M+H)⁺
¹H-NMR (DMSO-d₆, 200 MHz): δ = 5.29 (br. s, 2H), 5.35 (br. s, 2H), 6.30 (d, 1H), 6.53 - 6.62 (m, 2H), 6.73 (d, 1H), 6.94 (d, 1H), 7.59 (d, 1H), 11.06 (s, 1H).

### Beispiel LV

### 3-(2-Amino-6-oxo-1,6-dihydropyrimidin-4-yl)benzonitril

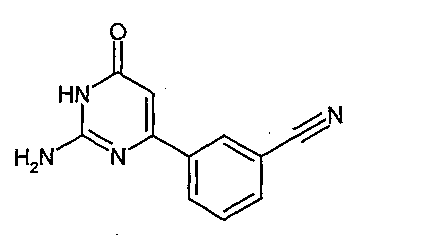

12.0 g (49.8 mmol) 3-(3-Cyanophenyl)-3-oxopropansäureethylester (Fevig, John M. et al.; *Bioorg.Med.Chem.Lett*. **2001,** *11* (5), 641 - 646) werden in 110 ml Ethanol gelöst. Man fügt 5.39 g (29.9 mmol) Guanidiniumcarbonat und 0.95 ml konz. Salzsäure hinzu und erhitzt über Nacht auf RF. Man läßt abkühlen, saugt die ausgefallenen Kristalle ab und wäscht mit Ethanol nach. Man erhält die Titelverbindung.
Ausbeute: 8.02 g (76 %)
HPLC (Methode 7): Rₜ = 2.99 min.
MS (ESI pos.): m/z = 213 (M+H)⁺
¹H-NMR (DMSO-d₆, 200 MHz): δ = 6.27 (s, 1H), 6.71 (br. s, 2H), 7.65 (t, 1H), 7.91 (d, 1H), 8.28 (d, 1H), 8.39 (s, 1H), 10.96 (s, 1H).

### Beispiel LVI

### 3-(2-Amino-6-chlorpyrimidin-4-yl)benzonitril

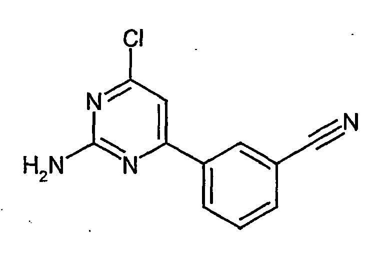

2.00 g (9.42 mmol) 3-(2-Amino-6-oxo-1,6-dihydropyrimidin-4-yl)benzonitril (aus Beispiel LV) werden in 10 ml Phosphorylchlorid suspendiert. Man tropft 180 mg (1.23 mmol) N,N-Diethylanilin innerhalb einer Stunde zu und heizt innerhalb einer weiteren Stunde auf 115°C. Dann wird für eine Stunde auf RF erhitzt. Man lässt auf RT abkühlen und gießt auf Eiswasser. Man extrahiert zweimal mit Essigsäureethylester und entfernt das Lösungsmittel im Vakuum. Der Rückstand wird in Wasser suspendiert. Man neutralisiert die Lösung unter Eiskühlung durch Zugabe konz. Natriumhydroxidlösung. Man setzt gesättigte Natriumcarbonatlösung zu und rührt über Nacht bei RT. Man saugt den Niederschlag ab und wäscht mit Wasser nach. Man erhält die Titelverbindung.
Ausbeute: 1.58 g (73 %)
LC-MS (Methode 9): Rₜ = 2.54 min. MS (ESI pos.): m/z = 231 (M+H)⁺
¹H-NMR (DMSO-d₆, 300 MHz): δ = 7.27 (br. s, 2H), 7.41 (s, 1H), 7.73 (t, 1H), 8.00 (dt, 1H), 8.42 (dt, 1H), 8.53 (t, 1H).

Analog zu Beispiel LVI werden hergestellt:

| **Bsp.-Nr.** | **Struktur** | **MS** | HPLC, LC-MS |
|---|---|---|---|
| **LVII** | 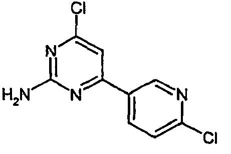 | MS (ESI pos.): m/z = 241 (M+H)⁺ | LC-MS (Methode 10): Rₜ = 2.98 min. |
| **LVIII** | 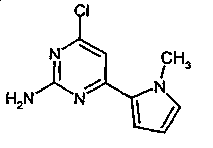 | MS (ESI pos.): m/z = 209 (M+H)⁺ | LC-MS (Methode 16): Rₜ = 2.02 min. |
| **LIX** | 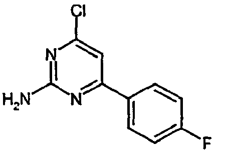 | MS (ESI pos.): m/z = 223 (M+H)⁺ | LC-MS (Methode 8): Rₜ = 3.52 min. |
| **LX** | 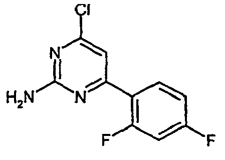 | MS (ESI pos.): m/z = 242 (M+H)⁺ | HPLC (Methode 7): Rₜ = 4.38 min. |
| **LXI** | 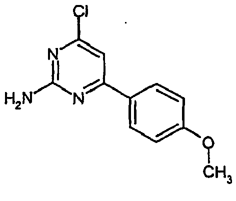 | MS (ESI pos.): m/z = 236 (M+H)⁺ | LC-MS (Methode 10): Rₜ = 2.80 min. |
| **LXII** | 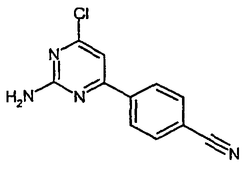 | MS (ESI pos.): m/z = 231 (M+H)⁺ | LC-MS (Methode 9): Rₜ = 2.54 min. |
| **LXIII** | 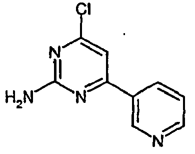 | MS (ESI pos.): m/z = 207 (M+H)⁺ | LC-MS (Methode 8): Rₜ = 2.12 min. |
| **LXIV** | 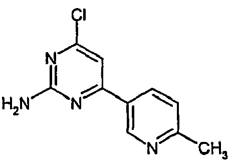 | MS (ESI pos.): m/z = 221 (M+H)⁺ | LC-MS (Methode 10): Rₜ= 2.15 min. |
| **LXV** | 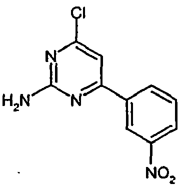 | MS (ESI pos.): m/z = 251 (M+H)⁺ | LC-MS (Methode 2): Rₜ = 3.40 min. |
| **LXVI** | 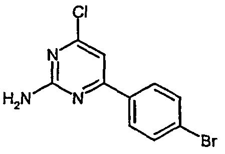 | MS (ESI pos.): m/z = 284 (M+H)⁺ | LC-MS (Methode 10): Rₜ = 3.60 min. |
| **LXVII** | 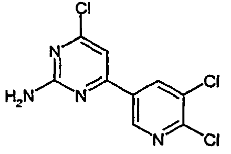 | MS (ESI pos.): m/z = 275 (M+H)⁺ | LC-MS (Methode 18): Rₜ = 3.48 min. |
| **LXVIII** | 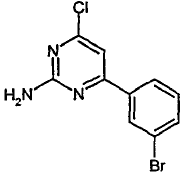 | MS (ESI pos.): m/z = 284 (M+H)⁺ | LC-MS (Methode 9): Rₜ = 4.21 min. |

### Beispiel LXIX

### Ethyl-(2-amino-6-chlorpyrimidin-4-yl)acetat

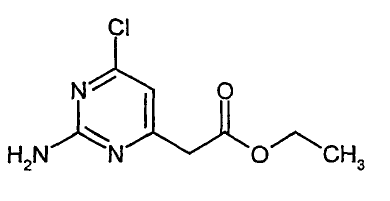

Die Titelverbindung wird analog zu Beispiel LVI in zwei Stufen aus 3-Diethyl-3-oxopentandioat synthetisiert.
Ausbeute: 394 mg (32 %)
LC-MS (Methode 10): Rₜ = 2.62 min.
MS (ESI pos.): m/z = 216 (M+H)⁺
¹H-NMR (DMSO-d₆, 300 MHz): δ = 1.18 (t, 3H), 3.60 (s, 2H), 4.09 (q, 2H), 6.65 (s, 1H), 7.11 (s, 2H).

### Beispiel LXX

### 4-(2-Amino-6-chlorpyrimidin-4-yl)buttersäure

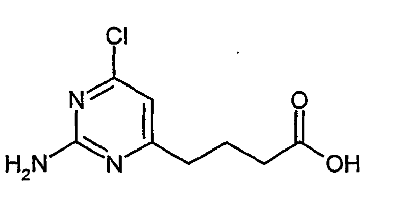

Die Titelverbindung wird analog zu Beispiel LVI in zwei Stufen aus 3-Oxoheptandisäurediethylester synthetisiert. Dabei wird während der zweiten Reaktionsstufe der Ester zur Carbonsäure hydrolysiert.
Ausbeute: 394 mg (32 %)
LC-MS (Methode 12): Rt = 2.46 min.
MS (ESI pos.): m/z = 216 (M+H)⁺
¹H-NMR (DMSO-d₆, 400 MHz): δ = 1.83 (tt, 2H), 2.23 (t, 2H), 2.50 (t, 2H), 6.65 (s, 1H), 7.02 (s, 2H), 12.08 (br. s, 1H).

### Beispiel LXXI

### Benzyl-3-(2-amino-6-hydroxypyrimidin-4-yl)piperidin-1-carboxylat

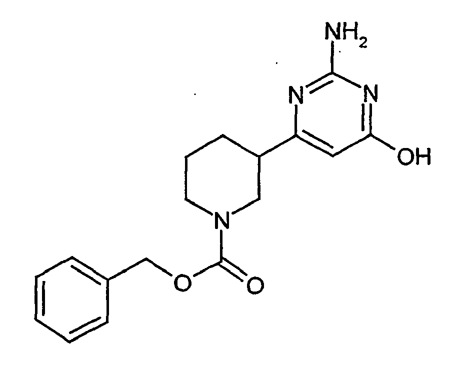

2 g (7.59 mmol) Piperidin-1,3-dicarbonsäure-1-benzylester, 1.31 g (9.11 mmol) 2,2-Dimethyl-1,3-dioxolan-4,6-dion und 1.85 g (15.19 mmol) 4-Dimethylaminopyridin werden in 12 ml Dichlormethan vorgelegt und auf 0°C gekühlt. Dazu werden 1.60 g (8.35 mmol) (3-Dimethylamino-propyl)-ethyl-carbodiimid-hydrochlorid gegeben. Es wird 1 Stunde bei 0°C und dann 18 Stunden bei Raumtemperatur nachgerührt. Das Reaktionsgemisch wird mit Dichlormethan (50 ml) verdünnt, es wird nacheinander mit Wasser (20 ml), 1N Salzsäure (30 ml), gesättigter wässriger Natriumhydrogencarbonatlösung (30 ml) und gesättigter wässriger Natriumchloridlösung gewaschen. Die organische Phase wird über wasserfreiem Magnesiumsulfat getrocknet. Die Lösung wird im Vakuum eingeengt. Der Rückstand (1.2 g) wird in 30 ml Ethanol gelöst und mit 0.13 ml (0.61 mmol) 37%iger Salzsäure versetzt und eine Stunde unter Rückfluss gerührt. Nach dem Abkühlen werden 0.59 g (3.18 mmol) Guanidiniumcarbonat zugegeben, und das Reaktionsgemisch wird 18 Stunden bei Rückfluss (Ölbadtemperatur 93°C) nachgerührt. Die Lösung wird eingeengt. Das Rohprodukt wird über eine Kieselgelsäule mit Dichlormethan-Methanol 20:1 gereinigt. Man erhält 0.68 g (27 % d. Th.) Produkt.

HPLC (Methode 7): Rₜ = 3.77 min.
MS (ESIpos): m/z = 329 (M+H)⁺
¹H-NMR (200 MHz, DMSO-d₆): δ = 1.29 (m, 2H), 1.76 (dd, 2H), 2.29 (m, 1H), 2.79 (m, 2H), 4.03 (m, 2H), 5.07 (s, 2H), 5.42 (s, 1H), 6.49 (s, 2H), 7.35 (m, 5H), 10.65 (s, 1H).

### Beispiel LXXII

### Benzyl-3-(2-amino-6-chlorpyrimidin-4-yl)piperidin-1-carboxylat

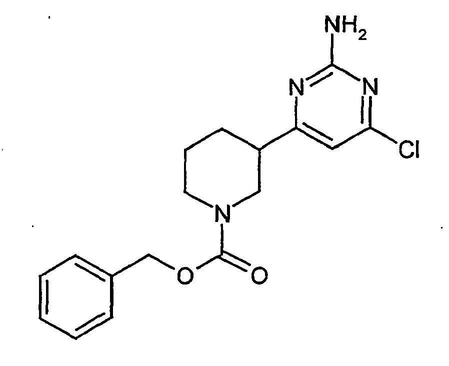

4.14 g (12.60 mmol) Benzyl-3-(2-amino-6-hydroxypyrimidin-4-yl)piperidin-1-carboxylat (aus Beispiel LXXI) werden in 150 ml Acetonitril suspendiert. Dazu werden nacheinander 3.29 ml (18.91 mmol) N,N-Diisopropylethylamin und 0.57 g (2.52 mmol) Benzyltriethylammoniumchlorid gegeben. Das Gemisch wird auf 0°C gekühlt. 4.7 ml (50.43 mmol) Phosphorylchlorid werden dann bei 0°C langsam zugetropft. Es wird bei Raumtemperatur 18 Stunden nachgerührt. Zur Aufarbeitung wird die Reaktionslösung auf 0°C gekühlt und vorsichtig auf Eiswasser (150 ml) getropft. Die wässrige Suspension wird mit einer 45%igen Natriumhydroxidlösung neutralisiert und dreimal mit Ethylacetat (jeweils 70 ml) extrahiert. Die organische Phase wird über wasserfreiem Magnesiumsulfat getrocknet und eingeengt. Der Rückstand wird über einer Kieselgelsäule mit Dichlormethan-Methanol 30:1 gereinigt. Man erhält 2.80 g (64 % d. Th.) Produkt.
HPLC (Methode 7): Rₜ = 4.30 min.
MS (ESIpos): m/z = 347 (M+H)⁺
¹H-NMR (300 MHz, DMSO-d₆): δ = 1.44 (m, 1H), 1.67 (m, 2H), 1.92 (m, 1H), 2.57 (m, 1H), 2.91 (m, 2H), 4.04 (dd, 2H), 5.09 (m, 2H), 6.61 (s, 1H), 7.02 (s, 2H), 7.35 (m, 5H).

### Beispiel LXXIII

### Ethyl-(2-amino-6-chlorpyrimidin-4-yl)acetat

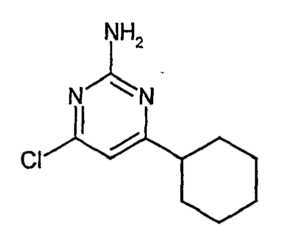

Die Titelverbindung wird analog zu Beispiel LXXI in zwei Stufen aus Cyclohexancarbonsäure synthetisiert.
Ausbeute: 100 mg (44 %)
LC-MS (Methode 16): Rₜ = 2.27 min.
MS (ESI pos.): m/z = 212 (M+H)⁺
¹H-NMR (DMSO-d₆, 200 MHz): δ = 1.35 (m, 5H), 1.75 (m, 5H), 2.40 (m, 1H), 6.54 (s, 1H), 6.97 (s, 2H).

### Beispiel LXXIV

### Benzyl-3-(2-amino-6-{[3-fluor-4-(1H-pyrrolo[2,3-b]pyridin-4-yloxy)phenyl]amino}-pyrimidin-4-yl)piperidin-1-carboxylat

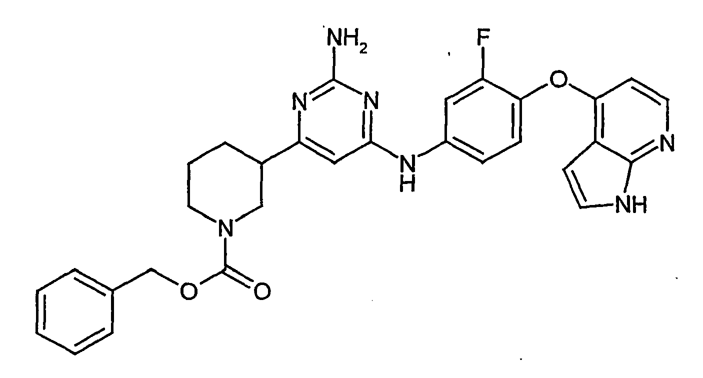

1.05 g (2.89 mmol) Benzyl-3-(2-amino-6-chlorpyrimidin-4-yl)piperidin-1-carboxylat (aus Beispiel LXXII) und 0.75 g (2.89 mmol) [3-Fluor-4-(1H-pyrrolo[2,3-b]pyridin-4-yloxy)phenyl]-amin (aus Beispiel XIX) werden in 35 ml Wasser und 4 ml Ethanol suspendiert. Dazu werden 0.29 ml (3.47 mmol) 37%ige Salzsäure gegeben. Das Gemisch wird bei 100°C 18 Stunden nachgerührt. Nach dem Abkühlen wird es mit einer gesättigten wässrigen Natriumhydrogencarbonatlösung neutralisiert und dreimal mit Ethylacetat (jeweils 30 ml) extrahiert. Die organische Phase wird über wasserfreiem Magnesiumsulfat getrocknet und eingeengt. Der Rückstand wird über einer Kieselgelsäule mit Dichlormethan-Methanol 20:1 gereinigt. Man erhält 1.38 g (86 % d. Th.) Produkt.
HPLC (Methode 7): Rₜ = 4.23 min.
MS (ESIpos): m/z = 554 (M+H)⁺
¹H-NMR (200 MHz, DMSO-d₆): δ = 1.55 (m, 2H), 1.67 (m, 2H), 1.84 (m, 2H), 2.54 (m, 1H), 2.85 (m, 2H), 4.10 (dd, 2H), 5.09 (s, 2H), 5.92 (s, 1H), 6.25 (dd, 1H), 6.35 (m, 3H), 7.27 (t, 1H), 7.36 (m, 7H), 8.06 (d, 1H), 8.21 (dd, 1H), 9.42 (s, 1H), 11.08 (s, 1H).

Analog zu Beispiel LXXIV werden hergestellt:

| **Bsp.-Nr.** | **Struktur** | **MS** | **HPLC (Methode 11):** |
|---|---|---|---|
| **LXXV** | 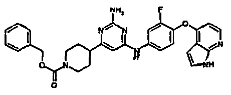 | MS (ESI pos.): m/z = 554 (M+H)⁺ | Rₜ = 4.14 min. |
| **LXXVI** | 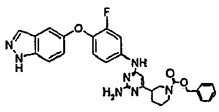 | MS (ESI pos.): m/z = 554 (M+H)⁺ | LC-MS (Methode 15): Rₜ = 1.49 min. |
| **LXXVII** | 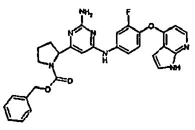 | MS (ESI pos.): m/z = 209 (M+H)⁺ | LC-MS (Methode 16): Rₜ = 2.02 min. |

### Beispiel LXXVIII

### (2-Amino-6-chlorpyrimidin-4-yl)methanol

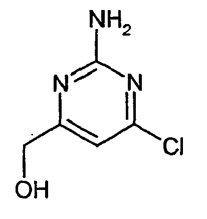

10.5 g (55.97 mmol) Methyl-2-amino-6-chlorpyrimidin-4-carboxylat (hergestellt nach G. Doyle Daves, Fred Baiocchi, Roland K. Robins, and C. C. Cheng, *J. Org. Chem*., **26** (1961), 2755-2763) werden in 450 ml Ethanol vorgelegt. Dazu werden 21.17 g (559.74 mmol) Natriumborhydrid gegeben und das Gemisch wird 2 Stunden bei Raumtemperatur gerührt. 500 ml Ethylacetat und 500 ml Wasser werden zugegeben und die Lösung wird 30 Minuten nachgerührt. Die Phasen werden getrennt und die wässrige Phase wird dreimal mit Ethylacetat (jeweils 100 ml) extrahiert. Die vereinigten organischen Phasen werden mit gesättigter wässriger Natriumchlorid-Lösung gewaschen, mit wasserfreiem Magnesiumsulfat getrocknet und eingeengt. Man erhält 7.21 g (85 % d. Th.) Produkt.
LC-MS (Methode 13): Rₜ = 0.58 min.
MS (ESIpos): m/z = 160 (M+H)⁺
¹H-NMR (200 MHz, DMSO-d₆): δ = 4.31 (d, 2H), 5.49 (t, 1H), 6.66 (s, 1H), 7.05 (s, 2H).

### Beispiel LXXIX

### 4-(Brommethyl)-6-chlorpyrimidin-2-amin und 4-(Brommethyl)-6-brompyrimidin-2-amin

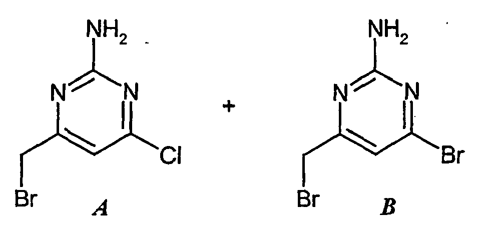

0.55 g (3.49 mmol) (2-Amino-6-chlorpyrimidin-4-yl)methanol (aus Beispiel LXXVIII) werden in 11 ml THF vorgelegt. Dazu werden 0.73 ml (7.67 mmol) Phosphortribromid gegeben und das Gemisch wird 3 Stunden bei Raumtemperatur gerührt. 20 ml Ethylacetat und 20 ml Eiswasser werden zugegeben und die Lösung wird 30 Minuten nachgerührt. Die Phasen werden getrennt und die wässrige Phase wird dreimal mit Ethylacetat (jeweils 10 ml) extrahiert. Die vereinigten organischen Phasen werden mit gesättigter wässriger Natriumhydrogencarbonat- und Natriumchlorid-Lösung gewaschen, mit wasserfreiem Magnesiumsulfat getrocknet und eingeengt. Man erhält 0.55 g (62 % d. Th.) eines Gemisches aus A und B (8:1).
LC-MS (Methode 14): Rₜ = 1.74 min und 1.83 min.
MS (ESIpos): m/z = 224 und 268 (M+H)⁺
¹H-NMR (200 MHz, DMSO-d₆): δ = 4.34 (d, 0.7 H), 4.37 (d, 1.3 H), 6.81 (s, 0.7 H), 6.94 (s, 0.3 H), 7.28 (s, 2H).

### Beispiel LXXX

### 4-[(tert-Butylamino)methyl]-6-chlorpyrimidin-2-amin und 4-[(tert-Butylamino)-methyl]-6-brompyrimidin-2-amin

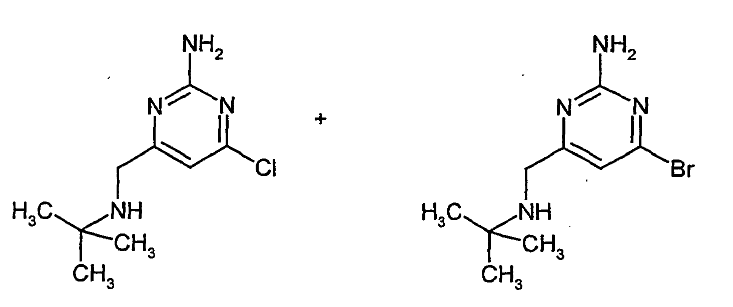

0.1 g (0.45 mmol) des Gemisches aus 4-(Brommethyl)-6-chlorpyrimidin-2-amin und 4-(Brommethyl)-6-brompyrimidin-2-amin (aus Beispiel LXXIX) werden in 1 ml Dimethylformamid vorgelegt. Dazu werden 0.07 ml (0.67 mmol) *tert*-Butylamin gegeben und das Gemisch wird 2 Stunden bei Raumtemperatur gerührt. Die Lösung wird mittels einer RP-HPLC-Chromatographie (Gradient: Acetonitril/Wasser) gereinigt. Man erhält 0.063 g (65 %) Produkt.
LC-MS (Methode 14): Rₜ = 0.46 min.
MS (ESIpos): m/z = 215 und 259 (M+H)⁺
Analog zu Beispiel LXXX werden hergestellt:

| **Bsp.-Nr.** | **Struktur** | **MS** | **HPLC, LC-MS** |
|---|---|---|---|
| **LXXXI** | 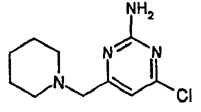 | MS (ESI pos.): m/z = 227 (M[Cl]+H)⁺, 271 (M[Br]+H)⁺ | LC-MS (Methode 14): Rₜ = 0.35 (Cl), 0.41 (Br) min. |
| **LXXXII** | 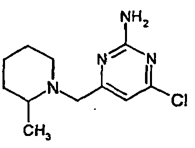 | MS (ESI pos.): m/z = 241 (M[Cl]+H)⁺, 285 (M[Br]+H)⁺ | LC-MS (Methode 15): Rₜ = 0.47 min. |
| **LXXXIII** | 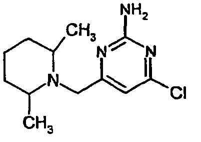 | MS (ESI pos.): m/z = 255 (M[Cl]+H)⁺, 299 (M[Br]+H)⁺ | LC-MS (Methode 15): Rₜ = 0.60, 0.68 min. |
| **LXXXIV** | 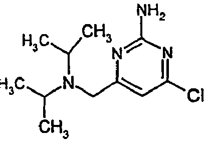 | MS (ESI pos.): m/z = 243 (M[Cl]+H)⁺ | LC-MS (Methode 15): Rₜ = 0.55 min. |
| **LXXXV** | 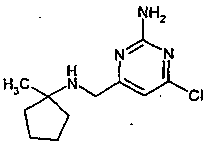 | MS (ESI pos.): m/z = 241 (M[Cl]+H)⁺, 285 (M[Br]+H)⁺ | LC-MS (Methode 15): Rₜ = 0.59 min. |
| **LXXXVI** | 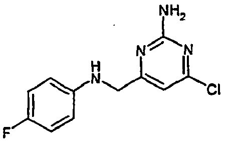 | MS (ESI pos.): m/z = 253 (M[Cl]+H)⁺, 297 (M[Br]+H)⁺ | LC-MS (Methode 13): Rₜ = 2.10 (Cl), 2.15 (Br) min. |
| **LXXXVII** | 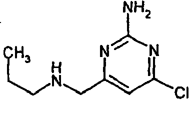 | MS (ESI pos.): m/z = 201 (M[Cl]+H)⁺, 245 (M[Br]+H)⁺ | LC-MS (Methode 13): Rₜ = 0.27 min. |
| **LXXXVIII** | 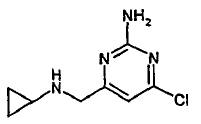 | MS (ESI pos.): m/z = 199 (M[Cl]+H)⁺, 243 (M[Br]+H)⁺ | LC-MS (Methode 14): Rₜ = 0.44 min. |
| **LXXXIX** | 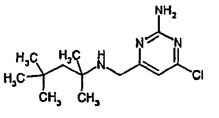 | MS (ESI pos.): m/z = 281 (M[Cl]+H)⁺, 315 (M[Br]+H)⁺ | LC-MS (Methode 15): Rₜ = 1.12 (Cl), 1.21 (Br) min. |
| **XC** | 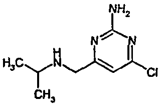 | MS (ESI pos.): m/z = 201 (M[Cl]+H)⁺, 245 (M[Br]+H)⁺ | LC-MS (Methode 15): Rₜ = 0.51 min. |

Analog zu Beispiel LXXIV wird hergestellt:

| **Bsp.-Nr.** | **Struktur** | **MS** | **HPLC, LC-MS** |
|---|---|---|---|
| **XCI** | 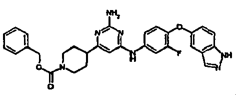 | MS (ESI pos.): m/z = 554 (M+H)⁺ | LC-MS (Methode 9): Rₜ = 2.31 min. |

### Herstellungsbeispiele:

### Beispiel 1

### N-[2-Amino-6-(4-pyridinyl)-4-pyrimidinyl]-N-{3-fluor-4-[(3-methyl-1H-indazol-4-yl)oxy]phenyl}amin

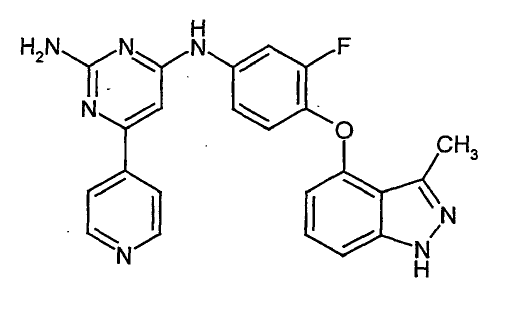

60 mg (0.23 mmol) 3-Fluor-4-[(3-methyl-1H-indazol-4-yl)oxy]phenylamin (aus Beispiel III) werden mit 48 mg (0.23 mmol) 4-Chlor-6-(4-pyridinyl)-2-pyrimidinamin (aus Beispiel XXIX) in 4 ml Wasser suspendiert und mit 0.02 ml konzentrierter Salzsäure versetzt. Die Reaktionsmischung wird über Nacht zum Rückfluss erhitzt, wobei sich ein brauner Niederschlag bildet. Dieser wird abfiltriert, mehrfach mit Wasser gewaschen und am Hochvakuum getrocknet. Man erhält 65 mg (65% d. Th.) des Produktes als braunen Feststoff.
LC-MS (Methode 2): Rₜ = 2.9 min.
MS (ESI pos.): m/z = 428 (M+H)⁺
¹H-NMR (DMSO-d₆, 300 MHz): δ = 2.56 (s, 3H), 6.27 (d, 1H), 6.79 (s, 1H), 7.18 (m, 3H), 7.47 (d, 1H), 7.89 (d, 2H), 8.22 (d, 1H), 8.86 (d, 2H), 10.88 (s, 1H), 12.75 (s, 1H).

### Beispiel 2

### N-{4-[(3-Amino-1,2-benzisoxazol-4-yl)oxy]-3-fluorphenyl}-N-[2-amino-6-(4-pyridinyl)-4-pyrimidinyl]amin

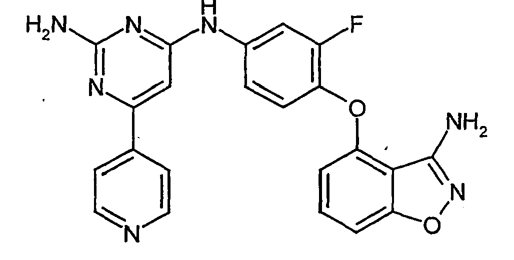

70 mg (0.27 mmol) 4-(4-Amino-2-fluorphenoxy)-1,2-benzisoxazol-3-amin (aus Beispiel VII) werden mit 55.8 mg (0.27 mmol) 4-Chlor-6-(4-pyridinyl)-2-pyrimidinamin (aus Beispiel XXIX) in 4 ml Wasser suspendiert und mit 0.02 ml konzentrierter Salzsäure versetzt. Die Reaktionsmischung wird über Nacht zum Rückfluss erhitzt, wobei sich ein heller Niederschlag bildet. Dieser wird abfiltriert, mehrfach mit Wasser gewaschen und am Hochvakuum getrocknet.
Ausbeute: 60 mg (52% d. Th.)
LC-MS (Methode 6): Rₜ = 2.7 min.
MS (ESI pos.): m/z = 429 (M+H)⁺
¹H-NMR (DMSO-d₆, 300 MHz): δ = 5.28 (m, 4 H), 6.33 (d, 1H), 6.97 (s, 1H), 7.14 (d, 1H), 7.43 (m, 2H), 7.58 (d, 1H), 8.02 (d, 2H), 8.28 (d, 1H), 8.93 (d, 2H), 11.43 (s, 1H).

### Beispiel 3

### N-{4-[(3-Amino-1,2-benzisoxazol-4-yl)oxy]-3-fluorphenyl}-N-(2-amino-6-chlor-4-pyrimidinyl)amin

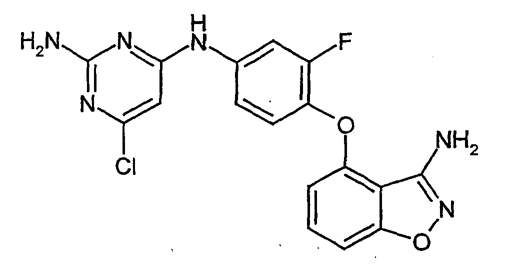

100 mg (0.39 mmol) 4-(4-Amino-2-fluorphenoxy)-1,2-benzisoxazol-3-amin (aus Beispiel VII) werden mit 63.3 mg (0.39 mmol) 2-Amino-4,6-dichlorpyrimidin in 3 ml Wasser suspendiert und mit 0.04 ml konzentrierter Salzsäure versetzt. Die Reaktionsmischung wird über Nacht zum Rückfluss erhitzt, wobei sich ein farbloser Niederschlag bildet. Dieser wird abfiltriert, mehrfach mit Wasser gewaschen und am Hochvakuum getrocknet.
Ausbeute: 100 mg (67% d. Th.)
LC-MS (Methode 8): Rₜ = 3.38 min.
MS (ESI pos.): m/z = 387 (M+H)⁺

### Beispiel 4

### 4-[(4aR,7aR)-Octahydro-6H-pyrrolo[3,4-b]pyridin-6-yl]-6-({4-[(3-amino-1,2-benz-isoxazol-4-yl)oxy]-3-fluorphenyl}amino)-2-pyrimidinylamin

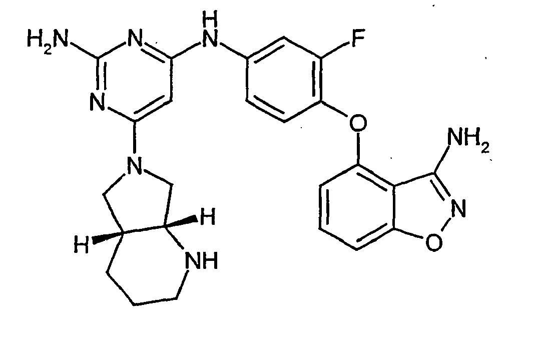

88 mg (0.23 mmol) N-{4-[(3-Amino-1,2-benzisoxazol-4-yl)oxy]-3-fluorphenyl}-N-(2-amino-6-chlor-4-pyrimidinyl)amin (aus Beispiel 3) und 114.8 mg (0.91 mmol) [(4aR,7aR)-Octahydro-6H-pyrrolo[3,4-b]pyridin werden mit 294.1 mg (2.28 mmol) Diisopropylethylamin in 4 ml 2-Ethylhexanol gelöst und 3 h bei 150°C gerührt. Nach Abkühlen wird die Reaktionsmischung filtriert und mit Wasser versetzt. Man extrahiert mehrfach mit Ethylacetat, trocknet die organische Phase über Magnesiumsulfat und entfernt das Lösungsmittel im Vakuum. Der Rückstand wird mittels präparativer HPLC gereinigt.
Ausbeute: 15 mg (14% d. Th.)
LC-MS (Methode 8): Rₜ = 2.2 min.
MS (ESI pos.): m/z = 477 (M+H)⁺

### Beispiel 5

### N-[2-Amino-6-(4-pyridinyl)-4-pyrimidinyl]-N-[3-fluor-4-(1H-indazol-5-yloxy)-phenyl]amin

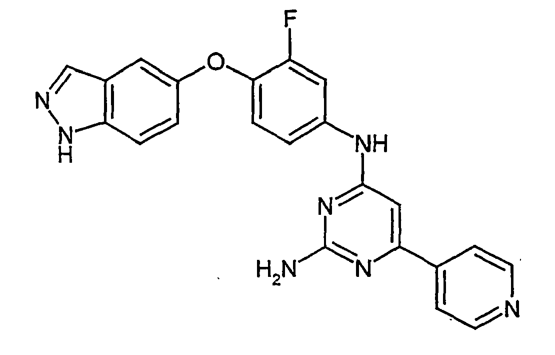

600 mg (2.47 mmol) 5-(4-Amino-2-fluorphenoxy)-1H-indazol (aus Beispiel XII) werden in 100 ml Wasser suspendiert. Dazu gibt man 510 mg (2.47 mmol) 4-Chlor-6-(4-pyridinyl)-2-pyrimidinamin (aus Beispiel XXIX) und 0.25 ml konzentrierte wässrige Chlorwasserstofflösung und lässt über Nacht bei 100°C rühren. Zur Aufarbeitung wurde die Reaktionslösung mit gesättigter Natriumhydrogencarbonat-Lösung alkalisch gestellt und 3x mit Ethylacetat extrahiert. Der dabei anfallende Niederschlag enthält Rohprodukt und wird abgesaugt. Die organische Phase wird mit Natriumsulfat getrocknet und am Rotationsverdampfer eingeengt. Der Rückstand wird mit dem Niederschlag vereint und mittels präparativer HPLC gereinigt.
Ausbeute: 631 mg (62% d. Th.)
MS (ESI pos.): m/z = 414 (M+H)⁺
¹H-NMR (DMSO-d₆, 200 MHz): δ = 6.56 (s, 1H), 6.63 (s, 2H), 7.06 - 7.19 (m, 3H), 7.33 (d, 1H), 7.53 (d, 1H), 7.84 (dd, 2H), 7.97 (s, 1H), 8.22 (dd, 1H), 8.72 (dd, 2H), 9.60 (s, 1H), 13.03 - 13.12 (br. s, 1H).

Analog zu Beispiel 5 werden hergestellt:

| **Bsp.- Nr.** | **Struktur** | **MS, HPLC, LC-MS** | **NMR** |
|---|---|---|---|
| **6** | 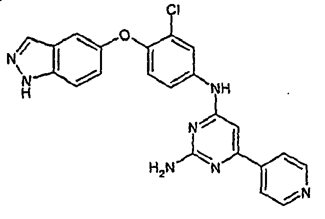 | MS (ESI pos.): m/z = 430 (M+H)⁺ | ¹H-NMR (DMSO-d₆, 200 MHz): δ = 6.58 (m, 3H), 7.04 (d, 1H), 7.11-7.19 (m, 2H), 7.52-7.68 (m, 2H), 7.85 (d, 2H), 7.99 (s, 1H), 8.16 (d, 1H), 8.71 (d, 2H), 9.52 (s, 1H), 13.03-13.13 (br. s, 1H). |
| **7** | 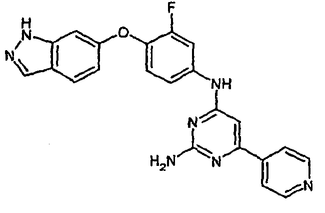 | MS (ESI pos.): m/z = 413 (M+H)⁺, | ¹H-NMR (DMSO-d₆, 300 MHz): δ = 6.58 (s, 2H), 6.80 (s, 1H), 6.92 (dd, 1H), 7.22 (t, 1H), 7.39 (dd, 1H), 7.73 (d, 1H), 7.85 (d, 2H), 8.01 (s, 1H), 8.24 (dd, 1H), 8.72 (d, 2H), 9.62 (s, 1H), 12.77 (s, 1H). |
| | | LC-MS (Methode Methode 8): Rₜ = 2.72 min. | |

### Beispiel 8

### N-[2-Amino-6-chlor-4-pyrimidinyl]-N-[3-fluor-4-(1H-indazol-5-yloxy)phenyl]amin

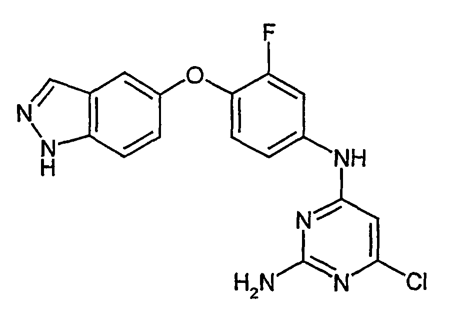

1.65 g (6.78 mmol) 5-(4-Amino-2-fluorphenoxy)-1H-indazol (aus Beispiel XII) werden in 90 ml Wasser suspendiert. Dazu gibt man 1.11 g (6.78 mmol) 4,6-Dichlor-2-pyrimidinamin und 0.68 ml konzentrierte wässrige Chlorwasserstofflösung und lässt über Nacht bei 100°C rühren. Zur Aufarbeitung wird die Reaktionslösung mit gesättigter Natriumhydrogencarbonat-Lösung alkalisch gestellt und 3x mit Ethylacetat extrahiert. Die organische Phase wird mit Natriumsulfat getrocknet und am Rotationsverdampfer eingeengt.
Ausbeute: 2.88 g (quantitativ)
LC-MS (Methode 8): Rₜ = 3.21 min.
MS (ESI pos.): m/z = 371 (M+H)⁺

Analog zu Beispiel 8 werden hergestellt:

| **Bsp.- Nr.** | **Struktur** | **MS, HPLC, LC-MS** | **NMR** |
|---|---|---|---|
| **9** | 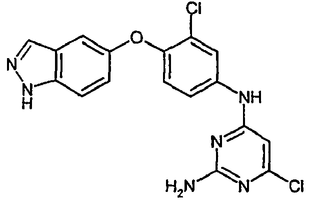 | MS (ESI pos.): m/z = 387 (M+H)⁺ | ¹H-NMR (DMSO-d₆, 300 MHz): δ = 5.98 (s, 1H), 6.75 (s, 2H), 6.99 (d, 1H), 7.08-7.18 (m, 2H), 7.49-7.57 (m, 2H), 7.94-8.02 (m, 2H), 9.42 (s,1H), 13.05 (s, 1H). |
| | | HPLC (Methode 7): Rₜ = 3.93 min. | |
| **10** | 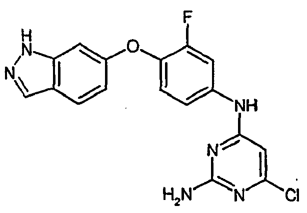 | MS (ESI pos.): m/z = 371 (M+H)⁺ | ¹H-NMR (DMSO-d₆, 300 MHz): δ = 6.58 (s, 2H), 6.80 (s, 1H), 6.92 (dd, 1H), 7.22 (t, 1H), 7.39 (dd, 1H), 7.73 (d, 1H), 7.85 (d, 2H), 8.01 (s, 1H), 8.24 (dd, 1H), 8.72 (d, 2H), 9.62 (s, 1H), 12.77 (s, 1H). |
| | | LC-MS (Methode 9): Rₜ = 4.02 min. | |

### Beispiel 11

### 6-[(4aR,7aR)-Octahydro-6H-pyrrolo[3,4-b]pyridin-6-yl]-N4-[3-fluor-4-(1H-indazol-5-yloxy)phenyl]-2,4-pyrimidindiamin

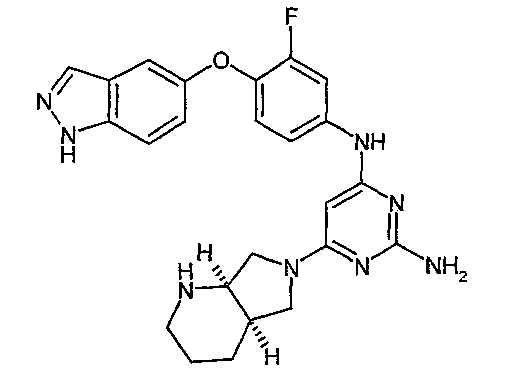

127 mg (340 µmol) N-[2-Amino-6-chlor-4-pyrimidinyl]-N-[3-fluor-4-(1H-indazol-5-yloxy)phenyl]amin (aus Beispiel 8) werden in 10 ml 2-Ethylhexanol suspendiert. Anschließend wird mit 173 mg (1.37 mmol) [(4aR,7aR)-Octahydro-6H-pyrrolo[3,4-b]-pyridin und 0.60 ml (3.43 mmol) Diisopropylethylamin versetzt und über Nacht bei 150°C gerührt. Die Reaktionslösung wird mittels MPLC mit Dichlormethan/-Methanol/konzentrierte wässrige Ammoniaklösung 20:1:0 → 10:1:0 → 5:1:0 → 3:1:0.1 chromatographiert.
Ausbeute: 147 mg (93 %)
LC-MS (Methode 5): Rₜ = 3.12 min.
MS (ESI pos.): m/z = 461 (M+H)⁺
¹H-NMR (DMSO-d₆, 200 MHz): δ = 1.17-1.30 (m, 1H), 1.60-1.85 (m, 4H), 2.54-2.70 (m, 1H), 2.80-2.97 (m, 1H), 3.05-3.63 (mehrere m, teilweise von H₂O-Peak überlagert, insgesamt 4 H), 3.65-3.80 (m, 1H), 5.12 (s, 1H), 5.98 (s, 2H), 6.92-7.26 (m, 4H), 7.52 (d, 1H), 7.93 (s, 1H), 8.11 (dd, 1H), 8.94 (s,1H), 13.05 (s, 1H).

Analog zu Beispiel 11 werden hergestellt:

| **Bsp.-Nr.** | **Struktur** | **MS, HPLC, LC-MS, DC** | **NMR** |
|---|---|---|---|
| **12** | 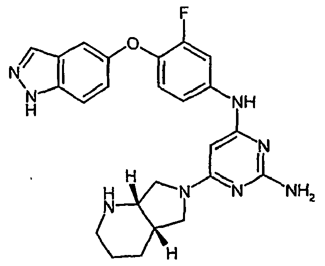 | MS (ESI pos.): m/z = 461 (M+H)⁺ | |
| | | LC-MS (Methode 8): Rₜ = 2.12 min. | |
| **13** | 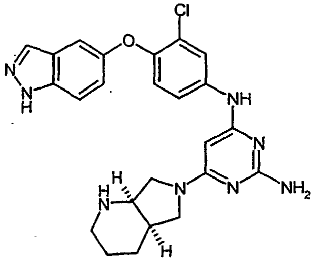 | MS (ESI pos.): m/z = 477 (M+H)⁺ | |
| | | LC-MS (Methode 8): Rₜ = 2.23 min. DC (Kieselgel): R_{f}= 0.09 (Dichlormethan/ Methanol 10:1) | |
| **14** | 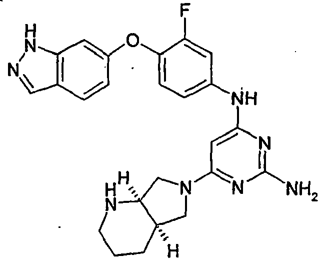 | MS (ESI pos.): m/z = 461 (M+H)⁺ | |
| | | LC-MS (Methode 2): Rₜ = 2.40 min. | |
| **15** | 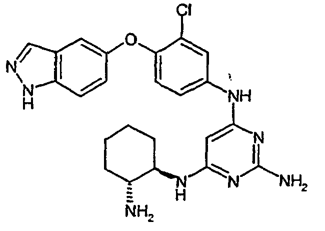 | MS (ESI pos.): m/z = 465 (M+H)⁺ | ¹H-NMR (DMSO-d₆, 400 MHz): δ = 1.12-1.34 (m, 6H), 1.63-1.74 (m, 2H), 1.85-2.01 (m, 2H), 2.69-2.78 (m, 1H), 3.50-3.63 (br. s, 1H), 5.20 (s, 1H), 5.80 (s, 2H), 6.20-6.43 (m, 3H), 6.94 (d, 1H), 7.05-7.13 (m, 2H), 7.48 (dd, 1H), 7.53 (d, 1H), 7.92-7.99 (m, 2H), 8.74 (s,1H), 13.01-13.12 (br. s, 1H). |
| | | MS (ESI neg.): m/z = 463 (M-H)- | |
| | | LC-MS (Methode 8): Rₜ = 2.09 min. | |
| **16** | 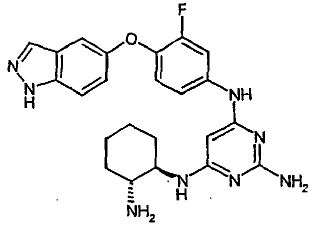 | MS (ESI pos.): m/z = 449 (M+H)⁺ | |
| | | HPLC (Methode 7): Rₜ = 3.78 min. | |
| | | DC (Kieselgel): R_{f} = 0.22 (Dichlormethan/ Methanol 3: 1) | |
| **17** | 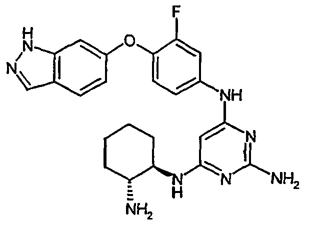 | MS (ESI pos.): m/z = 449 (M+H)⁺ | |
| | | HPLC (Methode 7): Rₜ = 3.82 min. | |

### Beispiel 18

### N-(2-Amino-6-phenyl-4-pyrimidinyl)-N-[3-fluor-4-(1H-indazol-5-yloxy)phenyl]amin

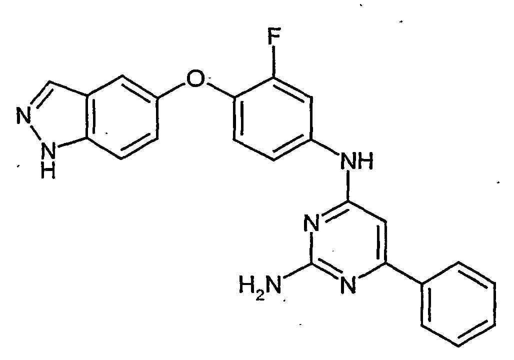

50 mg (130 µmol) N-[2-Amino-6-chlor-4-pyrimidinyl]-N-[3-fluor-4-(1H-indazol-5-yloxy)phenyl]amin (aus Beispiel 8) werden in 3 ml Toluol / Ethanol (2:1) suspendiert und mit 4.68 mg Tetrakis(triphenylphosphin)palladium(0) versetzt. Nach Zugabe von 19.7 mg (160 µmol) Phenylboronsäure und 0.50 ml 2M wässriger NatriumcarbonatLösung wird über Nacht bei 100°C gerührt. Die Mischung wird am Rotationsverdampfer zur Trockene eingeengt und mittels präparativer HPLC gereinigt.
Ausbeute: 10 mg (17% d. Th.)
LC-MS (Methode 9): Rₜ = 3.55 min.
MS (ESI pos.): m/z = 413 (M+H)⁺

### Beispiel 19

### N-[2-Amino-6-(4-pyridinyl)-4-pyrimidinyl]-N-[3-fluoro-4-(1H-pyrrolo[2,3-b]pyridin-4-yloxy)phenyl]amin

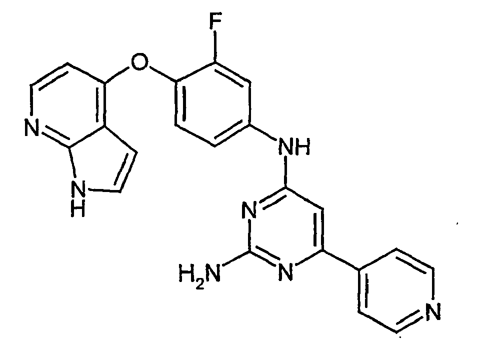

55.6 mg (230 µmol) 3-Fluor-4-(1H-pyrrolo[2,3-b]pyridin-4-yloxy)anilin (aus Beispiel XIX) werden in 5 ml Wasser suspendiert. Dazu gibt man 47.2 mg (230 µmol) 4-Chlor-6-(4-pyridinyl)-2-pyrimidinamin (aus Beispiel XXIX) und 0.01 ml konzentrierte Salzsäure und lässt über Nacht bei 100°C rühren. Zur Aufarbeitung wird die Reaktionslösung mit gesättigter Natriumhydrogencarbonat-Lösung alkalisch gestellt, 3x mit Ethylacetat extrahiert, über Natriumsulfat getrocknet und am Rotationsverdampfer eingeengt. Der Rückstand wird über präparative HPLC gereinigt
Ausbeute: 6.5 mg (6.0% d. Th.)
LC-MS (Methode 6): R_{f} = 2.52 min.
MS (ESI pos.): m/z = 207 (M+H)²⁺
MS (ESI neg.): m/z = 412 (M-H)⁻

### Beispiel 20

### N-[2-Amino-6-(4-pyridinyl)-4-pyrimidinyl]-N-[2-fluoro-4-(1H-pyrrolo[2,3-b]-pyridin-4-yloxy)phenyl]amin

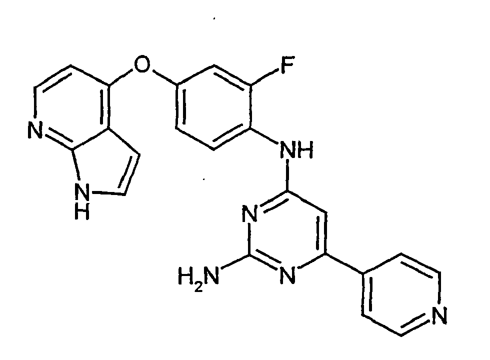

11 mg (50 µmol) 2-Fluor-4-(1H-pyrrolo[2,3-b]pyridin-4-yloxy)anilin (aus Beispiel XX) werden in 5 ml Wasser suspendiert. Dazu gibt man 9.3 mg (50 µmol) 4-Chlor-6-(4-pyridinyl)-2-pyrimidinamin (aus Beispiel XXIX) und 0.01 ml konzentrierte Salzsäure und lässt über Nacht bei 100°C rühren. Zur Aufarbeitung wird die Reaktionslösung mit gesättigter Natriumhydrogencarbonat-Lösung alkalisch gestellt, 3x mit Ethylacetat extrahiert, über Natriumsulfat getrocknet und am Rotationsverdampfer eingeengt.
Ausbeute: 20 mg (98% d. Th.)
LC-MS (Methode 6): Rₜ = 2.47 min.
MS (ESI pos.): m/z = 414 (M+H)⁺, 207 (M+H)²⁺
MS (ESI neg.): m/z = 412 (M-H)⁻
¹H-NMR (DMSO-d₆, 400 MHz): δ = 6.25 (d, 1H), 6.45 (s, 1H), 6.52 (d, 1H), 6.76 (s, 2H), 7.00 (d, 1H), 7.23 (dd, 1H), 7.38 (s, 1H), 7.80 - 7.89 (m, 2H), 8.14 (d, 1H), 8.25 (t, 1H), 8.62 - 8.77 (m, 2H), 9.06 (s, 1H), 11.88 - 12.08 (br. s, 1H).

### Beispiel 21

### N-[2-Amino-6-(4-pyridinyl)-4-pyrimidinyl]-N-[4-(1-benzofuran-4-yloxy)-3-fluorphenyl]amin

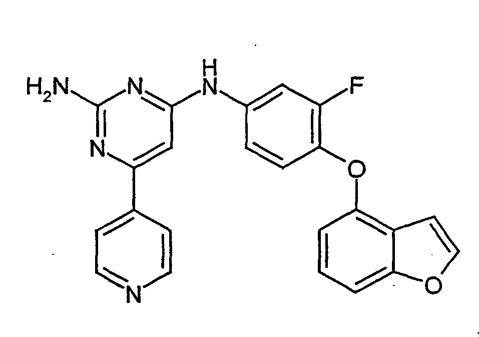

32 mg (0.13 mmol) 4-(1-Benzofuran-4-yloxy)-3-fluorphenylamin (aus Beispiel XXIII) werden mit 28.5 mg (0.14 mmol)) 4-Chlor-6-(4-pyridinyl)-2-pyrimidin-amin (aus Beispiel XXIX) in 1.5 ml Wasser suspendiert und mit 19 µl konzentrierter Salzsäure versetzt. Die Reaktionsmischung wird über Nacht zum Rückfluss erhitzt, wobei sich ein brauner Niederschlag bildet. Die Suspension wird mit 1N Natriumhydroxid-Lösung auf pH 10 gestellt. Der Niederschlag wird abgesaugt und das Filtrat verworfen. Das Rohprodukt wird mittels präparativer HPLC gereinigt.
Ausbeute: 43 mg (79% d. Th.)
LC-MS (Methode 2): Rₜ = 3.2 min.
MS (ESI pos.): m/z = 414 (M+H)⁺
¹H-NMR (DMSO-d₆, 200 MHz): δ = 6.67 (s, 2H), 6.88 (s, 1H), 7.32 (m, 4H), 7.86 (m, 2H), 8.00 (d, 1H), 8.28 (dd, 1H), 8.84 (d, 2H), 10.46 (s, 1H).

### Beispiel 22

### N-[2-Amino-6-(4-pyridinyl)-4-pyrimidinyl]-N-[3-fluor-4-(1H-indazol-4-yloxy)phenyl]amin

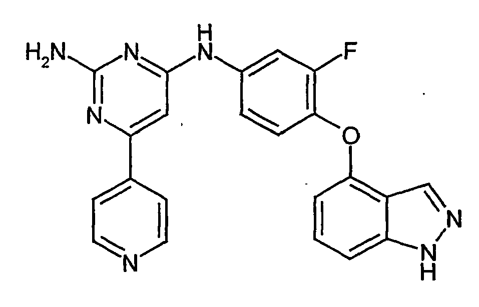

45 mg (0.19 mmol) 3-Fluor-4-(1H-indazol-4-yloxy)anilin (aus Beispiel XXVI) werden mit 40.1 mg (0.19 mmol) 4-Chlor-6-(4-pyridinyl)-2-pyrimidinamin (aus Beispiel XXIX) in 2 ml Wasser suspendiert und mit 27 µl konzentrierter Salzsäure versetzt. Die Reaktionsmischung wird über Nacht zum Rückfluss erhitzt, wobei sich ein brauner Niederschlage bildet. Die Suspension wird mit 1N Natriumhydroxid-Lösung auf pH 10 eingestellt. Der Niederschlag wird von der wässrigen Phase abfiltriert und das Filtrat verworfen. Das Rohprodukt wird mittels HPLC gereinigt.
Ausbeute: 20 mg (26% d. Th.)
LC-MS (Methode 2): Rₜ = 2.9 min.
MS (ESI pos.): m/z = 414 (M+H)⁺
¹H-NMR (DMSO-d₆, 200 MHz): δ = 6.34 (dd, 1H), 6.64 (s, 2H), 6.60 (s 1H), 7.28 (m, 4H), 7.85 (d, 2H), 7.96 (s, 1H), 8.26 (dd, 1H), 8.70(d, 2H), 9.67 (s, 1H), 13.2 (s, 1H).

### Beispiel 23

### N⁴-{3-Fluor-4-[(3-methyl-1H-pyrrolo[2,3-b]pyridin-4-yl)oxy]phenyl}-6-pyridin-4-ylpyrimidin-2,4-diamin

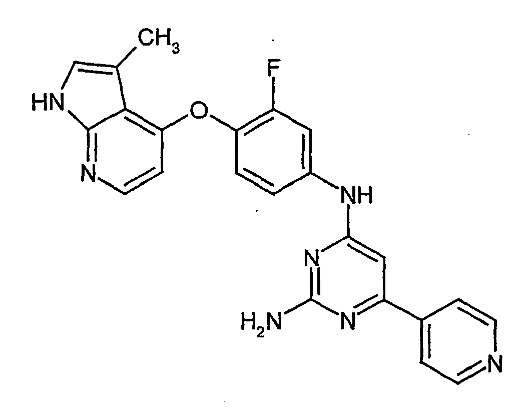

10 mg (0.04 mmol) {3-Fluor-4-[(3-methyl-1H-pyrrolo[2,3'-b]pyridin-4-yl)oxy]-phenyl}amin (aus Beispiel XLII) und 8.40 mg (0.04 mmol) 4-Chlor-6-(4-pyridinyl)-2-pyrimidinamin (aus Beispiel XXIX) werden in 1.5 ml Wasser suspendiert Dazu gibt man 5.6µl konzentrierte Salzsäure und lässt über Nacht bei 100°C rühren. Zur Aufarbeitung wird die Reaktionslösung mit gesättigter Natriumcarbonatlösung alkalisch gestellt, dreimal mit Essigsäureethylester extrahiert, über Natriumsulfat getrocknet und am Rotationsverdampfer eingeengt. Der Rückstand wird mittels präparativer HPLC gereinigt.
Ausbeute: 3.30 mg (20% d. Th.)
LC-MS (Methode 9): Rₜ = 1.97 min.

### Beispiel 24

### N⁴-{3-Fluor-4-[(3-methyl-1H-pyrrolo[2,3-b]pyridin-4-yl)oxy]phenyl}-6-(4-fluorphenyl)pyrimidin-2,4-diamin

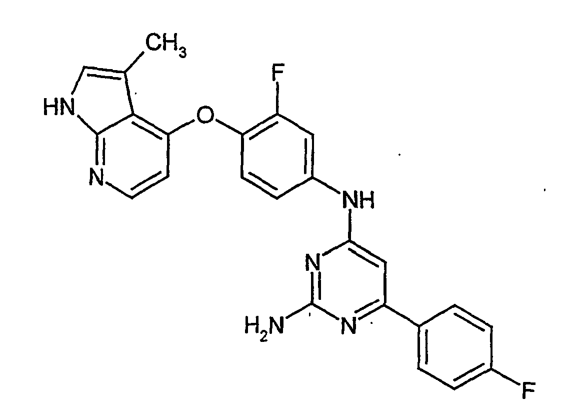

180 mg (0.70 mmol) {3-Fluor-4-[(3-methyl-1H-pyrrolo[2,3-b]pyridin-4-yl)oxy]-phenyl}amin (aus Beispiel XLII) und 191 mg (0.73 mmol) 4-Chlor-6-(4-fluorphenyl)pyrimidin-2-amin (aus Beispiel LIX) werden in 10 ml Wasser suspendiert. Dazu gibt man 101 µl konzentrierte Salzsäure und lässt über Nacht bei 100°C rühren. Zur Aufarbeitung wird die Reaktionslösung mit gesättigter Natriumcarbonatlösung alkalisch gestellt, dreimal mit Essigsäureethylester extrahiert, über Natriumsulfat getrocknet und am Rotationsverdampfer eingeengt. Der Rückstand wird mittels präparativer HPLC gereinigt.
Ausbeute: 70 mg (23% d. Th.)
LC-MS (Methode 17): Rₜ = 1.73 min.
MS (ESI pos.): m/z = 445 (M+H)⁺

### Beispiel 25

### 6-(4-Fluorphenyl)-N⁴-[3-fluor-4-(1H-pyrrolo[2,3-b]pyridin-4-yloxy)phenyl]-pyrimidin-2,4-diamin

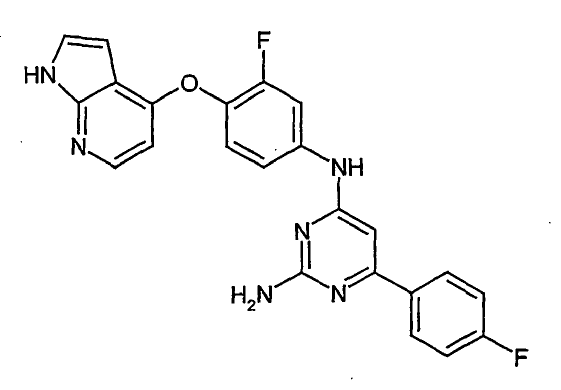

36 mg (0.15 mmol) 3-Fluor-4-(1H-pyrrolo[2,3-b]pyridin-4-yloxy)anilin (aus Beispiel XIX) und 38.5 mg (0.15 mmol) 4-Chlor-6-(4-fluorphenyl)pyrimidin-2-amin (aus Beispiel LIX) werden in 1.5 ml Wasser suspendiert und mit 0.1 ml 2 molarer Salzsäure versetzt. Man erhitzt über Nacht unter Rückfluss. Dann wird mit Essigsäureethylester und einigen Tropfen Dimethylformamid versetzt. Man stellt mit gesättigter Natriumcarbonatlösung alkalisch, trennt die organische Phase ab und entfernt das Lösungsmittel im Vakuum. Man reinigt mittels präparativer HPLC. Ausbeute: 28 mg (40% d. Th.)
LC-MS (Methode 10): Rₜ = 2.22 min.
MS (ESI pos.): m/z = 431 (M+H)⁺
¹H-NMR (DMSO-d₆, 400 MHz): δ = 6.27 (d, 1H), 6.37 (d, 1H), 6.50 (s, 1H), 6.52 (s, 2H), 7.27 - 7.43 (m, 5H), 8.00 (dd, 2H), 8.07 (d, 1H), 8.28 (dd, 1H), 9.58 (s, 1H), 11.75 (s, 1H).

### Beispiel 26

### N⁴-[3,5-Difluor-4-(1H-pyrrolo[2,3-b]pyridin-4-yloxy)phenyl]-6-(4-fluorphenyl)-pyrimidin-2,4-diamin

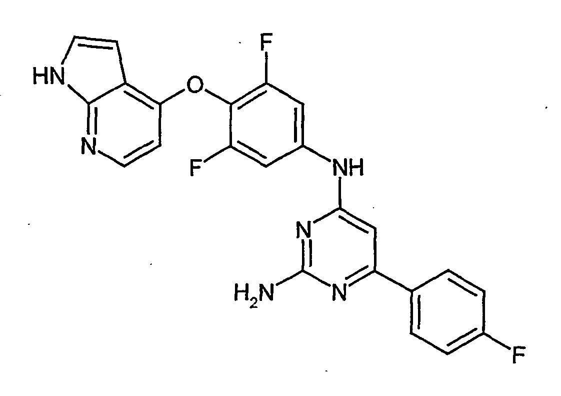

Analog zu Beispiel 25 wird die Titelverbindung aus 100 mg (0.36 mmol) [3,5-Difluor-4-(1H-pyrrolo[2,3-b]pyridin-4-yloxy)phenyl]amin (aus Beispiel XXXIV) und 110 mg (0.36 mmol) 4-Chlor-6-(4-fluorphenyl)pyrimidin-2-amin (aus Beispiel LIX) synthetisiert. Nach Reinigung mittels präparativer HPLC erhält man das Produkt.
Ausbeute: 17 mg (10% d. Th.)
LC-MS (Methode 10): Rₜ = 2.32 min.
MS (ESI pos.): m/z = 449 (M+H)⁺
¹H-NMR (DMSO-d₆, 400 MHz): δ = 6.31 (dd, 1H), 6.43 (d, 1H), 6.48 (s, 1H), 6.63 (br. s, 2H), 7.33 (t, 2H), 7.41 (dd, 1H), 7.83 (d, 2H), 8.01 (dd, 2H), 8.09 (d, 1H), 9.72 (s, 1H), 11.82 (s, 1H).

### Beispiel 27

### N⁴-[3,5-Difluor-4-(1H-pyrrolo[2,3-b]pyridin-4-yloxy)phenyl]-6-pyridin-4-yl-pyrimidin-2,4-diamin

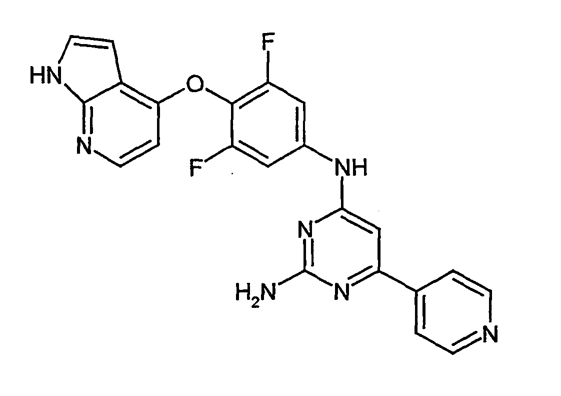

Analog zu Beispiel 25 wird die Titelverbindung aus 100 mg (0.36 mmol) [3,5-Difluor-4-(1H-pyrrolo[2,3-b]pyridin-4-yloxy)phenyl]amin (aus Beispiel XXXIV) und 87 mg (0.36 mmol) 4-Chlor-6-(4-pyridinyl)-2-pyrimidinamin (aus Beispiel XXIX) synthetisiert. Nach Reinigung mittels präparativer HPLC erhält man das Produkt.
Ausbeute: 67 mg (43% d. Th.)
LC-MS (Methode 10): Rₜ = 2.17 min.
MS (ESI pos.): m/z = 432 (M+H)⁺
¹H-NMR (DMSO-d₆, 400 MHz): δ = 6.32 (dd, 1H), 6.44 (d, 1H), 6.59 (s, 1H), 6.76 (br. s, 2H), 7.41 (dd, 1H), 7.82 - 7.88 (m, 4H), 8.09 (d, 1H), 8.72 (d, 2H), 9.85 (s, 1H), 11.83 (s, 1H).

### Beispiel 28

### N⁴-[4-(2,3-Dihydro-1H-pyrrolo[2,3-b]pyridin-4-yloxy)-3-fluorphenyl]-6-pyridin-4-ylpyrimidin-2,4-diamin

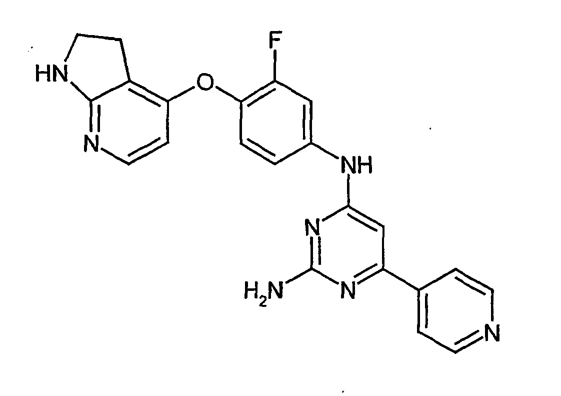

Analog zu Beispiel 25 wird die Titelverbindung aus 19.7 mg (0.08 mmol) [4-(2,3-Dihydro-1H-pyrrolo[2,3-b]pyridin-4-yloxy)-3-fluorphenyl]amin (aus Beispiel XLVI) und 17.4 mg (0.08 mmol) 4-Chlor-6-(4-pyridinyl)-2-pyrimidinamin (aus Beispiel XXIX) synthetisiert. Nach Reinigung mittels präparativer HPLC erhält man das Produkt.
Ausbeute: 7 mg (21 % d. Th.)
LC-MS (Methode 12): Rₜ = 1.74 min.
MS (ESI pos.): m/z = 416 (M+H)⁺
¹H-NMR (DMSO-d₆, 300 MHz): δ = 2.85 (t, 2H), 3.50 (t, 2H), 5.92 (d, 1H), 6.55 (s, 1H), 6.60 (s, 2H), 6.85 (s, 1H), 7.22 (t 1H), 7.40 (m, 1H), 7.62 (d, 1H), 7.85 (d, 2H), 8.30 (m 1H), 8.70 (d, 2H), 9.62 (s, 1H).

### Beispiel 29

### N⁴-[4-(2,3-Dihydro-1H-pyrrolo[2,3-b]pyridin-4-yloxy)-3-fluorphenyl]-6-(4-fluorphenyl)pyrimidin-2,4-diamin

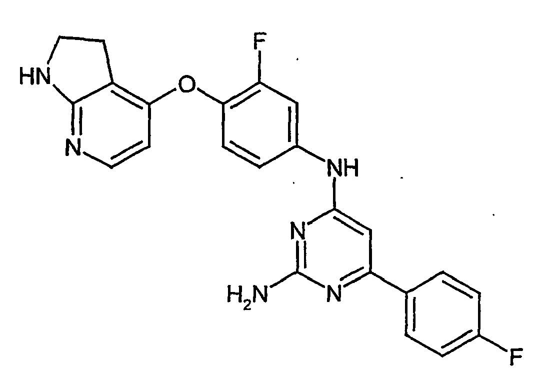

Analog zu Beispiel 25 wird die Titelverbindung aus 35 mg (0.14 mmol) [4-(2,3-Dihydro-1H-pyrrolo[2,3-b]pyridin-4-yloxy)-3-fluorphenyl]amin (aus Beispiel XLVI) und 39 mg (0.15 mmol) 4-Chlor-6-(4-fluorphenyl)pyrimidin-2-amin (aus Beispiel LIX) synthetisiert. Nach Reinigung mittels präparativer HPLC erhält man das Produkt.
Ausbeute: 20 mg (32% d. Th.)
LC-MS (Methode 9): Rₜ = 2.3 5 min.
MS (ESI pos.): m/z = 433 (M+H)⁺
¹H-NMR (DMSO-d₆, 300 MHz): δ = 2.85 (t, 2H), 3.51 (t, 2H), 5.85(d, 1H), 6.55 - 6.65 (m, 4H), 7.10 - 7.40 (m, 4H), 7.62 (d, 1H), 7.95 (m, 2H), 8.25 (m 1H), 9.52 (s, 1H).

Analog zu Beispiel 25 werden hergestellt:

| **Bsp.-Nr.** | **Edukte (Bsp.-Nr.)** | **Struktur** | **MS, HPLC, LC-MS,** ^{**1**}**H-NMR** |
|---|---|---|---|
| **30** | XXIX | 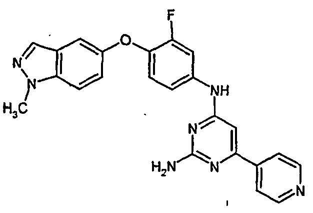 | MS (ESI pos.): m/z = 428 (M+H)⁺ HPLC (Methode 7): Rₜ = 3.86 min. |
| **31** | LIII, XXIX | 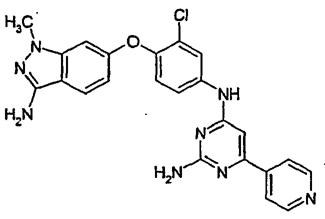 | MS (ESI pos.): m/z = 459 (M+H)⁺ |
| | | | LC-MS (Methode 9): Rₜ = 3.56 min. |
| | | | ¹H-NMR (DMSO-d₆, 400 MHz): δ = 3.61 (s, 3H), 5.42 (br. s, 2H), 6.57 - 6.60 (m, 4H), 6.74 (d, 1H), 7.09 (d, 1H), 7.63 - 7.68 (m, 2H), 7.85 (d, 2H), 8.17 (d, 1H), 8.70 (d, 2H), 9.56 (s, 1H). |
| **32** | XXXVI, XXIX | 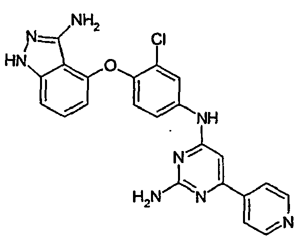 | MS (ESI pos.): m/z = 445 (M+H)⁺ HPLC (Methode 7): Rₜ = 3.40 min. |
| **33** | XII, LIX | 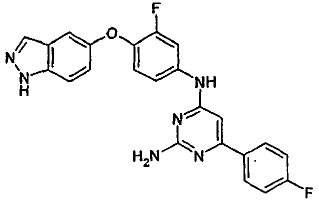 | MS (ESI pos.): m/z = 431 (M+H)⁺ HPLC (Methode 7): Rₜ = 4.18 min. |
| 34 | XIII, LIX | 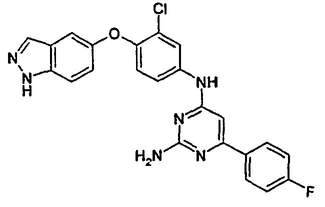 | MS (ESI pos.): m/z = 447 (M+H)⁺ HPLC (Methode 7): Rₜ = 4.21 min. |
| **35** | LIII, LIX | 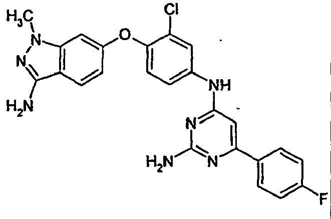 | MS (ESI pos.): m/z = 476 (M+H)⁺ LC-MS (Methode 9): Rₜ = 3.73 min. |
| 36 | LIV, LIX | 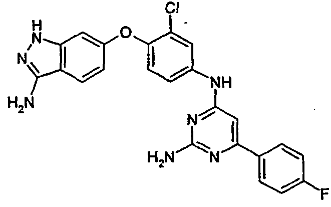 | MS (ESI pos.): m/z = 462 (M+H)⁺ |
| | | | LC-MS (Methode 9): Rₜ = 3.66 min. |
| | | | ¹H-NMR (DMSO-d₆, 400 MHz): δ = 5.33 (s, 2H), 6.45-6.48 (m, 4H), 6.66 (d, 1H), 7.15 (d, 1H), 7.32 (dt, 2H), 7.67 (mc, 2H), 8.16 (d, 1H), 9.46 (s, 1H), 11.15 (s, 1H). |
| 37 | XXXVI, LIX | 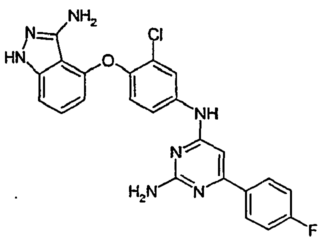 | MS (ESI pos.): m/z = 462 (M+H)⁺ |
| | | | HPLC (Methode 7): Rₜ = 3.91 min. |
| | | | ¹H-NMR (DMSO-d₆, 400 MHz): δ = 5.05 (br. s, 2H), 5.91 (d, 1H), 6.47 (s, 3H), 6.90 (d, 1H), 7.06 (t, 1H), 7.24 (d, 1H), 7.32 (t, 2H), 7.72 (dd, 1H), 7.99 (dd, 2H), 8.18 (d, 1H), 9.47 (s, 1H), 11.59 (s, 1H). |
| **38** | XIX, LVII | 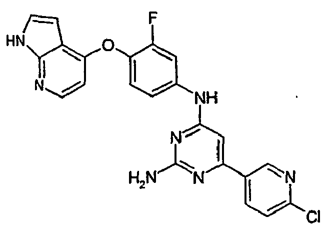 | MS (ESI pos.): m/z = 448 (M+H)⁺ |
| | | | LC-MS (Methode 10): Rₜ = 2.21 min. |
| | | | ¹H-NMR (DMSO-d₆, 400 MHz): δ = 6.27 (dd, 1H), 6.37 (d, 1H), 6.55 (s, 1H), 6.65 (br. s, 2H), 7.31 (t, 1H), 7.37 (dd, 1H), 7.66 (d, 1H), 8.07 (d, 1H), 8.28 (dd, 1H), 8.32 (dd, 1H), 8.93 (d, 1H), 9.66 (s, 1H), 11.75 (s, 1H). |
| 39 | XIX, LVIII | 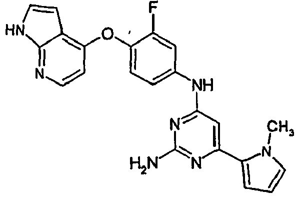 | MS (ESI pos.): m/z = 416 (M+H)⁺ |
| | | | LC-MS (Methode 9): Rₜ = 1.94 min. |
| | | | ¹H-NMR (400 MHz, DMSO-d₆): δ = 3.97 (s, 3H), 6.09 (s, 1H), 6.25 (m, 2H), 6.36 (d, 1H), 6.35 (m, 3H), 6.53 (m, 1H), 6.87 (s, 1H), 7.28 (t, 1H), 7.36 (m, 2H), 8.06 (d, 1H), 8.24 (dd, 1H), 9.38 (s, 1H), 11.74 (s, 1H) |
| 40 | XIX, LXXIII | 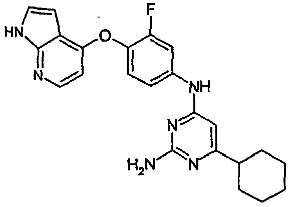 | MS (ESI pos.): m/z = 419.2 (M+H)⁺ |
| | | | HPLC (Methode 7): Rₜ = 3.98 min. |
| **41** | XII, LVII | 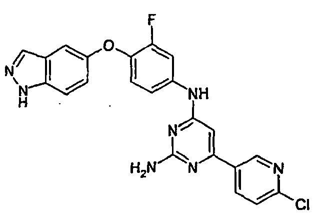 | MS (ESI pos.): m/z = 448 (M+H)⁺ |
| | | | LC-MS (Methode 2): Rₜ = 2.87 min. |
| **42** | XII, LX | 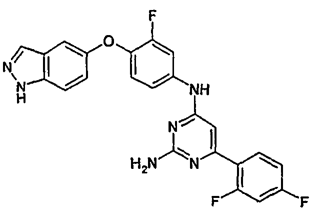 | MS (ESI pos.): m/z = 449 (M+H)⁺ |
| | | | HPLC (Methode 7): Rₜ = 4.18 min. |
| **43** | XII, LXI | 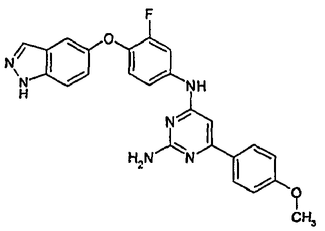 | MS (ESI pos.): m/z = 438 (M+H)⁺ HPLC (Methode 11): Rₜ = 4.17 min. |
| **44** | XII, LVI | 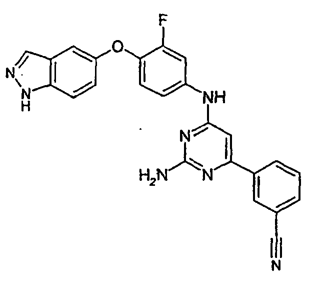 | MS (ESI pos.): m/z = 438 (M+H)⁺ |
| | | | HPLC (Methode 11): Rₜ = 4.06 min. |
| | | | ¹H-NMR (DMSO-d₆, 400 MHz): δ = 6.55 (s, 1H), 6.57 (s, 2H), 7.10 (t, 1H), 7.14-7.18 (m, 2H), 7.34 (dd, 1H), 7.55 (d, 1H), 7.72 (t, 1H), 7.94 (d, 1H), 7.98 (s, 1H), 8.16-8.24 (m, 2H), 8.34 (s, 1H), 9.52 (s, 1H), 13.06 (s, 1H). |
| **45** | XII, LXII | 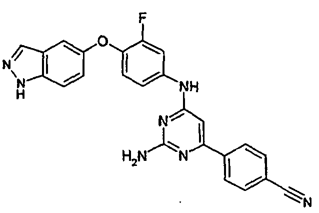 | MS (ESI pos.): m/z = 438 (M+H)⁺ HPLC (Methode 11): Rₜ = 4.06 min. |
| **46** | XII, LXIII | 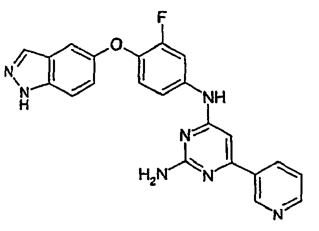 | MS (ESI pos.): m/z = 414 (M+H)⁺ HPLC (Methode 11): Rₜ = 3.69 min. |
| 47 | XII, LXIV | 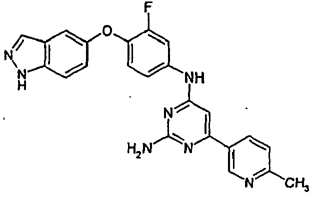 | MS (ESI pos.): m/z = 428 (M+H)⁺ HPLC (Methode 7): Rₜ = 3.64 min. |
| 48 | XII, LXV | 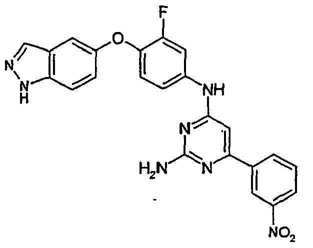 | MS (ESI pos.): m/z = 458 (M+H)⁺ HPLC (Methode 7): Rₜ = 4.13 min. |
| 49 | XIII, LXVI | 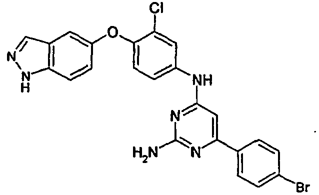 | MS (ESI pos.): m/z = 507 (M+H)⁺ HPLC (Methode 7): Rₜ = 4.35 min. |
| **50** | XIII, LXVII | 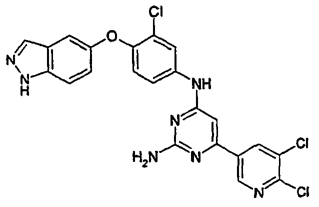 | MS (ESI pos.): m/z = 498 (M+H)⁺ |
| | | | HPLC (Methode 7): Rt = 4.27 min. |
| | | | ¹H-NMR (DMSO-d₆, 400 MHz): δ = 6.56 (s, 1H), 6.62 (br. s, 2H), 7.03 (d, 1H), 7.11-7.17 (m, 2H), 7.57 (d, 1H), 7.63 (dd, 1H), 7.98 (s, 1H), 8.12 (d, 1H), 8.53 (d, 1H), 8.87 (d, 1H), 9.52 (s, 1H), 13.07 (s, 1H). |
| **51** | XIII, LVII | 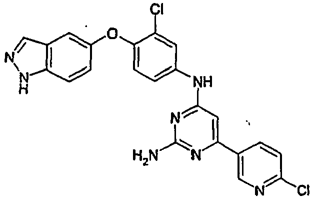 | MS (ESI pos.): m/z = 466 (M+H)⁺ |
| | | | HPLC (Methode 7): Rₜ = 4.11 min. |
| | | | ¹H-NMR (DMSO-d₆, 400 MHz): δ = 6.51 (s, 1H), 6.57 (br. s, 2H), 7.03 (d, 1H), 7.12-7.16 (m, 2H), 7.56 (d, 1H), 7.62-7.66 (m, 2H), 7.98 (s, 1H), 8.13 (d, 1H), 8.30 (dd, 1H), 8.92 (d, 1H), 9.51 (s, 1H), 13.07 (s, 1H). |
| **52** | XIII, LXVIII | 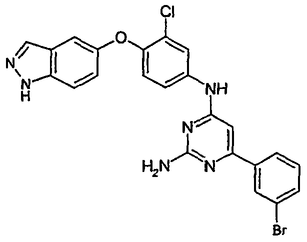 | MS (ESI pos.): m/z = 508 (M+H)⁺ HPLC (Methode 7): Rₜ = 4.33 min. |

### Beispiel 53

### Methyl-2-amino-6-{[3-fluor-4-(1H-pyrrolo[2,3-b]pyridin-4-yloxy)phenyl]amino}-pyrimidin-4-carboxylat

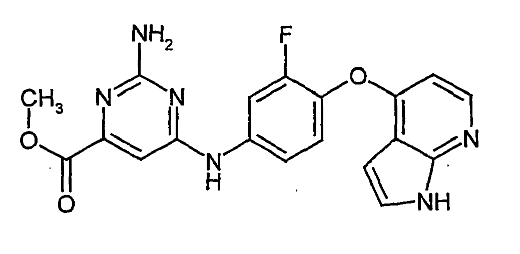

0.209 g (1.12 mmol) Methyl-2-amino-6-chlorpyrimidin-4-carboxylat (hergestellt nach G. Doyle Daves, Fred Baiocchi, Roland K. Robins, and C. C. Cheng, *J. Org. Chem*., **26** (1961), 2755-2763) und 0.4 g (1.12 mmol) [3-Fluor-4-(1H-pyrrolo[2,3-b]pyridin-4-yloxy)phenyl]amin (Beispiel XIX) werden in 5 ml Wasser und 5 ml Ethanol suspendiert. Dazu werden 0.11 ml (1.34 mmol) 37%ige Salzsäure gegeben. Das Gemisch wird bei 100°C 18 Stunden nachgerührt. Nach dem Abkühlen wird es mit einer wässrigen gesättigten Natriumhydrogencarbonatlösung neutralisiert. Der resultierende Niederschlag wird abgesaugt und getrocknet. Man erhält 0.46 g Produkt (68% d. Th.).
HPLC (Methode 11): Rₜ = 3.53 min.
MS (ESIpos): m/z = 395 (M+H)⁺
¹H-NMR (400 MHz, DMSO-d₆): δ = 3.82 (s, 3H), 6.17 (s, 1H), 6.25 (dd, 1H), 6.36 (d, 1H), 6.68 (s, 1H), 6.78 (s, 2H), 7.31 (t, 1H), 7.37 (dd, 2H), 7.39 (dd, 1H), 8.06 (d, 1H), 8.25 (dd, 1H), 9.76 (s, 1H), 11.75 (s, 1H).

### Beispiel 54

### Methyl-2-amino-6-{[3-fluor-4-(1H-indazol-5-yloxy)phenyl]amino}pyrimidin-4-carboxylat

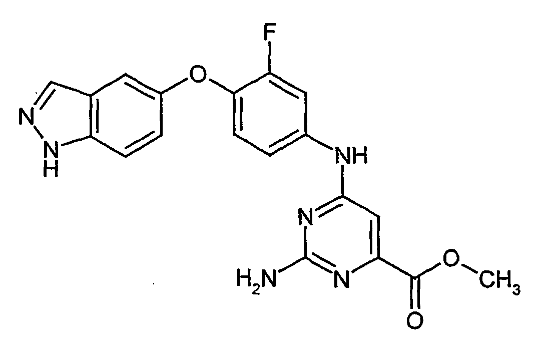

Analog zu Beispiel 53 wird die Titelverbindung aus 1.01 g (4.05 mmol) 5-(4-Amino-2-fluorphenoxy)-1H-indazol (aus Beispiel XII) und 0.84 g (4.06 mmol) Methyl-2-amino-6-chlorpyrimidin-4-carboxylat synthetisiert.
Ausbeute: 1.55 g (93% d. Th.)
LC-MS (Methode 16): Rₜ = 1.56 min.
MS (ESI pos.): m/z = 395 (M+H)⁺
¹H-NMR (300 MHz, DMSO-d₆): δ = 3.85 (s, 3H), 6.72 (s, 1H), 7.101 (t, 1H), 7.15 (dd, 1H), 7.20 (d, 1H), 7.34 (m, 1H), 7.56 (d, 1H), 7.98 (s, 1H), 8.16 (dd, 1H), 10.04 (s, 1H), 13.06 (s, 1H).

### Beispiel 55

### N⁴-[3-Fluor-4-(1H-pyrrolo[2,3-b]pyridin-4-yloxy)phenyl]-6-(piperazin-1-yl-carbonyl)pyrimidin-2,4-diamin

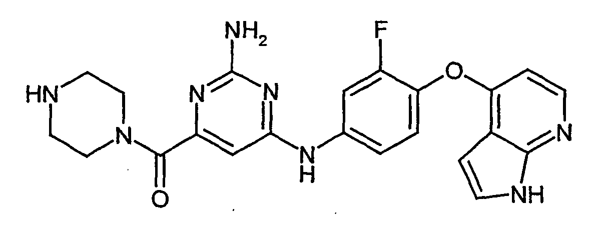

0.072 g (0.18 mmol) Methyl-2-amino-6-{[3-fluor-4-(1H-pyrrolo[2,3-b]pyridin-4-yl-oxy)phenyl]amino}pyrimidin-4-carboxylat (aus Beispiel 53) werden in 3 ml Wasser suspendiert. 0.9 ml einer 1 molaren Natriumhydroxidlösung werden dazugegeben. Die Reaktionsmischung wird bei 100°C 18 Stunden gerührt. Nach dem Abkühlen wird das Gemisch mit 0.95 ml einer 1N Salzsäurelösung auf pH 4.5 gebracht. Der gebildete Niederschlag wird abgesaugt und getrocknet. Der Feststoff wird in DMF suspendiert. Es werden nacheinander 0.066 mg (0.175 mmol) HATU, 0.025 g (0.184 mmol) HOAT, 0.064 ml (0.368 mmol) Diisopropylethylamin und 0.031 g (0.368 mmol) Piperazin dazugegeben. Die Reaktionsmischung wird bei 40°C 5 Stunden gerührt. Nach dem Abkühlen wird die Lösung mittels präparativer HPLC gereinigt. Man erhält 0.012 g (15 % d. Th.) Produkt.
LC-MS (Methode 14): Rₜ = 1.24 min.
MS (ESIpos): m/z = 449 (M+H)⁺
¹H-NMR (200 MHz, DMSO-d₆): δ = 2.76 (m, 4H), 3.54 (m, 4H), 6.05 (s, 1H), 6.25 (dd, 1H), 6.36 (d, 1H), 6.78 (s, 2H), 7.30 (t, 1H), 7.36 (m, 2H), 8.07 (d, 1H), 8.23 (dd, 1H), 9.62 (s, 1H), 11.77 (s, 1H).

Analog zu Beispiel 55 werden hergestellt:

| **Bsp.-Nr.** | **Edukte (Bsp.-Nr.)** | **Struktur** | **MS, HPLC, LC-MS,** ^{**1**}**H-NMR** |
|---|---|---|---|
| **56** | 53 | 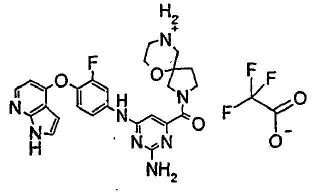 | LC-MS (Methode 16): Rₜ = 1.26 min. |
| | | | MS (ESIpos): m/z = 505 (M+H)⁺ |
| **57** | 53 | 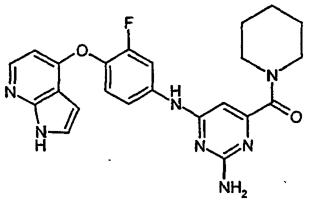 | LC-MS (Methode 9): Rₜ = 2.59 min. |
| | | | MS (ESIpos): m/z = 505 (M+H)⁺ |
| **58** | 53 | 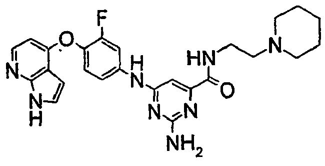 | LC-MS (Methode 9): Rₜ = 2.32 min. |
| | | | MS (ESIpos): m/z = 491 (M+H)⁺ |
| **59** | 54 | 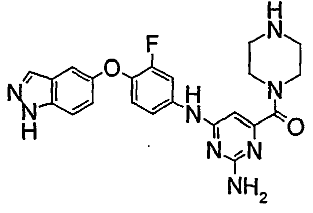 | LC-MS (Methode 10): Rt = 1.85 min. |
| | | | MS (ESIpos): m/z = 449 (M+H)⁺ |

### Beispiel 60

### 6-Chlor-N⁴-[3-fluor-4-(1H-pyrrolo[2,3-b]pyridin-4-yloxy)phenyl]pyrimidin-2,4-diamin

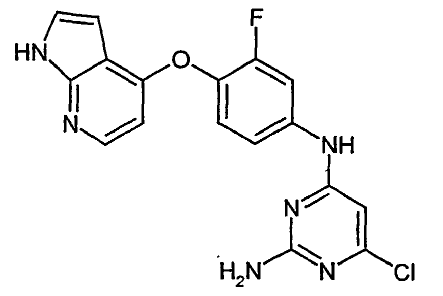

Analog zu Beispiel 8 wird die Titelverbindung aus 266 mg (1.09 mmol) 3-Fluor-4-(1H-pyrrolo[2,3-b]pyridin-4-yloxy)anilin (aus Beispiel XIX) und 179 mg (1.09 mmol) 4,6-Dichlor-2-pyrimidinamin synthetisiert.
Ausbeute: 275 mg (68% d. Th.)
LC-MS (Methode 10): Rₜ = 2.41 min.
MS (ESI pos.): m/z = 371 (M+H)⁺
¹H-NMR (DMSO-d₆, 300 MHz): δ = 6.04 (s, 1H), 6.25 (dd, 1H), 6.36 (d, 1H), 6.86 (br. s, 2H), 7.25 - 7.37 (m, 3H), 8.07 (d, 1H), 8.17 (dd, 1H), 9.60 (s, 1H), 11.73 (s, 1H).

### Beispiel 61

### 6-Chlor-N⁴-[3,5-difluor-4-(1H-pyrrolo[2,3-b]pyridin-4-yloxy)phenyl]pyrimidin-2,4-diamin

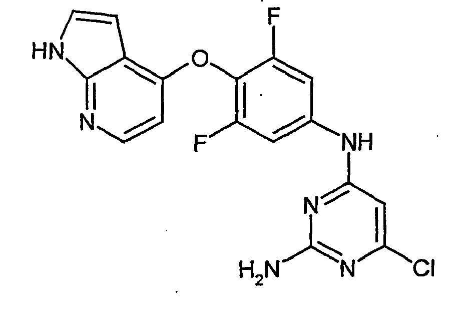

Analog zu Beispiel 8 wird die Titelverbindung aus 384 mg (1.47 mmol) [3,5-Difluor-4-(1H-pyrrolo[2,3-b]pyridin-4-yloxy)phenyl]amin (aus Beispiel XXXIV) und 241 mg (1.47 mmol) 4,6-Dichlor-2-pyrimidinamin synthetisiert.
Ausbeute: 550 mg (96% d. Th.)
HPLC (Methode 7): Rₜ = 2.41 min.
MS (ESI pos.): m/z = 389 (M+H)⁺
¹H-NMR (DMSO-d₆, 400 MHz): δ = 6.04 (s, 1H), 6.31 (dd, 1H), 6.42 (d, 1H), 7.00 (br. s, 2H), 7.41 (dd, 1H), 7.75 (d, 2H), 8.08 (d, 1H), 9.79 (s, 1H), 11.83 (s, 1H).

### Beispiel 62

### N⁴-[3-Fluor-4-(1H-pyrrolo[2,3-b]pyridin-4-yloxy)phenyl]-6-piperazin-1-ylpyrimidin-2,4-diamin

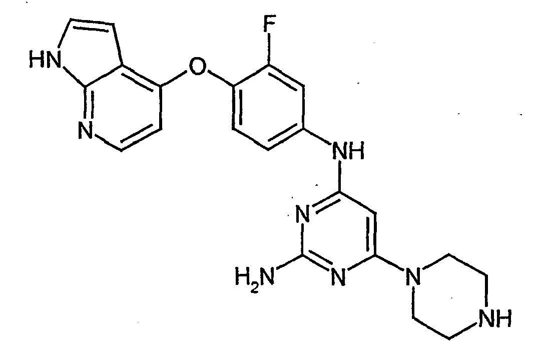

75 mg (0.20 mmol) 6-Chlor-N⁴-[3-fluor-4-(1H-pyrrolo[2,3-b]pyridin-4-yloxy)-phenyl]pyrimidin-2,4-diamin (aus Beispiel 60) werden in 3 ml 1-Butanol gelöst. Man gibt 0.35 ml N-Ethyldiisopropylamin und 70 mg (0.81 mmol) Piperazin hinzu und erhitzt 5 Stunden am Rückfluss. Dann wird das Lösungsmittel im Vakuum entfernt und der Rückstand durch präparative HPLC gereinigt.
Ausbeute: 37 mg (44% d. Th.)
LC-MS (Methode 12): Rₜ = 1.60 min.
MS (ESI neg.): m/z = 419 (M-H)⁻
¹H-NMR (DMSO-d₆, 400 MHz): δ = 2.75 - 2.82 (m, 4H), 3.24 - 3.26 (m, 2H), 3.41 -3.44 (m, 2H), 4.18 (mc, 1H), 5.41 (s, 1H), 6.02 (br. s, 2H), 6.34 (mc, 1H), 6.42 (d, 1H), 7.30 (t, 1H), 7.35 - 7.38 (m, 1H), 7.44 (mc, 1H), 8.14 (d, 1H), 8.25 (dd, 1H), 9.07 (s, 1H), 11.81 (s, 1H).

### Beispiel 63

### N⁴-[3-Fluor-4-(1H-pyrrolo[2,3-b]pyridin-4-yloxy)phenyl]-6-morpholin-4-yl-pyrimidin-2,4-diamin

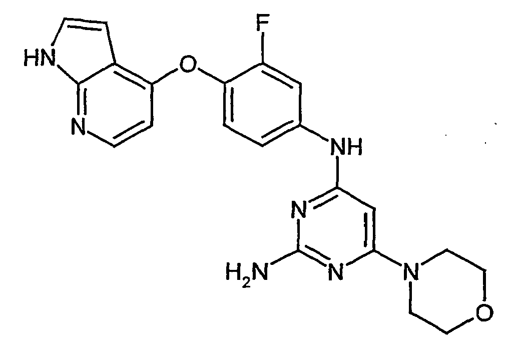

52 mg (0.14 mmol) 6-Chlor-N⁴-[3-fluor-4-(1H-pyrrolo[2,3-b]pyridin-4-yloxy)-phenyl]pyrimidin-2,4-diamin (aus Beispiel 60) werden in 2.5 ml 2-Ethylhexanol gelöst. Man gibt 0.24 ml N-Ethyldiisopropylamin und 36 mg (0.42 mmol) Morpholin hinzu und erhitzt 20 Stunden auf 150°C Badtemperatur. Nach dem Abkühlen wird etwas DMF zugesetzt und an Kieselgel 60 chromatographiert (Gradientensäule: Eluens: DCM : Methanol = 50 : 1, dann 3 : 1). Man erhält ein Öl, das mittels präparativer HPLC gereinigt wird.
Ausbeute: 13 mg (22% d. Th.)
HPLC (Methode 7): Rₜ = 3.61 min.
MS (ESI pos.): m/z = 422 (M+H)⁺
¹H-NMR (DMSO-d₆, 400 MHz): δ = 3.39 (mc, 4H), 3.66 (mc, 4H), 5.33 (s, 1H), 6.00 (br. s, 2H), 6.25 (mc, 1H), 6.32 (d, 1H), 7.22 (t, 1H), 7.27 - 7.30 (m 1H), 7.35 (dd, 1H), 8.05 (d, 1H), 8.17 (d, 1H), 9.05 (s, 1H), 11.73 (s, 1H).

Analog zu Beispiel 63 werden hergestellt:

| **Bsp.-Nr.** | **Edukte (Bsp.-Nr.)** | **Struktur** | **MS, HPLC, LC-MS,** ^{**1**}**H-NMR** |
|---|---|---|---|
| **64** | 61 | 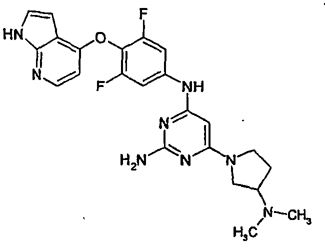 | MS (ESI pos.): m/z = 467 (M+H)⁺ |
| | | | LC-MS (Methode 15): Rₜ = 1.05 min. |
| **65** | 61 | 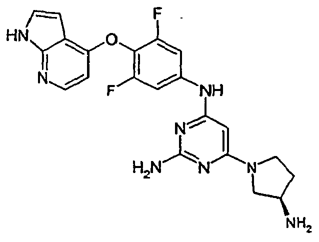 | MS (ESI pos.): m/z = 439 (M+H)⁺ |
| | | | LC-MS (Methode 9): Rₜ = 1.50 min. |
| **66** | 60 | 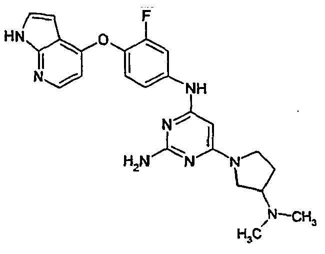 | MS (ESI neg.): m/z = 447 (M-H)⁻ |
| | | | LC-MS (Methode 12): Rₜ = 1.59 min. |
| **67** | 60 | 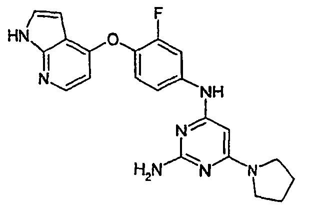 | MS (ESI pos.): m/z = 406 (M+H)⁺ |
| | | | LC-MS (Methode 16): Rₜ = 1.50 min. |
| **68** | 60 | 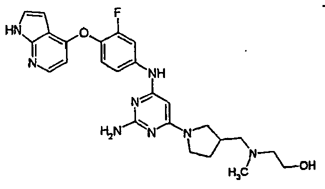 | MS (ESI pos.): m/z = 493 (M+H)⁺ |
| | | | HPLC (Methode 11): Rₜ = 3.46 min. |
| **69** | 60 | 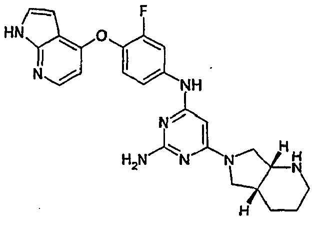 | MS (ESI pos.): m/z = 461 (M+H)⁺ |
| | | | LC-MS (Methode 10): Rₜ = 1.67 min. |
| **70** | 60 | 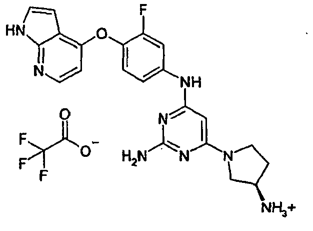 | LC-MS (Methode 12): Rₜ = 1.52 min. |
| **71** | 60 | 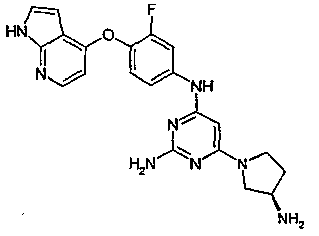 | MS (ESI neg.): m/z = 419 (M-H)⁻ |
| | | | LC-MS (Methode 12): Rₜ = 1.53 min. |
| **72** | 60 | 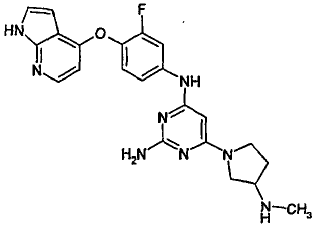 | MS (ESI pos.): m/z = 435 (M+H)⁺ |
| | | | LC-MS (Methode 13): Rₜ = 1.10 min. |
| **73** | 60 | 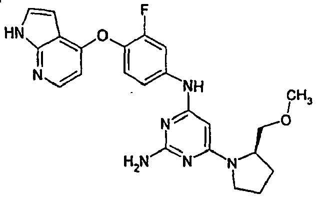 | MS (ESI pos.): m/z = 450 (M+H)⁺ |
| | | | LC-MS (Methode 9): Rₜ = 1.95 min. |
| **74** | 60 | 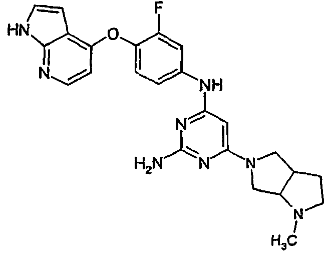 | MS (ESI pos.): m/z = 461 (M+H)⁺ |
| | | | LC-MS (Methode 16): Rₜ = 1.18 min. |
| **75** | 60 | 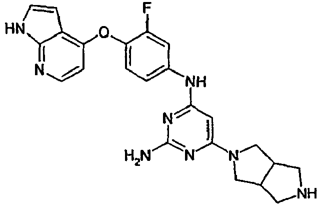 | MS (ESI pos.): m/z = 447 (M+H)⁺ |
| | | | LC-MS (Methode 9): Rₜ = 2.01 min. |
| **76** | 60 | 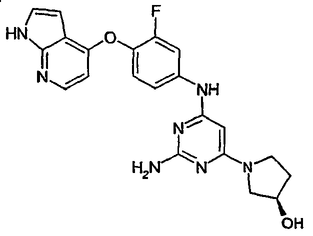 | MS (ESI pos.): m/z = 422 (M+H)⁺ |
| | | | LC-MS (Methode 16): Rₜ = 1.36 min. |
| **77** | 60 | 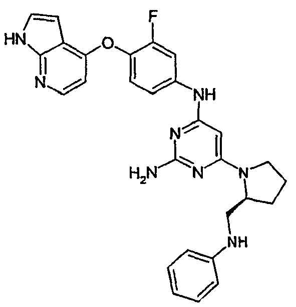 | MS (ESI pos.): m/z = 511 (M+H)⁺ |
| | | | HPLC (Methode 11): Rₜ = 3.94 min. |
| **78** | XIX | 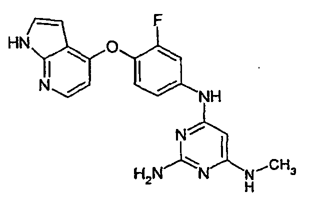 | MS (ESI pos.): m/z = 366 (M+H)⁺ |
| | | | LC-MS (Methode 16): Rₜ = 1.39 min. |
| **79** | 60 | 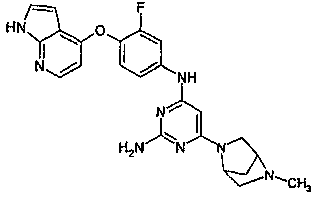 | MS (ESI pos.): m/z = 447 (M+H)⁺ |
| | | | LC-MS (Methode 9): Rₜ = 1.37 min. |
| **80** | 60 | 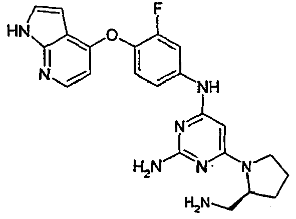 | MS (ESI pos.): m/z = 435 (M+H)⁺ |
| | | | LC-MS (Methode 10): Rₜ = 2.13 min. |
| **81** | 60 | 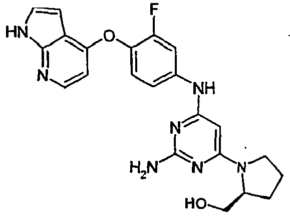 | MS (ESI pos.): m/z = 436 (M+H)⁺ |
| | | | LC-MS (Methode 9): Rₜ = 1.82 min. |
| **82** | 60 | 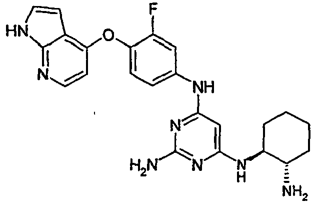 | MS (ESI pos.): m/z = 449 (M+H)⁺ |
| | | | HPLC (Methode 7): Rₜ = 3.57 min. |
| **83** | 60 | 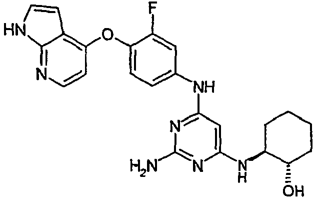 | MS (ESI pos.): m/z = 450 (M+H)⁺ |
| | | | LC-MS (Methode 15): Rₜ = 1.33 min. |
| **84** | 60 | 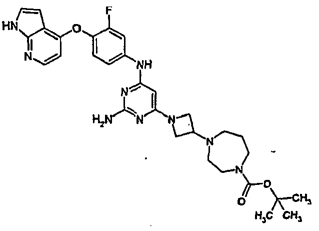 | MS (ESI pos.): m/z = 590 (M+H)⁺ |
| | | | LC-MS (Methode 14): Rₜ = 1.57 min. |
| **85** | 60 | 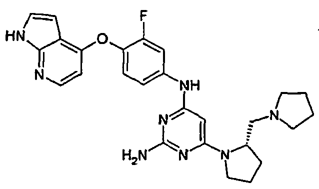 | MS (ESI pos.): m/z = 489 (M+H)⁺ |
| | | | LC-MS (Methode 13): Rₜ = 1.34 min. |
| **86** | 60 | 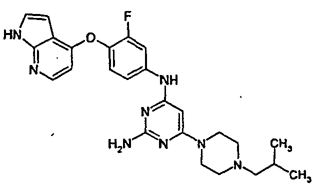 | MS (ESI pos.): m/z = 477 (M+H)⁺ |
| | | | LC-MS (Methode 17): Rₜ = 1.21 min. |
| **87** | 60 | 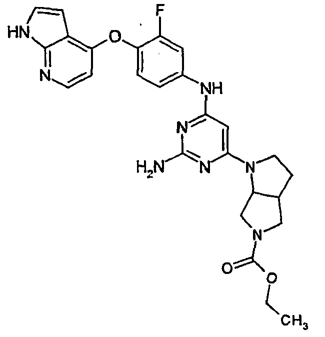 | MS (ESI pos.): m/z = 519 (M+H)⁺ |
| | | | LC-MS (Methode 10): Rₜ = 2.12 min. |
| **88** | 60 | 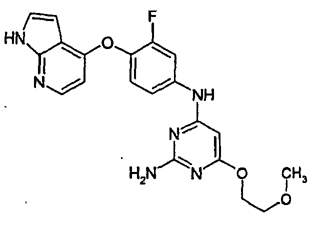 | MS (ESI pos.): m/z = 411 (M+H)⁺ |
| | | | LC-MS (Methode 9): Rₜ = 1.93 min. |
| | | | ¹H-NMR (DMSO-d₆, 400 MHz): δ = 3.30 (s, 3H), 3.59 (dd, 2H), 4.29 (dd, 2H), 5.39 (s, 1H), 6.25 (d, 1H), 6.34 (d, 1H), 6.41 (br. s, 2H), 7.22-7.3 6 (m, 3H), 8.06 (d, 1H), 8.12-8.18 (m, 1H), 9.27 (s, 1H), 11.74 (s, 1H). |
| **89** | 8 | 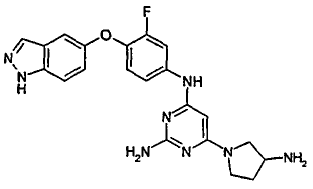 | MS (ESI pos.): m/z = 421 (M+H)⁺ |
| | | | LC-MS (Methode 2): Rₜ = 2.20 min. |
| **90** | 8 | 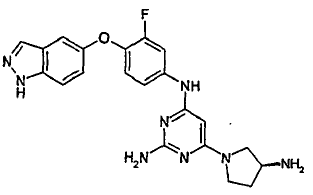 | MS (ESI pos.): m/z = 421 (M+H)⁺ |
| | | | LC-MS (Methode 2): Rₜ = 2.20 min. |
| **91** | 8 | 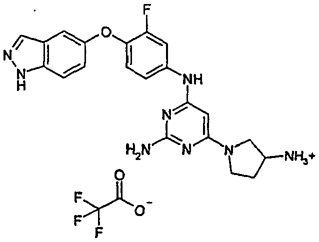 | MS (ESI pos.): m/z = 421 (M+H)⁺ |
| | | | LC-MS (Methode 8): Rₜ = 1.91 min. |
| **92** | 8 | 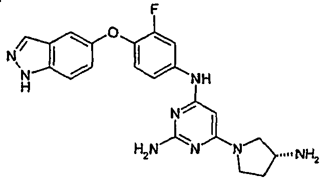 | MS (ESI pos.): m/z = 421 (M+H)⁺ |
| | | | LC-MS (Methode 2): Rₜ = 2.20 min. |
| **93** | 8 | 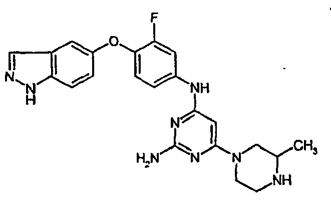 | MS (ESI pos.): m/z = 435 (M+H)⁺ |
| | | | HPLC (Methode 7): Rₜ = 3.56 min. |
| **94** | 8 | 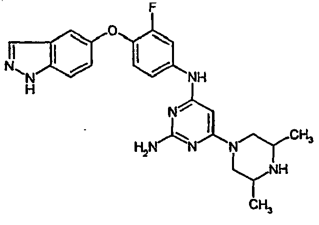 | MS (ESI pos.): m/z = 449 (M+H)⁺ |
| | | | HPLC (Methode 7): Rₜ = 3.59 min. |
| **95** | 8 | 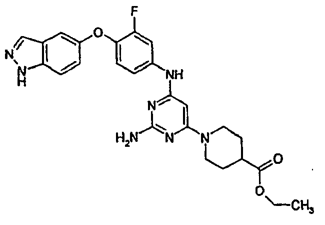 | MS (ESI pos.): m/z = 492 (M+H)⁺ |
| | | | LC-MS (Methode 8): Rₜ = 2.45 min. |
| **96** | 8 | 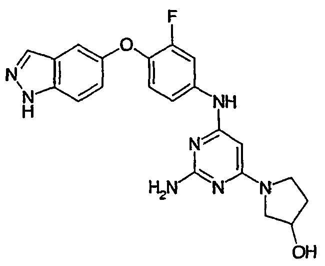 | MS (ESI pos.): m/z = 422 (M+H)⁺ |
| | | | LC-MS (Methode 2): Rₜ = 2.50 min. |
| **97** | 9 | 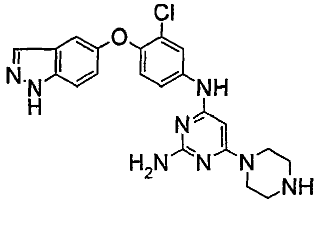 | MS (ESI pos.): m/z = 435 (M+H)⁺ |
| | | | LC-MS (Methode 10): Rₜ = 1.63 min. |
| **98** | 9 | 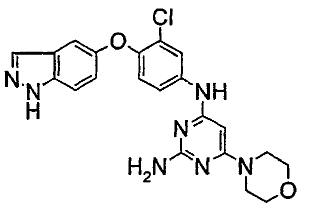 | MS (ESI pos.): m/z = 438 (M+H)⁺ |
| | | | HPLC (Methode 7): Rₜ = 3.89 min. |
| **99** | 60 | 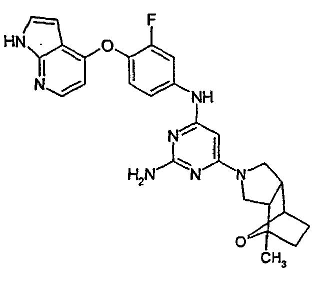 | MS (ESI pos.): m/z = 488 (M+H)⁺ |
| | | | HPLC (Methode 7): Rₜ = 3.83 min. |

### Beispiel 100

### 6-[3-(1,4-Diazepan-1-yl)azetidin-1-yl]-N4-[3-fluor-4-(1H-pyrrolo[2,3-b]pyridin-4-yloxy)phenyl]pyrimidin-2,4-diamin

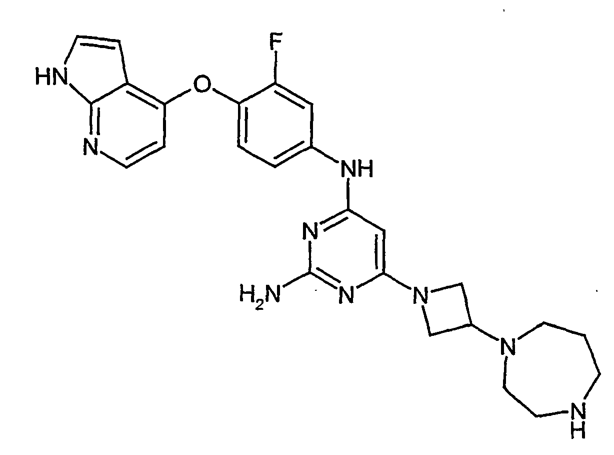

34 mg (0.06 mmol) der Verbindung aus Beispiel 84 werden in 1 ml 4 molarer Chlorwasserstofflösung in Dioxan bei RT gerührt. Dann wird im Vakuum eingeengt und mit Diethylether verrieben. Man dekantiert ab, trocknet den Rückstand im Hochvakuum und erhält das Hydrochlorid der Titelverbindung.
Ausbeute: 30 mg (96% d. Th.)
LC-MS (Methode 17): Rₜ = 1.18 min.
MS (ESI pos.): m/z = 490 (M+H)⁺

### Beispiel 101

### 3-[(2-Amino-6-{[3-fluor-4-(1H-pyrrolo[2,3-b]pyridin-4-yloxy)phenyl]amino}-pyrimidin-4-yl)amino]propan-1-ol

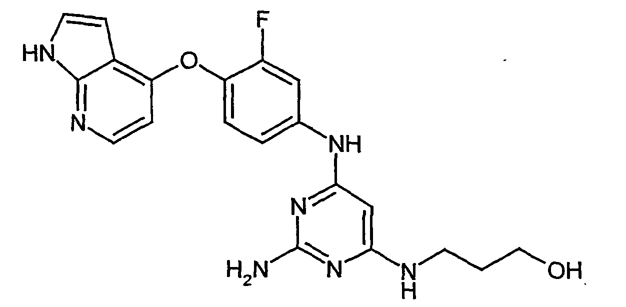

78 mg (0.21 mmol) 6-Chlor-N⁴-[3-fluor-4-(1H-pyrrolo[2,3-b]pyridin-4-yloxy)-phenyl]pyrimidin-2,4-diamin (aus Beispiel 60) werden mit 190 mg (0.84 mmol) 3-[(2,4-Dimethoxybenzyl)amino]propan-1-ol in 1 ml 1-Butanol und 0.37 ml N-Ethyldiisopropylamin im geschlossenen Druckgefäß über Nacht auf 130°C erhitzt. Man entfernt flüchtige Bestandteile im Vakuum und reinigt den Rückstand durch präparative HPLC. Das erhaltene Substrat wird in 2 ml DCM aufgenommen und 0.5 ml TFA zugesetzt. Man rührt 20 min. bei RT, gibt dann auf Essigsäureethylester und schüttelt mit ges. Natriumcarbonatlösung. Man trocknet die organische Phase über Natriumsulfat und entfernt das Lösungsmittel im Vakuum. Der Rückstand wird durch präparative HPLC gereinigt.
Ausbeute: 10 mg (16% d. Th.)
LC-MS (Methode 10): Rₜ = 1.85 min.
MS (ESI pos.): m/z = 410 (M+H)⁺

### Beispiel 102

### N⁴-[3-Fluor-4-(1H-indazol-5-yloxy)phenyl]-6-[4-(trifluormethyl)phenyl]pyrimidin-2,4-diamin

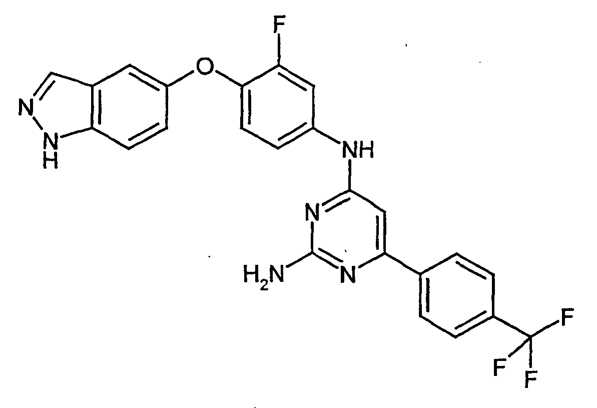

52.55 mg (0.14 mmol) 6-Chlor-N⁴-[3-fluor-4-(1H-indazol-5-yloxy)phenyl]pyrimidin-2,4-diamin (aus Beispiel 8) und 35.71 mg (0.42 mmol) Natriumhydrogencarbonat werden in 2.4 ml Dimethoxyethan und 0.7 ml Wasser 30 Minuten bei 85°C gerührt. Dazu werden 5 mg (0.007 mmol) 1,1'-Bis(diphenylphosphino)ferrocen-palladium(II)-chlorid gegeben. Das Gemisch wird bei 85°C 12 Stunden nachgerührt. Zur Aufarbeitung wird es mit Dimethoxyethan verdünnt und mit einer gesättigten Natriumchloridlösung gewaschen. Die organische Phase wird getrocknet und eingeengt. Der Rückstand wird mittels präparativer HPLC gereinigt. Man erhält 2.80 g (64 % d. Th.) Produkt.
LC-MS (Methode 16): Rₜ = 1.84 min.
MS (ESIpos): m/z = 481 (M+H)⁺
¹H-NMR (200 MHz, DMSO-d₆): δ = 6.57 (s, 3H), 7.10 (dd, 1H), 7.16 (m, 2H), 7.34 (dd, 1H), 7.56 (d, 1H), 7.86 (d, 2H), 7.98 (s, 1H), 8.14 (m, 2H), 8.21 (dd, 1H), 9.56 (s, 1H), 13.07 (s, 1H).

Analog zu Beispiel 102 werden hergestellt:

| **Bsp.-Nr.** | **Edukte (Bsp.-Nr.)** | **Struktur** | **MS, HPLC, LC-MS,** ^{**1**}**H-NMR** |
|---|---|---|---|
| 103 | 8 | 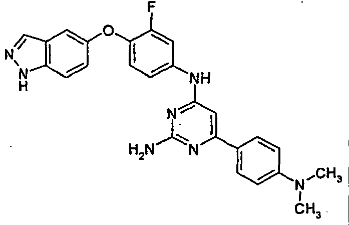 | MS (ESI pos.): m/z = 456 (M+H)⁺ |
| | | | LC-MS (Methode 16): Rₜ = 1.84 min. |
| | | | ¹H-NMR (200 MHz, DMSO-d₆): δ = 2.97 (s, 6H), 6.29 (s, 2H), 6.27 (s, 1H), 6.77 (d, 2H), 7.08 (t, 1H), 7.15 (m, 2H), 7.31 (dd, 1H), 7.54 (d, 1H), 7.82 (d, 2H), 7.97 (s, 1H), 8.19 (dd, 1H), 9.56 (s, 1H), 13.07 (s, 1H). |
| 104 | 8 | 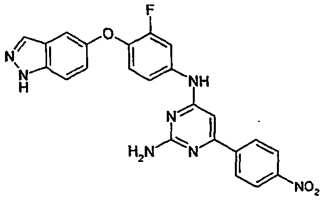 | MS (ESI pos.): m/z = 458 (M+H)⁺ LC-MS (Methode 10): Rₜ = 2.4 min. |
| 105 | 60 | 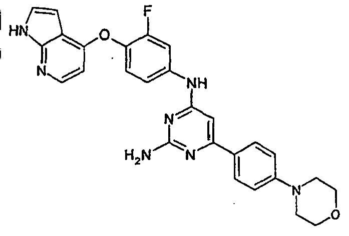 | MS (ESI pos.): m/z = 498 (M+H)⁺ LC-MS (Methode 16): Rₜ = 1.58 min. |
| 106 | 60 | 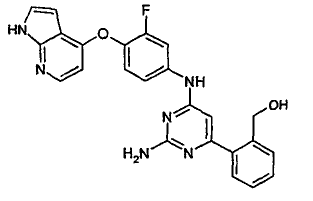 | MS (ESI pos.): m/z = 443 (M+H)⁺ LC-MS (Methode 12): Rₜ = 2.10 min. |
| 107 | 60 | 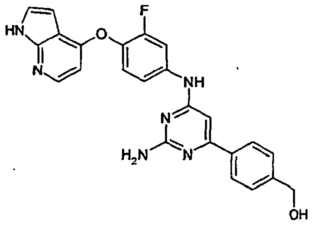 | MS (ESI pos.): m/z = 443 (M+H)⁺ |
| | | | LC-MS (Methode 9): Rₜ = 1.85 min. |
| | | | ¹H-NMR (DMSO-d₆, 400 MHz): δ = 4.56 (d, 2H), 5.27 (t, 1H), 6.27 (dd, 1H), 6.36 (d, 1H), 6.48 (s, 2H), 6.52 (s, 1H), 7.30 (t, 1H), 7.36 - 7.44 (m, 4H), 7.92 (d, 2H), 8.07 (d, 1H), 8.28 (dd, 1H), 9.54 (s, 1H), 11.75 (s, 1H). |
| 108 | 60 | 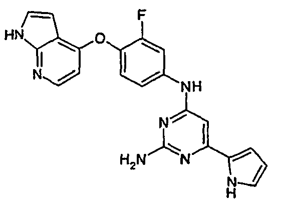 | MS (ESI pos.): m/z = 402 (M+H)⁺ LC-MS (Methode 9): Rₜ = 1.83 min. |

### Beispiel 109

### N⁴-[3-Fluor-4-(1H-indazol-5-yloxy)phenyl]pyrimidin-2,4,6-triamin

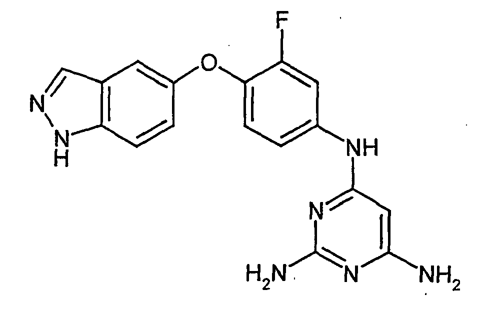

Analog zu Beispiel 25 wird die Titelverbindung aus 150 mg (0.62 mmol) [3-Fluor-4-(1H-indazol-5-yloxy)phenyl]amin (aus Beispiel XII) und 94 mg (0.65 mmol) 6-Chlorpyrimidin-2,4-diamin synthetisiert. Nach Reinigung mittels präparativer HPLC erhält man das Produkt.
Ausbeute: 11mg (5% d. Th.)
LC-MS (Methode 8): Rₜ = 2.10 min.
MS (ESI pos.): m/z = 416 (M+H)⁺
¹H-NMR (DMSO-d₆, 300 MHz): δ = 5.19 (m, 1H), 5.67 (m, 2H), 5.82 (m, 2H), 7.02 (t, 1H), 7.10 - 7.30 (m, 3H), 7.53 (m, 1H), 7.90 - 8.10 (m, 2H), 8.70 (s, 1H), 13.00 (s, 1H).

### Beispiel 110

### 4-[(2-Amino-6-{[3-fluor-4-(1H-pyrrolo[2,3-b]pyridin-4-yloxy)phenyl]amino}-pyrimidin-4-yl)amino]benzonitril

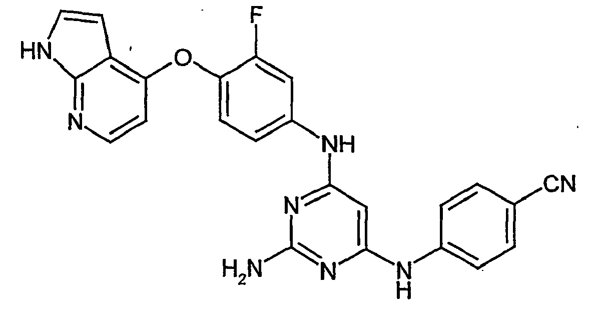

Analog zu Beispiel 25 wird die Titelverbindung aus 51 mg (0.21 mmol) [3-Fluor-4-(1H-pyrrolo[2,3-b]pyridin-4-yloxy)phenyl]amin (aus Beispiel XIX) und 54 mg (0.22 mmol) 4-[(2-Amino-6-chlorpyrimidin-4-yl)amino]benzonitril (aus Beispiel XLVII) synthetisiert. Nach Reinigung mittels präparativer HPLC erhält man das Produkt.
Ausbeute: 8 mg (8% d. Th.)
LC-MS (Methode 8): Rₜ = 2.30 min.
MS (ESI pos.): m/z = 453 (M+H)⁺
¹H-NMR (DMSO-d₆, 300 MHz): δ = 5.62 (s, 1H), 6.25 (m, 1H), 6.30 (s, 2H), 6.35 (d, 1H), 7.25 (m, 1H), 7.30 - 7.40 (m, 2H), 7.65 (d, 2H), 7.90 (d, 2H), 8.00 - 8.19 (m, 2H), 9.15 (s, 1H), 9.35 (s, 1H), 11.70 (s, 1H).

Analog zu Beispiel 110 werden hergestellt:

| **Bsp.-Nr.** | **Edukte (Bsp.-Nr.)** | **Struktur** | **MS, HPLC, LC-MS,** ^{**1**}**H-NMR** |
|---|---|---|---|
| **111** | XIX, XLVIII | 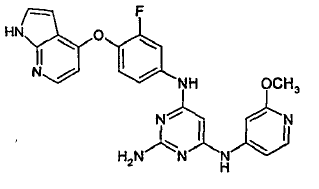 | MS (ESI pos.): m/z = 459 (M+H)⁺ |
| | | | LC-MS (Methode 9): Rₜ =1.95 min. |
| **112** | XIX, XLIX | 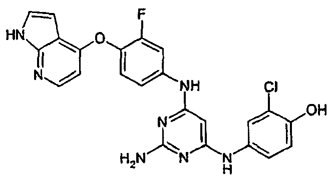 | MS (ESI pos.): m/z = 478 (M+H)⁺ |
| | | | LC-MS (Methode 16): Rₜ =1.53 min. |
| **113** | XIX, XLIII | 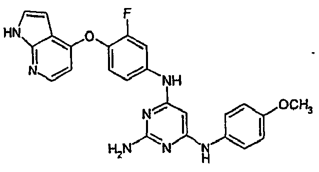 | LC-MS (Methode 2): Rₜ = 2.73 min. |
| **114** | XIX, L | 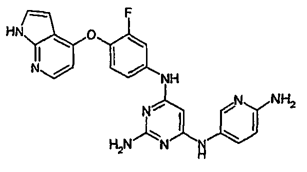 | MS (ESI pos.): m/z = 444 (M+H)⁺ |
| | | | LC-MS (Methode 16): Rₜ = 1.26 min. |
| **115** | XIX, LI | 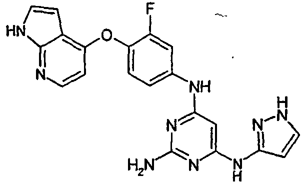 | MS (ESI pos.): m/z = 418 (M+H)⁺ |
| | | | LC-MS (Methode 10): Rₜ = 1.90 min. |

### Beispiel 116

Benzyl-6-[(tert-butylamino) methyl]-N4-[3-fluor-4-(1H-pyrrolo[2,3-b]pyridin-4-yloxy)phenyl]pyrimidin-2,4-diamin 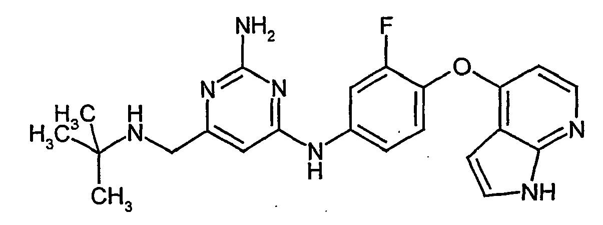 0.060 g (0.28 mmol) 4-[(tert-Butylamino)methyl]-6-chlorpyrimidin-2-amin und 4-[(tert-Butylamino)methyl]-6-brompyrimidin-2-amin (aus Beispiel LXXX) und 0.068 g (0.28 mmol) [3-Fluor-4-(1H-pyrrolo[2,3-b]pyridin-4-yloxy)phenyl]amin (aus Beispiel XIX) werden in 2 ml Wasser und 2 ml Ethanol ssupendiert. Dazu werden 0.051 ml (0.61 mmol) 37%iger Salzsäure gegeben. Das gemisch wird bei 100°C 18 Stunden nachgerührt. Nach dem Abkühlen wird es mit einer 12N Natrium-hydroxidlösung neutralisiert. Die Suspension wird nach Zugabe von 1 ml DMSO wieder in Lösung gebracht und mittels präparativer HPLC gereingt. Man erhält 0.074 g (63%d. Th.) Produkt.
LC-MS (Methode 15): Rₜ = 1.12 min.
MS (ESIpos): m/z = 422 (M+H)⁺
¹H-NMR (200 MHz, DMSO-d₆): δ = 1.11 (s, 9H), 3.52 (s, 2H), 6.17 (s, 1H), 6.27 (m, 1H), 6.34 (m, 3H), 7.27 (t, 1H), 7.39 (m, 2H), 8.06 (d, 1H), 8.24 (dd, 1H), 9.46 (s, 1H), 11.76 (s, 1H).

### Beispiel 117

### N⁴-[3-Fluor-4-(1H-pyrrolo[2,3-b]pyridin-4-yloxy)phenyl]-6-(piperidin-1-ylmethyl)-pyrimidin-2,4-diamin

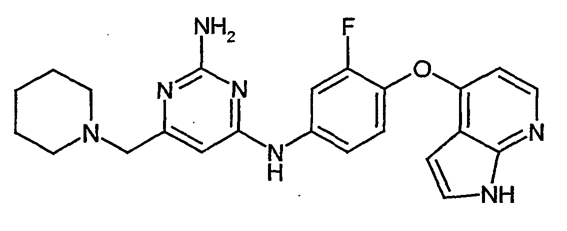

Analog zu Beispiel 116 wird die Titelverbindung durch Reaktion von 33 mg (0.137 mmol) [3-Fluor-4-(1H-pyrrolo[2,3-b]pyridin-4-yloxy)phenyl]amin (aus Beispiel XIX) und 31 mg (0.137 mmol) 4-Chlor-6-(piperidin-1-ylmethyl)pyrimidin-2-amin (aus Beispiel LXXXI) gewonnen.
Ausbeute: 13 mg (22% d. Th.)
LC-MS (Methode 13): Rₜ = 1.39 min.
MS (ESIpos): m/z = 218 1/2(M+H)²⁺
¹H-NMR (200 MHz, DMSO-d₆): δ = 1.42 (m, 2H), 1.54 (m, 4H), 2.39 (m, 4H), 3.17 (d, 2H), 4.06 (q, 1H), 6.19 (s, 1H), 6.24 (dd, 1H), 6.28 (s, 2H), 6.35 (d, 1H), 7.26 (t, 1H), 7.37 (m, 2H), 8.06 (d, 1H), 8.21 (dd, 1H), 9.37 (s, 1H), 11.71 (s, 1H).

Analog zu Beispiel 116 werden hergestellt:

| **Bsp.-Nr.** | **Edukte (Bsp.-Nr.)** | **Struktur** | **MS, HPLC, LC-MS,** ^{**1**}**H-NMR** |
|---|---|---|---|
| **118** | LXXXV XIX | 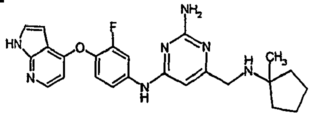 | LC-MS (Methode 15): Rₜ = 1.22 min. |
| | | | MS (ESIpos): m/z = 448 (M+H)⁺ |
| | | | ¹H-NMR (300 MHz, DMSO-d₆): δ = 1.14 (s, 3H), 1.24 (m, 1H), 1.40 (m, 2H), 1.62 (m, 6H), 3.43 (s, 2H), 6.23 (m, 4H), 6.34 (d, 1H), 7.25 (t, 1H), 7.37 (m, 2H), 8.06 (d, 1H), 8.21 (dd, 1H), 9.37 (s, 1H), 11.71 (s, 1H). |
| **119** | LXXXIX XIX | 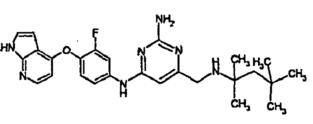 | LC-MS (Methode 15): Rₜ = 1.53 min. MS (ESIpos): m/z = 478 (M+H)⁺ |
| **120** | LXXXII XIX | 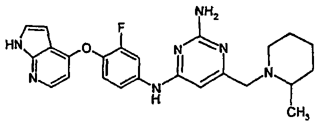 | LC-MS (Methode 15): Rₜ = 1.19 min. MS (ESIpos): m/z = 448 (M+H)⁺ |
| **121** | LXXXVIII XIX | 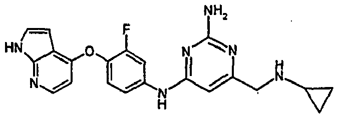 | LC-MS (Methode 15): Rₜ = 1.14 min. |
| | | | MS (ESIpos): m/z = 204 1/2(M+H)²⁺ |
| | | | ¹H-NMR (200 MHz, DMSO-d₆): δ = 0.28 (m, 2H), 0.37 (m, 2H), 2.12 (m, 1H), 3.50 (s, 2H), 6.11 (s, 1H), 6.26 (m, 1H), 6.36 (m, 3H), 7.27 (t, 1H), 7.36 (m, 2H), 8.06 (d, 1H), 8.23 (dd, 1H), 9.40 (s, 1H), 11.75 (s, 1H). |
| **122** | LXXXTV XIX | 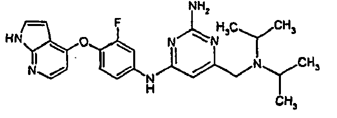 | LC-MS (Methode 13): Rₜ = 1.59 min. MS (ESIpos): m/z = 451 (M+H)⁺ |
| **123** | LXXXIII XIX | 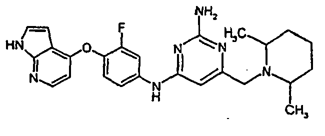 | LC-MS (Methode 15): Rₜ = 1.31 min. MS (ESIpos): m/z = 462 (M+H)⁺ |
| **124** | LXXXVI XIX | 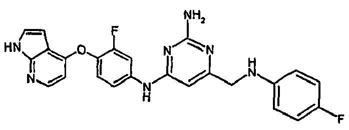 | LC-MS (Methode 13): Rₜ = 1.76 min. MS (ESIpos): m/z = 460 (M+H)⁺ |
| **125** | LXXXVII XIX | 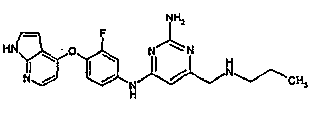 | LC-MS (Methode 13): Rₜ = 1.59 min. MS (ESIpos): m/z = 408 (M+H)⁺ |
| **126** | LXXX XII | 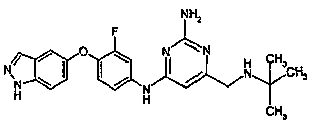 | LC-MS (Methode 15): Rₜ = 1.41 min. MS (ESIpos): m/z = 42 (M+H)⁺ |

### Beispiel 127

### (2-Amino-6-{[3-fluor-4-(1H-pyrrolo[2,3-b]pyridin-4-yloxy)phenyl]amino}pyrimidin-4-yl)methanol

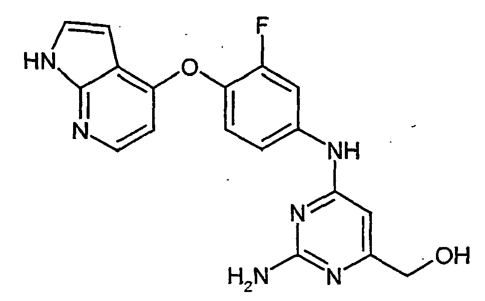

Analog zu Beispiel 116 wird die Titelverbindung durch Reaktion von 106 mg (0.44 mmol) [3-Fluor-4-(1H-pyrrolo[2,3-b]pyridin-4-yloxy)phenyl]amin (aus Beispiel XIX) mit 70 mg (0.44 mmol) (2-Amino-6-chlorpyrimidin-4-yl)methanol (aus Beispiel LXXVIII) gewonnen.
Ausbeute: 49 mg (30% d. Th.)
LC-MS (Methode 14): Rₜ = 1.30 min.
MS (ESIpos): m/z = 367 (M+H)⁺
¹H-NMR (200 MHz, DMSO-d₆): δ = 4.22 (d, 2H), 5.24 (t, 1H), 6.25 (m, 4H), 6.37 (d, 1H), 7.28 (t, 1H), 7.39 (m, 2H), 8.05 (d, 1H), 8.22 (dd, 1H), 9.42 (s, 1H), 11.71 (s, 1H).

### Beispiel 128

### (2-Amino-6-{[3-fluor-4-(1H-pyrrolo[2,3-b]pyridin-4-yloxy)phenyl]amino}pyrimidin-4-yl)essigsäureethylester

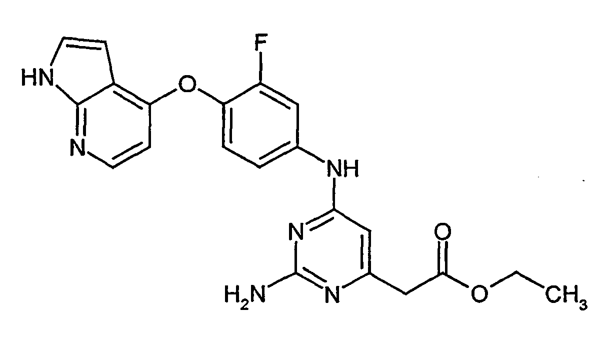

Die Titelverbindung wird analog zu Beispiel 25 aus 220 mg (0.908 mmol) 3-Fluor-4-(1H-pyrrolo[2,3-b]pyridin-4-yloxy)anilin (aus Beispiel XIX) und 195 mg (0.908 mmol) Ethyl-(2-amino-6-chlorpyrimidin-4-yl)acetat (aus Beispiel LXIX) synthetisiert.
Ausbeute: 16 mg (4% d. Th.)
LC-MS (Methode 16): Rₜ = 1.44 min.
MS (ESI pos.): m/z = 423 (M+H)⁺
¹H-NMR (DMSO-d₆, 400 MHz): δ = 1.20 (t, 3H), 3.46 (s, 2H), 4.09 (q, 2H), 6.01 (s, 1H), 6.25 (m, 1H), 6.35 (d, 1H), 6.43 (s, 2H), 7.28 (t, 1H), 7.37 (m, 2H), 8.06 (d, 1H), 8.21 (dd, 1H), 9.46 (s, 1H), 11.74 (s, 1H).

### Beispiel 129

### N⁴-[3-Fluor-4-(1H-pyrrolo[2,3-b]pyridin-4-yloxy)phenyl]-6-methylpyrimidin-2,4-diamin

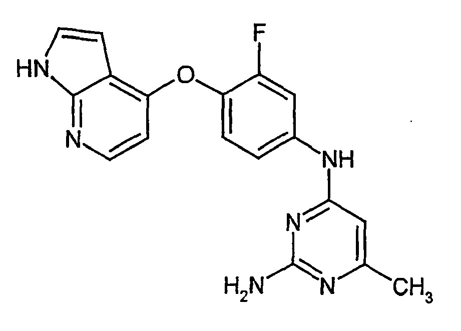

Im Beispiel 128 entsteht die Titelverbindung als Nebenprodukt durch Hydrolyse des Ethylesters und nachfolgende Decarboxylierung.
Ausbeute: 22 mg (7% d. Th.)
LC-MS (Methode 16): Rₜ = 1.35 min.
MS (ESI pos.): m/z = 351 (M+H)⁺
¹H-NMR (DMSO-d₆, 400 MHz): 8 = 2.12 (s, 3H), 5.90 (s, 1H), 6.26 (m, 1H), 6.32 (s, 2H), 6.34 (d, 1H), 7.27 (t, 1H), 7.36 (m, 2H), 8.06 (d, 1H), 8.21 (dd, 1H), 9.34 (s, 1H), 11.74 (s, 1H).

### Beispiel 130

### 4-(2-Amino-6-{[3-fluor-4-(1H-pyrrolo[2,3-b]pyridin-4-yloxy)phenyl]amino}-pyrimidin-4-yl)buttersäure

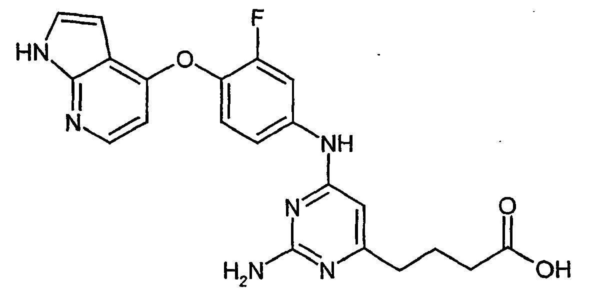

Die Titelverbindung wird analog zu Beispiel 25 aus 327 mg (1.34 mmol) 3-Fluor-4-(1H-pyrrolo[2,3-b]pyridin-4-yloxy)anilin (aus Beispiel XIX) und 230 mg (1.34 mmol) 4-(2-Amino-6-chlorpyrimidin-4-yl)buttersäure (aus Beispiel LXX) synthetisiert und als Hydrochlorid isoliert.
Ausbeute: 414 mg (67% d. Th.)
LC-MS (Methode 15): Rₜ = 1.15 min.
MS (ESI pos.): m/z = 423 (M+H)⁺
¹H-NMR (DMSO-d₆, 200 MHz): δ = 1.87 (tt, 2H), 2.34 (t, 2H), 2.62 (t, 2H), 4.02 (3H), 6.30 (s, 1H), 6.42 (m, 1H), 6.61 (d, 1H), 7.44 - 7.61 (m, 3H), 8.24 (d, 1H), 8.25 - 8.35 (m, 1H), 11.13 (s, 1H), 12.40 (s, 1H), 12.93 (s, 1H).

### Beispiel 131

### N⁴-[3-Fluor-4-(1H-pyrrolo[2,3-b]pyridin-4-yloxy)phenyl]-6-(4-oxo-4-piperidin-1-ylbutyl)pyrimidin-2,4-diamin

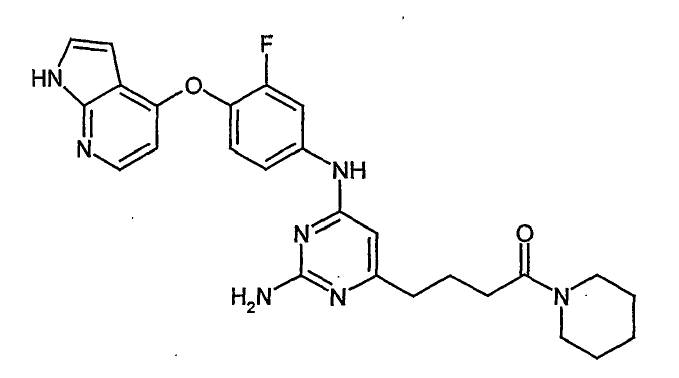

70 mg (0.17 mmol) 4-(2-Amino-6-{[3-fluor-4-(1H-pyrrolo[2,3-b]pyridin-4-yloxy)-phenyl]amino}pyrimidin-4-yl)buttersäure (aus Beispiel 130) werden in 2.0 ml DMF gelöst und mit 60 mg (0.16 mmol) HATU, 23 mg (0.17 mmol) 1-Hydroxy-1H-azobenzotriazolhydrat, 43 mg (0.33 mmol) Diisopropylamin und 28 mg (0.33 mmol) Piperidin versetzt und 20 Stunden bei RT gerührt. Man entfernt flüchtige Bestandteile im Vakuum und reinigt durch präparative HPLC. Das erhaltene Produkt wird mittels Dickschichtchromatographie (1 mm Kieselgel-Beschichtung, Eluens: DCM :
Methanol = 9 : 1 ) weiter aufgereinigt.
Ausbeute: 25 mg (31% d. Th.)
LC-MS (Methode 13): Rₜ = 1.69 min.
MS (ESI pos.): m/z = 490 (M+H)⁺
¹H-NMR (DMSO-d₆, 400 MHz): δ = 1.40 - 1.47 (mc, 4H), 1.56 (mc, 2H), 1.82 (tt, 2H), 2.32 (t, 2H), 2.40 (t, 2H), 3.42 (m, 4H), 5.90 (s, 1H), 6.25 - 6.29 (m, 3H), 6.35 (d, 1H), 7.30 (t, 1H), 7.35 (m, 2H), 8.06 (d, 1H), 8.21 (dd, 1H), 9.33 (s, 1H), 11.74 (s, 1H).

### Beispiel 132

### Ethyl-4-(2-amino-6-{[3-fluor-4-(1H-pyrrolo[2,3-b]pyridin-4-yloxy)phenyl]amino}-pyrimidin-4-yl)butanoat

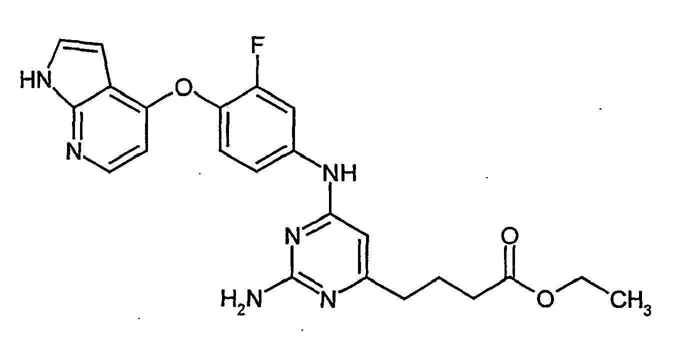

396 mg (0.86 mmol) 4-(2-Amino-6-{[3-fluor-4-(1H-pyrrolo[2,3-b]pyridin-4-yloxy)-phenyl]amino}pyrimidin-4-yl)buttersäure (aus Beispiel 130) werden in 20 ml Ethanol nach Zusatz von 0.10 ml Schwefelsäure 20 Stunden unter RF erhitzt. Man engt im Vakuum ein, versetzt mit Dichlormethan und etwas Methanol und extrahiert mit Natriumhydrogencarbonatlösung. Die organische Phase wird über Natriumsulfat getrocknet und das Lösungsmittel im Vakuum eingeengt. Man erhält die Titelverbindung.
Ausbeute: 379 mg (95% d. Th.)
LC-MS (Methode 13): Rₜ = 1.55 min.
MS (ESI pos.): m/z = 451 (M+H)⁺
¹H-NMR (DMSO-d₆, 400 MHz): δ = 1.19 (t, 3H), 1.87 (quint., 2H), 2.04 (t, 2H), 2.59 (t, 2H), 4.07 (q, 2H), 6.13 (br. s, 1H), 6.25 (dd, 1H), 6.38 (d, 1H), 7.31 - 7.44 (m, 3H), 8.09 (d, 1H), 8.22 (br. s, 1H), 10.51 (br. s, 1H), 11.79 (s, 1H), 12.33 (br. s, 1H).

### Beispiel 133

### 4-(2-Amino-6-{[3-fluor-4-(1H-pyrrolo[2,3-b]pyridin-4-yloxy)phenyl]amino}-pyrimidin-4-yl)butan-1-ol

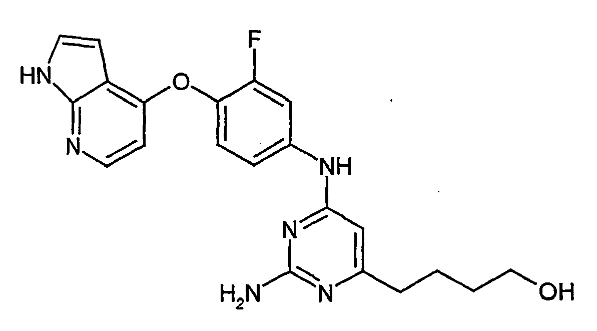

300 mg (0.67 mmol) 4-(2-Amino-6-{[3-fluor-4-(1H-pyrrolo[2,3-b]pyridin-4-yloxy)-phenyl]amino}pyrimidin-4-yl)butan-1-ol (aus Beispiel 132) werden in 15 ml Ethanol suspendiert und bei RT portionsweise mit 253 mg (6.66 mmol) Natriumborhydrid versetzt. Man lässt 24 Stunden bei RT rühren, engt ein und versetzt mit Essigsäureethylester und Wasser. Die organische Phase wird über Natriumsulfat getrocknet. Man reinigt durch Säulenchromatographie (Kieselgel 60; Gradientensäule, Eluens: DCM : Methanol = 9 : 1 bis 3 : 1). Man erhält 133 mg (27%) des Eduktes zurück. Die Produktfraktion wird durch präparative HPLC nachgereinigt. Man erhält die Titelverbindung.
Ausbeute: 43 mg (16% d. Th.)
LC-MS (Methode 13): Rₜ = 1.37 min.
MS (ESI neg.): m/z = 407 (M-H)⁻
¹H-NMR (DMSO-d₆, 400 MHz): δ = 1.45 (tt, 2H), 1.62 (tt, 2H), 2.39 (t, 2H), 3.41 (dt, 2H), 4.37 (t, 1H), 5.90 (s, 1H), 6.24 - 6.26 (m, 1H), 6.27 (br. s, 2H), 6.34 (d, 1H), 7.27 (dd, 1H), 7.34 - 7.37 (m, 2H), 8.06 (d, 1H), 8.21 (dd, 1H), 9.31 (s, 1H), 11.74 (s, 1H).

### Beispiel 134

### N⁴-[3-Fluor-4-(1H-pyrrolo[2,3-b]pyridin-4-yloxy)phenyl]-6-piperidin-3-ylpyrimidin-2,4-diamin

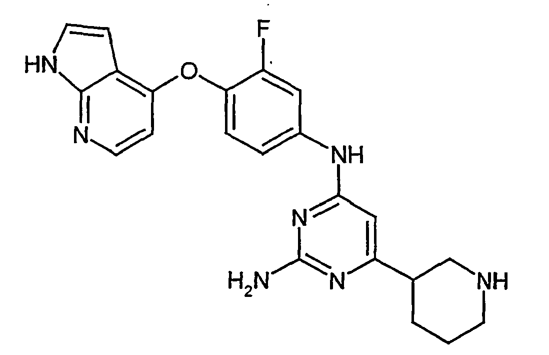

1.36 g (2.46 mmol) Benzyl-3-(2-amino-6-{[3-fluor-4-(1H-pyrrolo[2,3-b]pyridin-4-yloxy)phenyl]amino}pyrimidin-4-yl)piperidin-1-carboxylat (aus Beispiel LXXIV) und 0.4 g 10%iges Palladium auf Kohle werden in Ethanol unter Wasserstoffatmosphäre 24 Stunden bei Raumtemperatur gerührt. Die Suspension wird über Celite® abgesaugt, und das Filtrat wird eingeengt.
Ausbeute: 0.66 g (60% d. Th.)
LC-MS (Methode 13): Rₜ = 1.37 min.
MS (ESI neg.): m/z = 407 (M-H)⁻
¹H-NMR (DMSO-d₆, 300 MHz): δ = 1.46 - 1.70 (m, 4H), 1.89 (m, 1H), 2.57 (m, 1H), 2.67 (t, 1H), 2.97 (m, 1H), 3.10 (m, 1H), 4.07 (br. s, 1H), 5.90 (s, 1H), 6.25 (m, 1H), 6.30 (s, 2H), 6.35 (d, 1H), 7.26 (t, 1H), 7.37 (m, 2H), 8.06 (d, 1H), 8.18 (dd, 1H), 9.35 (s, 1H), 11.71 (s, 1H).

Analog zu Beispiel 134 werden hergestellt:

| **Bsp.-Nr.** | **Edukte (Bsp.-Nr.)** | **Struktur** | **MS, HPLC, LC-MS,** ^{**1**}**H-NMR** |
|---|---|---|---|
| **135** | XCI | 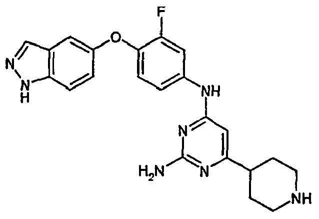 | LC-MS (Methode 12): Rₜ = 2.30 min. |
| | | | MS (ESIpos): m/z = 420 (M+H)⁺ |
| **136** | LXXV | 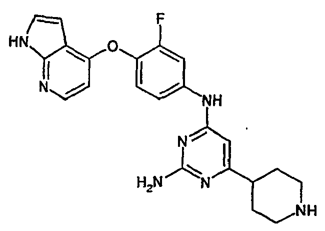 | LC-MS (Methode 16): Rₜ = 1.19 min. |
| | | | MS (ESIpos): m/z = 420 (M+H)⁺ |
| **137** | LXXVI | 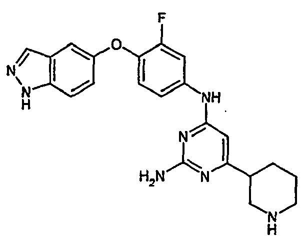 | HPLC (Methode 7): Rₜ = 3.66 min. |
| | | | MS (ESIpos): m/z = 420 (M+H)⁺ |
| **138** | LXXVII | 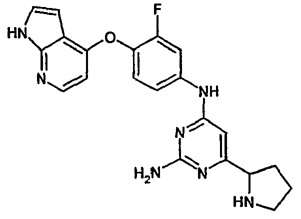 | HPLC (Methode 7): Rₜ = 3.35 min. |
| | | | MS (ESIpos): m/z = 406 (M+H)⁺ |

### Beispiel 139

### 6-(1-Acetylpiperidin-3-yl)-N⁴-[3-fluor-4-(1H-pyrrolo[2,3-b]pyridin-4-yloxy)phenyl]-pyrimidin-2,4-diamin

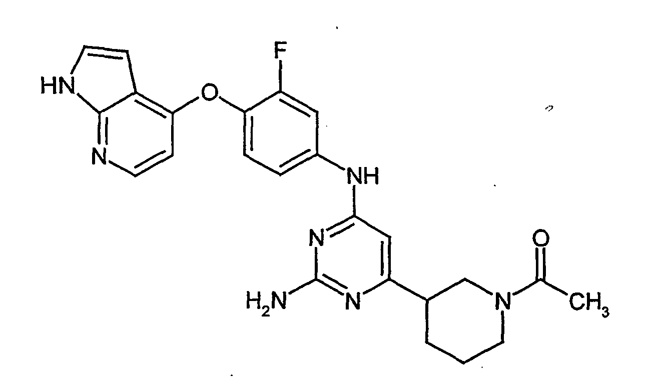

50 mg (0.119 mmol) N⁴-[3-Fluor-4-(1H-pyrrolo[2,3-b]pyridin-4-yloxy)phenyl]-6-piperidin-3-ylpyrimidin-2,4-diamin (aus Beispiel 134) werden in Ethanol suspendiert. Dazu werden 0.013 ml (0.146 mmol) Essigsäureanhydrid gegeben und das Gemisch wird bei Raumtemperatur 18 Stunden gerührt. Dann wird es eingeengt. Der Rückstand wird mittels präparativer HPLC gereinigt.
Ausbeute: 24 mg (40% d. Th.)
HPLC (Methode 7): Rₜ = 3.58 min.
MS (ESI pos.): m/z = 462 (M+H)⁺
¹H-NMR (DMSO-d₆, 300 MHz): δ = 1.23 - 1.78 (m, 4H), 1.94 (m, 1H), 2.02 (s, 3H), 2.32 (m, 1H), 2.57 (m, 1H), 2.99 (t, 1H), 3.84 (t, 1H), 4.31 (dd, 0.5H), 4.55 (dd, 0.5H), 5.92 (s, 0.5H), 5.95 (s, 0.5H), 6.25 (m, 1H), 6.30 (s, 2H), 6.35 (d, 1H), 7.26 (t, 1H), 7.37 (m, 2H), 8.06 (d, 1H), 8.19 (dd, 1H), 9.36 (d, 1H), 11.71 (s, 1H).

### Beispiel 140

### N⁴-[3-Fluor-4-(1H-pyrrolo[2,3-b]pyridin-4-yloxy)phenyl]-6-(1-isopropylpiperidin-3-yl)pyrimidin-2,4-diamin

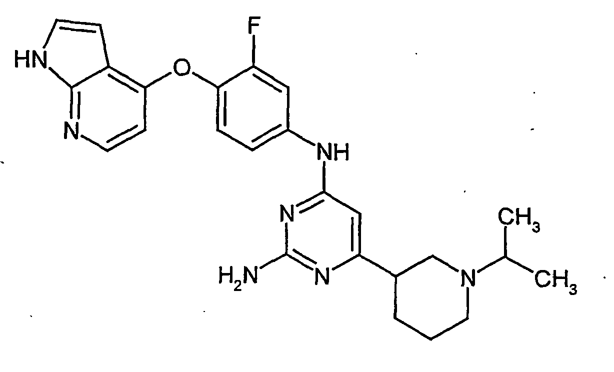

50 mg (0.119 mmol) N⁴-[3-Fluor-4-(1H-pyrrolo[2,3-b]pyridin-4-yloxy)phenyl]-6-piperidin-3-ylpyrimidin-2,4-diamin (aus Beispiel 134) werden in DMF gelöst. Dazu werden 0.013 ml (0.143 mmol) 2-Brompropan und 49 mg (0.358 mmol) Kaliumcarbonat gegeben und das Gemisch wird bei Raumtemperatur 18 Stunden gerührt. Die Suspension wird abgesaugt und das Filtrat wird mittels präparativer HPLC gereinigt.
Ausbeute: 15 mg (27% d. Th.)
LC-MS (Methode 13): Rₜ = 1.28 min.
MS (ESI neg.): m/z = 460.5 (M-H)⁻

### Beispiel 141

### N⁴-[3-Fluor-4-(1H-indazol-5-yloxy)phenyl]pyrimidin-2,4-diamin

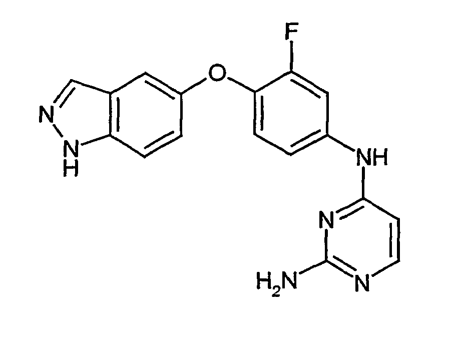

50 mg (0.135 mmol) 6-Chlor-N⁴-[3-fluor-4-(1H-indazol-5-yloxy)phenyl]pyrimidin-2,4-diamin (aus Beispiel 8) werden in Ethanol gelöst. Es werden 50 mg 10%iges Palladium auf Kohle dazugegeben und die Suspension wird über Nacht unter Wasserstoffatmosphäre gerührt. Der Palladiumkatalysator wird abgesaugt und das Filtrat eingeengt.
Ausbeute: 40 mg (70% d. Th.)
LC-MS (Methode 9): Rₜ = 1.81 min.
MS (ESI pos.): m/z = 337.2 (M+H)⁺

### Beispiel 142

### N⁴-[3-Fluor-4-(1H-pyrrolo[2,3-b]pyridin-4-yloxy)phenyl]pyrimidin-2,4-diamin

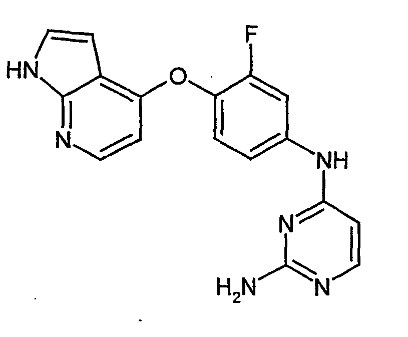

Analog zu Beispiel 141 wird die Titelverbindung durch Reduktion von 53 mg (0.14 mmol) 6-Chlor-N⁴-[3-fluor-4-(1H-pyrrolo[2,3-b]pyridin-4-yloxy)phenyl]-pyrimidin-2,4-diamin (aus Beispiel 60) gewonnen.
Ausbeute: 16 mg (32% d. Th.)
LC-MS (Methode 16): Rₜ = 1.30 min.
MS (ESI neg.): m/z = 335 (M-H)⁻
¹H-NMR (DMSO-d₆, 300 MHz): δ = 6.02 (d, 1H), 6.25 (dd, 1H), 6.32 (br. s, 2H), 6.35 (d, 1H), 7.27 (t, 1H), 7.34 - 7.40 (m, 2H), 7.86 (d, 1H), 8.06 (d, 1H), 8.21 (dd, 1H), 9.39 (s, 1H), 11.72 (s, 1H).

### B. Bewertung der physiologischen Wirksamkeit

In einem in vitro-Assay mit rekombinanter Rho-Kinase-II wird die Hemmung des Enzyms untersucht. Die gefäßrelaxierende Wirkung wird an Phenylephrin-induzierten Kontraktionen isolierter Ringe der Kaninchen-Arteria-Saphena bestimmt. Die Eignung der erfindungsgemäßen Verbindungen zur Behandlung von kardiovaskulären Erkrankungen kann durch Untersuchung der blutdrucksenkenden Wirkung an narkotisierten Ratten gezeigt werden.

### Hemmung der rekombinanten Rho-Kinase II (ROKα)

Die Aktivität der Rho-Kinase wird durch den Einbau von ³³P-Phosphat in ein Substratpeptid bestimmt. Dazu wird kommerziell erhältliche Rho-Kinase II (Upstate Biotechnology) in Gegenwart des S6 Phosphate-Acceptor-Peptides mit den Testsubstanzen oder einer Lösungsmittelkontrolle 10 min bei 37°C vorinkubiert. Anschließend wird die Kinase-Reaktion durch Zugabe von ³³P-markiertem ATP gestartet. Nach 20 min bei 37°C wird die Reaktion durch Zugabe von H₃PO₄ gestoppt. Aliquots werden auf Filter pipettiert, die Filter gewaschen und anschließend mit Scintillator beschichtet. Die Radioaktivität der am Filter gebundenen ³³P-markierten Peptide wird in einem Micro-Beta-Counter gemessen. Der IC₅₀-Wert entspricht der Konzentration einer Testsubstanz bei welcher der Rho-Kinase katalysierte Einbau von ³³P in das Peptid im Vergleich zu einer Lösungsmittelkontrolle um 50 % gehemmt ist. Die experimentellen Daten sind in der folgenden Tabelle zusammengestellt.

| **Beispiel-Nr.** | **IC**_{**50**} **(nM)** |
|---|---|
| 1 | 680 |
| 5 | 20 |
| 6 | 30 |
| 7 | 100 |
| 11 | 6 |
| 12 | 55 |
| 13 | 9 |
| 15 | 20 |
| 16 | 50 |
| 18 | 43 |
| 19 | 2 |
| 68 | 1 |
| 134 | 2 |

### Gefäßrelaxierende Wirkung in vitro

3 mm breite Ringe der isolierten Kaninchen-Arteria-Saphena werden einzeln in 5 ml-Organbäder mit 37°C warmer, carbogenbegaster Krebs-Henseleit-Lösung gebracht. Der Gefäßtonus wird isometrisch erfasst und registriert. Kontraktionen werden durch Zugabe von 3x10⁻⁸ g/ml Phenylephrin induziert und nach 4 min wieder ausgewaschen. Nach mehreren Kontrollzyklen wird die zu untersuchende Substanz mit jedem weiteren Durchgang in steigender Dosierung vorinkubiert und die darauffolgende Kontraktion mit der Höhe der letzten Kontrollkontraktion verglichen. Es wird die Konzentration errechnet, die erforderlich ist, um die Höhe des Kontrollwertes um 50 % zu reduzieren (IC₅₀). Die experimentellen Daten sind in der folgenden Tabelle zusammengestellt.

| **Beispiel-Nr.** | **IC**_{**50**} **(nM)** |
|---|---|
| 1 | 6800 |
| 5 | 1020 |
| 6 | 4330 |
| 7 | 6700 |
| 11 | 350 |
| 12 | 2700 |
| 13 | 2000 |
| 16 | 6900 |
| 19 | 350 |
| 68 | 260 |
| 116 | 150 |
| 117 | 80 |

### Blutdruckmessung an narkotisierten Ratten

Männliche Wistar-Ratten mit einem Körpergewicht von 300 - 350 g werden mit Thiopental (100 mg/kg i.p.) anästhesiert. Nach der Tracheotomie wird in die Femoralarterie ein Katheter zur Blutdruckmessung eingeführt. Die zu prüfenden Substanzen werden als Lösungen entweder oral mittels Schlundsonde oder über die Femoralvene intravenös verabreicht.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der Verbindung von Beispiel 1, 1000 mg Ethanol (96 %), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäßer Verbindung wird der Suspension zugefügt. Unter Rühren erfolgt die Zugabe des Wassers. Bis zum Abschluss der Quellung des Rhodigels wird ca. 6h gerührt.

## Patentansprüche

1. Verbindungen der Formel 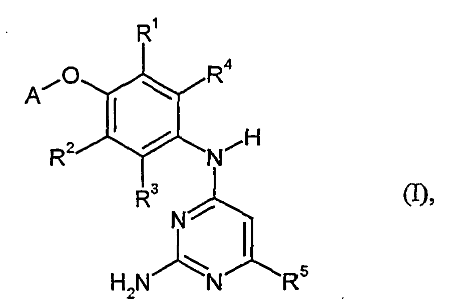 worin
A einen Rest 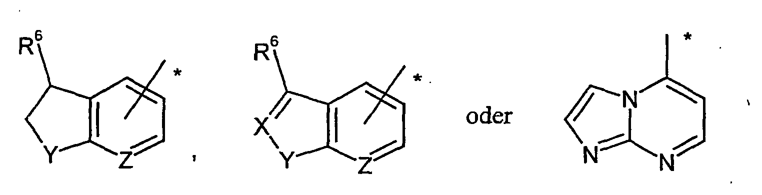 bedeutet,
worin
X für N oder C-H steht,
Y für N-R⁷, O oder S steht,
worin
R⁷ für Wasserstoff, Benzyl, Phenyl, (C₁-C₆)-Alkyl oder (C₃-C₈)-Cycloalkyl steht,
wobei Alkyl und Cycloalkyl ihrerseits durch Fluor, Hydroxy, Amino, Carboxyl, (C₁-C₆)-Alkoxy, (C₁-C₆)-Alkylamino oder Morpholinyl substituiert sein können,
Z für N oder C-H steht,
R⁶ für Wasserstoff, Halogen, Trifluormethyl, (C₁-C₆)-Alkylamino oder W-R⁷ steht,
worin
W für NH, O oder eine Bindung steht,
R⁷ die oben angegebene Bedeutung hat
und
* für die Anknüpfstelle an den phenolischen Sauerstoff steht,
R¹ und R² unabhängig voneinander Wasserstoff, Halogen oder Cyano bedeuten,
R³ und R⁴ unabhängig voneinander Wasserstoff, Fluor oder Chlor bedeuten,
R⁵ einen Rest bedeutet, der ausgewählt ist aus der Gruppe von:
Wasserstoff, Hydroxy, Halogen, Trifluormethyl,
(C₃-C₈)-Cycloalkyl, (C₁-C₆)-Alkyl, (C₁-C₆)-Alkoxy,
wobei Cycloalkyl, Alkyl und Alkoxy ihrerseits durch Hydroxy, Carboxyl, (C₁-C₆)-Alkoxy, (C₁-C₆)-Alkoxycarbonyl, (C₆-C₁₀)-Aryl, NR⁸R⁹ oder C(=O)NR⁸R⁹ substituiert sein können,
worin
R⁸ und R⁹ unabhängig voneinander für Wasserstoff, (C₁-C₈)-Alkyl, gegebenenfalls durch (C₁-C₆)-Alkyl substituiertes (C₃-C₆)-Cycloalkyl, gegebenenfalls durch Halogen substituiertes (C₆-C₁₀)-Aryl oder 5- bis 10-gliedriges Heteroaryl stehen
oder
R⁸ und R⁹ gemeinsam mit dem Stickstoffatom, an das sie gebunden sind, einen 5- oder 6-gliedrigen Heterocyclus bilden, der ein weiteres Hetero-atom O oder N im Ring enthalten kann und der durch (C₁-C₆)-Alkyl, (C₁-C₆)-Alkanoyl oder (C₁-C₆)-Alkoxycarbonyl substituiert sein kann,
(C₆-C₁₀)-Aryl, (C₆-C₁₀)-Aryloxy, 5- bis 10-gliedriges Heteroaryl, 5-bis 10-gliedriges Heteroaryloxy, über ein Kohlenstoffatom gebundenes 5- bis 10-gliedriges Heterocyclyl,
wobei Aryl, Aryloxy, Heteroaryl, Heteroaryloxy und Heterocyclyl ihrerseits durch Halogen, Cyano, Nitro, Carboxyl, Amino, Trifluormethyl, gegebenenfalls durch Hydroxy substituiertes (C₁-C₆)-Alkyl, (C₁-C₆)-Alkoxy, (C₁-C₆)-Alkylamino, (C₁-C₆)-Alkanoyl, (C₁-C₆)-Alkoxycarbonyl, (C₁-C₆)-Alkanoyl-amino, (C₁-C₆)-Alkoxycarbonylamino oder 5- oder 6-gliedriges Heterocyclyl substituiert sein können,
NR¹⁰R¹¹,
worin
R¹⁰ und R¹¹ unabhängig voneinander für Wasserstoff, (C₁-C₆)-Alkyl, (C₃-C₈)-Cycloalkyl, (C₆-C₁₀)-Aryl oder 5- bis 10-gliedriges Heteroaryl stehen,
wobei Alkyl und Cycloalkyl ihrerseits durch Hydroxy, (C₁-C₆)-Alkoxy, (C₆-C₁₀)-Aryl, 5- bis 10-gliedriges Heteroaryl oder NR¹⁵R¹⁶ substituiert sein können,
worin
R¹⁵ und R¹⁶ unabhängig voneinander für Wasserstoff, (C₁-C₆)-Alkyl, (C₃-C₆)-Cycloalkyl, (C₆-C₁₀)-Aryl oder 5- oder 6-gliedriges Heteroaryl stehen
oder
R¹⁵ und R¹⁶ gemeinsam mit dem Stickstoffatom, an das sie gebunden sind, einen 5- oder 6-gliedrigen Heterocyclus bilden, der ein weiteres Hetero-atom O oder N im Ring enthalten kann und der durch (C₁-C₆)-Alkyl, (C₁-C₆)-Alkanoyl oder (C₁-C₆)-Alkoxycarbonyl substituiert sein kann,
und
Aryl und Heteroaryl ihrerseits durch Halogen, Hydroxy, Amino, Cyano, Trifluormethyl, (C₁-C₆)-Alkyl, (C₁-C₆)-Alkoxy, (C₁-C₆)-Alkylamino oder (C₁-C₆)-Alkanoyl-amino substituiert sein können,
oder
R¹⁰ und R¹¹ gemeinsam mit dem Stickstoffatom, an das sie gebunden sind, einen 4- bis 6-gliedrigen Heterocyclus bilden, der ein weiteres Heteroatom O oder N im Ring enthalten kann und der durch Fluor, Hydroxy, Carboxyl, 5- bis 7-gliedriges Heterocyclyl, das ein oder zwei weitere Heteroatome N und/oder O im Ring enthalten kann und das seinerseits durch (C₁-C₄)-Alkyl oder (C₁-C₄)-Alkoxycarbonyl substituiert sein kann, (C₁-C₄)-Alkoxy, gegebenenfalls durch Hydroxy, (C₁₋C₄)-Alkoxy oder NR¹⁷R¹⁸ substituiertes (C₁-C₄)-Alkyl, (C₁-C₄)-Alkanoyl, (C₁-C₄)-Alkoxycarbonyl oder NR¹²R¹³ substituiert sein kann,
wobei
R¹² und R¹³ unabhängig voneinander für Wasserstoff, (C₁-C₆)-Alkyl, (C₁-C₄)-Alkoxycarbonyl, (C₃-C₈)-Cycloalkyl oder (C₁-C₄)-Alkanoyl stehen
oder
R¹² und R¹³ gemeinsam mit dem Stickstoffatom, an das sie gebunden sind, einen 5- oder 6-gliedrigen Heterocyclus bilden, der ein weiteres Hetero-atom O oder N im Ring enthalten kann und der durch (C₁-C₆)-Alkyl, (C₁-C₆)-Alkanoyl oder (C₁-C₆)-Alkoxycarbonyl substituiert sein kann,
und
R¹⁷ und R¹⁸ unabhängig voneinander für Wasserstoff, gegebenenfalls durch Hydroxy substituiertes (C₁-C₆)-Alkyl, (C₃-C₆)-Cycloalkyl, (C₆-C₁₀)-Aryl oder 5- oder 6-gliedriges Heteroaryl stehen
oder
R¹⁷ und R¹⁸ gemeinsam mit dem Stickstoffatom, an das sie gebunden sind, einen 5- oder 6-gliedrigen Heterocyclus bilden, der ein weiteres Hetero-atom O oder N im Ring enthalten kann und der durch (C₁-C₆)-Alkyl, (C₁-C₆)-Alkanoyl oder (C₁-C₆)-Alkoxycarbonyl substituiert sein kann,
oder
R¹⁰ und R¹¹ gemeinsam mit dem Stickstoffatom, an das sie gebunden sind, einen 7- bis 12-gliedrigen bicyclischen oder tricyclischen Heterocyclus bilden, der anneliert oder spirocyclisch ist und ein oder zwei weitere Hetero-atome aus der Reihe N und/oder O im Ring aufweisen kann und der durch Fluor, (C₁-C₄)-Alkyl, (C₁-C₄)-Alkoxycarbonyl, (C₁-C₄)-Alkanoyl oder Benzyl substituiert sein kann,
und C(=O)R¹⁴,
worin
R¹⁴ für (C₁-C₆)-Alkoxy, (C₁-C₆)-Alkylamino oder einen über ein Stickstoffatom gebundenen 5- bis 10-gliedrigen mono- oder bicyclischen Heterocyclus, der anneliert oder spirocyclisch ist und ein oder zwei weitere Heteroatome aus der Reihe N und/oder O im Ring aufweisen kann, steht,
wobei Alkylamino seinerseits durch einen 5- oder 6-gliedrigen Heterocyclyl substituiert sein kann,
und ihre Salze, Hydrate, Hydrate der Salze und Solvate.

2. Verbindungen nach Anspruch 1,
worin
A einen Rest 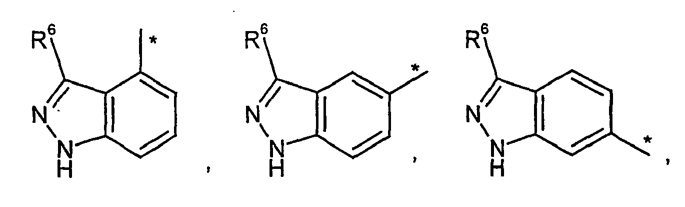 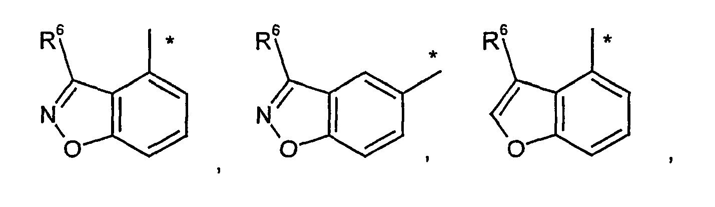 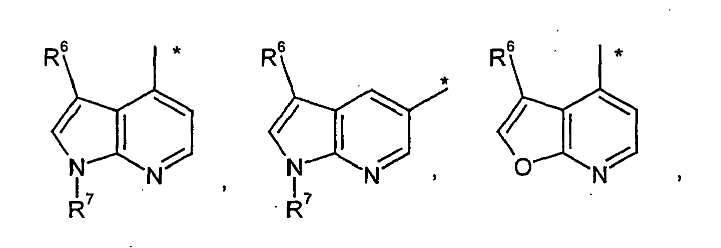 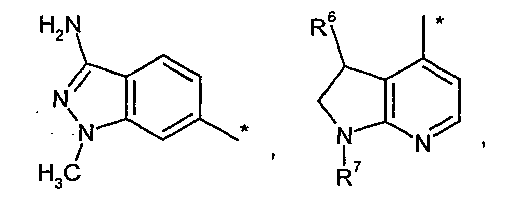 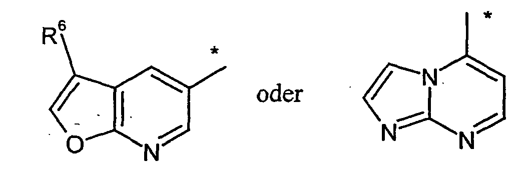 bedeutet,
worin
R⁶ für Wasserstoff, (C₁-C₄)-Alkyl oder NH-R⁷ steht,
R⁷ für Wasserstoff oder (C₁-C₄)-Alkyl steht
und
* für die Anknüpfstelle an den phenolischen Sauerstoff steht,
R¹ und R² unabhängig voneinander Wasserstoff, Fluor oder Chlor bedeuten,
R³ und R⁴ unabhängig voneinander Wasserstoff oder Fluor bedeuten,
R⁵ einen Rest bedeutet, der ausgewählt ist aus der Gruppe von:
Wasserstoff, Chlor, (C₃-C₈)-Cycloalkyl, (C₁-C₆)-Alkyl, (C₁-C₆)-Alkoxy,
wobei Alkyl und Alkoxy ihrerseits durch Hydroxy, Carboxyl, (C₁-C₄)-Alkoxy, (C₁-C₄)-Alkoxycarbonyl, NR⁸R⁹ oder C(=O)NR⁸R⁹ substituiert sein können,
worin
R⁸ und R⁹ unabhängig voneinander für Wasserstoff, (C₁-C₈)-Alkyl, gegebenenfalls durch (C₁-C₄)-Alkyl substituiertes (C₃-C₆)-Cycloalkyl, gegebenenfalls durch Halogen substituiertes Phenyl oder 5- oder 6-gliedriges Heteroaryl stehen
oder
R⁸ und R⁹ gemeinsam mit dem Stickstoffatom, an das sie gebunden sind, einen Morpholin-, Piperazin-, Piperidin- oder Pyrrolidinring bilden, wobei die Ringe ihrerseits durch (C₁-C₄)-Alkyl substituiert sein können,
(C₆-C₁₀)-Aryl, 5- oder 6-gliedriges Heteroaryl, über ein Kohlenstoffatom gebundenes 5- oder 6-gliedriges Heterocyclyl,
wobei Aryl, Heteroaryl und Heterocyclyl ihrerseits durch Halogen, Cyano, Nitro, Carboxyl, Amino, Trifluormethyl, gegebenenfalls durch Hydroxy substituiertes (C₁-C₄)-Alkyl, (C₁-C₄)-Alkoxy, (C₁-C₄)-Alkylamino, (C₁-C₄)-Alkanoyl, (C₁C₄)-Alkoxycarbonyl, (C₁-C₄)-Alkanoylamino, (C₁-C₄)-Alkoxycarbonylamino oder 6-gliedriges Heterocyclyl substituiert sein können,
NR¹⁰R¹¹,
worin
R¹⁰ und R¹¹ unabhängig voneinander für Wasserstoff, (C₁-C₆)Alkyl, (C₃-C₈)-Cycloalkyl, Phenyl oder 5- oder 6gliedriges Heteroaryl stehen,
wobei Alkyl und Cycloalkyl ihrerseits durch Hydroxy, (C₁-C₄)-Alkoxy, Phenyl, 5- oder 6-gliedriges Heteroaryl oder NR¹⁵R¹⁶ substituiert sein können,
worin
R¹⁵ und R¹⁶ unabhängig voneinander für Wasserstoff, (C₁-C₄)-Alkyl, (C₃-C₆)-Cycloalkyl, Phenyl oder 5- oder 6gliedriges Heteroaryl stehen
oder
R¹⁵ und R¹⁶ gemeinsam mit dem Stickstoffatom, an das sie gebunden sind, einen Morpholin-, Piperazin-, Piperidin- oder Pyrrolidinring bilden, wobei die Ringe ihrerseits durch (C₁-C₄)-Alkyl substituiert sein können,
und
Phenyl und Heteroaryl ihrerseits durch Fluor, Chlor, Hydroxy, Amino, Cyano, Trifluormethyl, (C₁-C₄)-Alkyl, (C₁-C₄)-Alkoxy, (C₁-C₄)-Alkylamino oder (C₁₋C₄)-Alkanoylamino substituiert sein können,
oder
R¹⁰ und R¹¹ gemeinsam mit dem Stickstoffatom, an das sie gebunden sind, einen 4- bis 6-gliedrigen Heterocyclus bilden, der ein weiteres Heteroatom O oder N im Ring enthalten kann und der durch Fluor, Hydroxy, Carboxyl, 5- bis 7-gliedriges Heterocyclyl, das ein oder zwei weitere Heteroatome N und/oder O im Ring enthalten kann, das seinerseits durch (C₁-C₄)-Alkyl oder (C₁-C₄)-Alkoxycarbonyl substituiert sein kann, (C₁-C₄)-Alkoxy, gegebenenfalls durch Hydroxy, (C₁₋C₄)-Alkoxy oder NR¹⁷R¹⁸ substituiertes (C₁-C₄)-Alkyl, (C₁-C₄)-Alkanoyl, (C₁-C₄)-Alkoxycarbonyl oder NR¹²R¹³ substituiert sein kann,
wobei
R¹² und R¹³ unabhängig voneinander für Wasserstoff oder (C₁-C₄)-Alkyl stehen
oder
R¹² und R¹³ gemeinsam mit dem Stickstoffatom, an das sie gebunden sind, einen 5- oder 6-gliedrigen Heterocyclus bilden, der ein weiteres Hetero-atom O oder N im Ring enthalten kann und der durch (C₁-C₆)-Alkyl, (C₁-C₆)-Alkanoyl oder (C₁-C₆)-Alkoxycarbonyl substituiert sein kann,
und
R¹⁷ und R¹⁸ unabhängig voneinander für Wasserstoff, gegebenenfalls durch Hydroxy substituiertes (C₁-C₄)-Alkyl oder Phenyl stehen
oder
R¹⁷ und R¹⁸ gemeinsam mit dem Stickstoffatom, an das sie gebunden sind, einen Pyrrolidinring bilden,
oder
R¹⁰ und R¹¹ gemeinsam mit dem Stickstoffatom, an das sie gebunden sind, einen 7- bis 12-gliedrigen bicyclischen oder tricyclischen Heterocyclus bilden, der anneliert oder spirocyclisch ist und ein oder zwei weitere Heteroatome aus der Reihe N und/oder O im Ring aufweisen kann und der durch (C₁-C₄)-Alkyl, (C₁-C₄)-Alkoxycarbonyl, (C₁-C₄)-Alkanoyl oder Benzyl substituiert sein kann,
und C(=O)R¹⁴,
worin
R¹⁴ für (C₁-C₆)-Alkoxy, (C₁-C₆)-Alkylamino oder einen über ein Stickstoffatom gebundenen 5- bis 10-gliedrigen mono- oder bicyclischen Heterocyclus, der anneliert oder spirocyclisch ist und ein oder zwei weitere Heteroatome aus der Reihe N und/oder O im Ring aufweisen kann, steht,
wobei Alkylamino seinerseits durch einem 5- oder 6-gliedrigen Heterocyclyl substituiert sein kann,
und ihre Salze, Hydrate, Hydrate der Salze und Solvate.

3. Verbindungen nach einem der Ansprüche 1 oder 2,
worin
A einen Rest 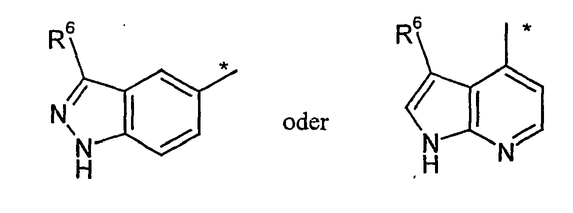 bedeutet,
worin
R⁶ für Wasserstoff oder Methyl steht
und
* für die Anknüpfstelle an den phenolischen Sauerstoff steht,
R¹ und R² unabhängig voneinander Wasserstoff, Fluor oder Chlor bedeuten,
R³ und R⁴ Wasserstoff bedeuten,
R⁵ einen Rest bedeutet, der ausgewählt ist aus der Gruppe von:
Wasserstoff, Chlor, Cyclohexyl, (C₁-C₄)-Alkyl, (C₁-C₄)-Alkoxy,
wobei Alkyl und Alkoxy ihrerseits durch Hydroxy, Carboxyl, (C₁-C₄)-Alkoxy, Methyloxycarbonyl, Ethyloxycarbonyl, NR⁸R⁹, oder C(=O)NR⁸R⁹ substituiert sein können,
worin
R⁸ und R⁹ unabhängig voneinander für Wasserstoff, (C₁-C₈)-Alkyl, Cyclopropyl, gegebenenfalls durch Methyl substituiertes Cyclopentyl oder gegebenenfalls durch Fluor substituiertes Phenyl stehen
oder
R⁸ und R⁹ gemeinsam mit dem Stickstoffatom, an das sie gebunden sind, einen Piperidin-, 2-Methylpiperidin- oder 2,6-Dimethylpiperidinring bilden,
Phenyl, Pyridyl, Pyrrolyl, Piperidin-3-yl, Piperidin-4-yl, Pyrrolidin-2-yl,
wobei Phenyl, Pyridyl und Pyrrolyl ihrerseits durch Fluor, Chlor, Brom, Cyano, Nitro, Trifluormethyl, Methyl, Hydroxymethyl, Methoxy, Dimethylamino oder Morpholinyl substituiert sein können,
und
Piperidin-3-yl, Piperidin-4-yl und Pyrrolidin-2-yl ihrerseits durch Methyl, Ethyl, n-Propyl, iso-Propyl, Methylcarbonyl oder Ethylcarbonyl substituiert sein können,
NR¹⁰R¹¹,
worin
R¹⁰ und R¹¹ unabhängig voneinander für Wasserstoff, (C₁-C₄)-Alkyl, 3-Hydroxypropyl, 2-Hydroxycyclohexyl, 2-Aminocyclohexyl, Phenyl, Pyridyl oder Pyrazolyl stehen,
wobei Phenyl und Pyridyl ihrerseits durch Chlor, Hydroxy, Amino, Cyano, Methyl oder Methoxy substituiert sein können,
oder
R¹⁰ und R¹¹ gemeinsam mit dem Stickstoffatom, an das sie gebunden sind, einen Piperazin-, 3-Methylpiperazin-, 3,5-Dimethylpiperazin-, 4-Isobutylpiperazin-, Morpholin-, Pyrrolidin-, 3-Aminopyrrolidin-, 3-Methylamino-pyrrolidin-, 3-(*N,N*-Dimethylamino)-pyrrolidin-, 2-Aminomethylpyrrolidin-, 3-Hydroaypyrrolidin-, 2-Hydroxymethylpyrrolidin- oder 2-Methoxymethyl-pyrrolidinring oder einen Rest 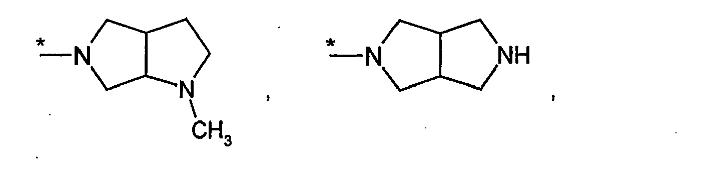 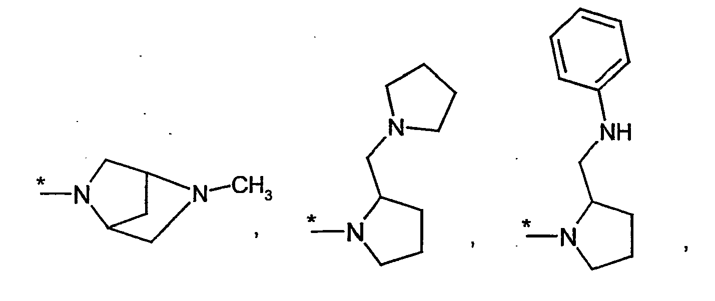 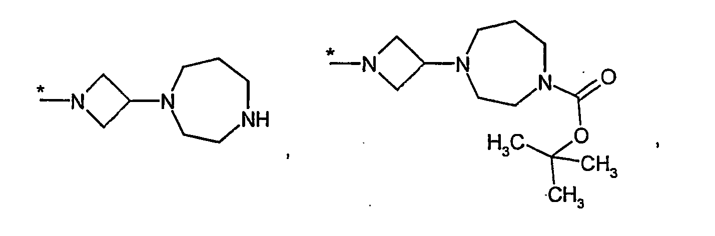 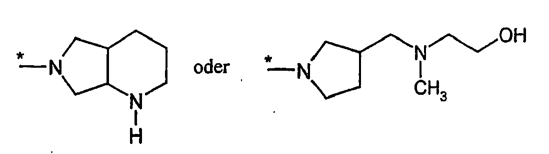 bilden,
worin
* für die Anknüpfstelle an den Pyrimidinring steht,
und C(=O)R¹⁴,
worin
R¹⁴ für Methoxy, Piperidinyl-N-ethylamino, Piperidinyl oder Piperazinyl steht,
und ihre Salze, Hydrate, Hydrate der Salze und Solvate.

4. Verfahren zur Herstellung von Verbindungen, wie in Anspruch 1 definiert, **dadurch gekennzeichnet, dass** man entweder
[A] Verbindungen der Formel (II) 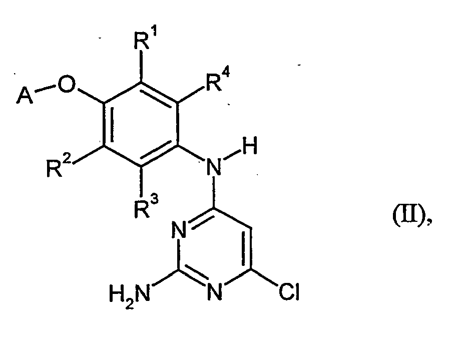 worin
A, R¹, R², R³ und R⁴ die in Anspruch 1 angegebene Bedeutung aufweisen,
mit Verbindungen der Formel (III)
R⁵―X¹ (III),
worin
R⁵ die in Anspruch 1 angegebene Bedeutung aufweist und
X¹ für Wasserstoff, B(OH)₂ oder einen Boronsäureester wie 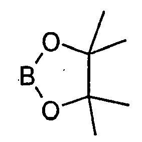 steht,
oder
**[B]** Verbindungen der Formel (IV) 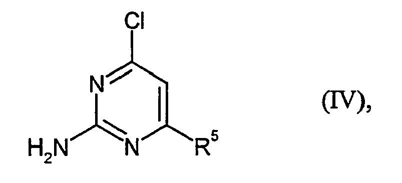 worin
R⁵ die in Anspruch 1 angegebene Bedeutung aufweist,
mit Verbindungen der Formel (V) 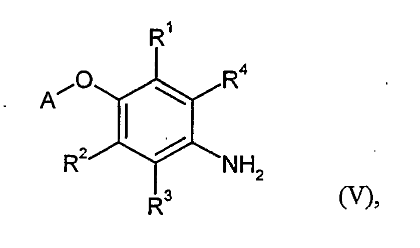 worin
A, R¹, R², R³ und R⁴ die in Anspruch 1 angegebene Bedeutung aufweisen,
umsetzt.

5. Verbindung, wie in einem der Ansprüche 1 bis 3 definiert, zur Behandlung und/oder Prophylaxe von Erkrankungen.

6. Verwendung einer Verbindung, wie in einem der Ansprüche 1 bis 3 definiert, zur Herstellung von Arzneimitteln zur Behandlung und/oder Prophylaxe von kardiovaskulären Erkrankungen.

7. Verwendung einer Verbindung, wie in einem der Ansprüche 1 bis 3 definiert, zur Herstellung von Arzneimitteln zur Behandlung und/oder Prophylaxe von erektiler Dysfunktion.

8. Arzneimittel enthaltend eine Verbindung, wie in einem der Ansprüche 1 bis 3 definiert, und einen weiteren Wirkstoff.

9. Arzneimittel enthaltend eine Verbindung, wie in einem der Ansprüche 1 bis 3 definiert, in Kombination mit einem inerten nichttoxischen, pharmazeutisch geeigneten Hilfsstoff.

## Claims

1. Compounds of the formula 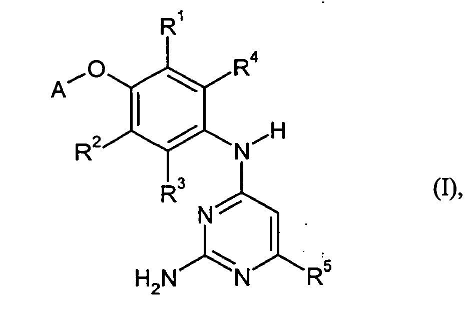 in which
A represents a radical 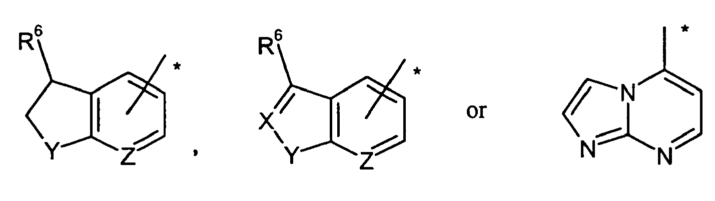 in which
X represents N or C-H,
Y represents N-R⁷, O or S
in which
R⁷ represents hydrogen, benzyl, phenyl, (C₁-C₆)-alkyl or (C₃-C₈)-cycloalkyl,
where alkyl and cycloalkyl for their part may be substituted by fluorine, hydroxyl, amino, carboxyl, (C₁-C₆)-alkoxy, (C₁-C₆)-alkylamino or morpholinyl,
Z represents N or C-H,
R⁶ represents hydrogen, halogen, trifluoromethyl, (C₁-C₆)-alkylamino or W-R⁷,
in which
W represents NH, O or a bond,
R⁷ is as defined above
and
* denotes the point of attachment to the phenolic oxygen,
R¹ and R² independently of one another represent hydrogen, halogen or cyano,
R³ and R⁴ independently of one another represent hydrogen, fluorine or chlorine,
R⁵ represents a radical selected from the group consisting of:
hydrogen, hydroxyl, halogen, trifluoromethyl,
(C₃-C₈)-cycloalkyl, (C₁-C₆)-alkyl, (C₁-C₆)-alkoxy,
where cycloalkyl, alkyl and alkoxy for their part may be substituted by hydroxyl, carboxyl, (C₁-C₆)-alkoxy, (C₁-C₆)-alkoxycarbonyl, (C₆-C₁₀)-aryl, NR⁸R⁹ or C(=O)NR⁸R⁹,
in which
R⁸ and R⁹ independently of one another represent hydrogen, (C₁-C₈)-alkyl, optionally (C₁-C₆)-alkyl-substituted (C₃-C₆)-cycloalkyl, optionally halogen-substituted (C₆-C₁₀)-aryl or 5- to 10-membered heteroaryl
or
R⁸ and R⁹ together with the nitrogen atom to which they are attached form a 5- or 6-membered heterocycle which may contain a further heteroatom O or N in the ring and which may be substituted by (C₁-C₆)-alkyl, (C₁-C₆)-alkanoyl or (C₁-C₆)-alkoxycarbonyl,
(C₆-C₁₀)-aryl, (C₆-C₁₀)-aryloxy, 5- to 10-membered heteroaryl, 5- to 10-membered heteroaryloxy, 5- to 10-membered heterocyclyl which is attached via a carbon atom,
where aryl, aryloxy, heteroaryl, heteroaryloxy and heterocyclyl for their part may be substituted by halogen, cyano, nitro, carboxyl, amino, trifluoromethyl, optionally hydroxyl-substituted (C₁-C₆)-alkyl, (C₁-C₆)-alkoxy, (C₁-C₆)-alkylamino, (C₁-C₆)-alkanoyl, (C₁-C₆)-alkoxycarbonyl, (C₁₋C₆)-alkanoylamino, (C₁-C₆)-alkoxycarbonylamino or 5- or 6-membered heterocyclyl,
NR¹⁰R¹¹
in which
R¹⁰ and R¹¹ independently of one another represent hydrogen, (C₁-C₆)-alkyl, (C₃-C₈)-cycloalkyl, (C₆₋C₁₀)-aryl or 5- to 10-membered heteroaryl,
where alkyl and cycloalkyl for their part may be substituted by hydroxyl, (C₁-C₆)-alkoxy, (C₆-C₁₀)-aryl, 5- to 10-membered heteroaryl or NR¹⁵R¹⁶,
in which
R¹⁵ and R¹⁶ independently of one another represent hydrogen, (C₁-C₆)-alkyl, (C₃-C₆)-cycloalkyl, (C₆-C₁₀)-aryl or 5- or 6-membered heteroaryl
or
R¹⁵ and R¹⁶ together with the nitrogen atom to which they are attached form a 5- or 6-membered heterocycle which may contain a further heteroatom O or N in the ring and which may be substituted by (C₁-C₆)-alkyl, (C₁-C₆)-alkanoyl or (C₁-C₆)-alkoxycarbonyl,
and
aryl and heteroaryl for their part may be substituted by halogen, hydroxyl, amino, cyano, trifluoromethyl, (C₁-C₆)-alkyl, (C₁-C₆)-alkoxy, (C₁-C₆)-alkylamino or (C₁-C₆)-alkanoylamino,
or
R¹⁰ and R¹¹ together with the nitrogen atom to which they are attached form a 4- to 6-membered heterocycle which may contain a further heteroatom O or N in the ring and which may be substituted by fluorine, hydroxyl, carboxyl, 5- to 7-membered heterocyclyl which may contain one or two further heteroatoms N and/or O in the ring and which for its part may be substituted by (C₁-C₄)-alkyl or (C₁-C₄)-alkoxycarbonyl, (C₁-C₄)-alkoxy, optionally hydroxyl-, (C₁-C₄)-alkoxy- or NR¹⁷R¹⁸-substituted (C₁-C₄)-alkyl, (C₁-C₄)-alka
where
R¹² and R¹³ independently of one another represent hydrogen, (C₁-C₆)-alkyl, (C₁-C₄)-alkoxycarbonyl, (C₃-C₈)-cycloalkyl or (C₁₋C₄)-alkanoyl
or
R¹² and R¹³ together with the nitrogen atom to which they are attached form a 5- or 6-membered heterocycle which may contain a further heteroatom O or N in the ring and which may be substituted by (C₁-C₆)-alkyl, (C₁-C₆)-alkanoyl or (C₁-C₆)-alkoxycarbonyl,
and
R¹⁷ and R¹⁸ independently of one another represent hydrogen, optionally hydroxyl-substituted (C₁-C₆)-alkyl, (C₃-C₆)-cycloalkyl, (C₆-C₁₀)-aryl or 5- or 6-membered heteroaryl
or
R¹⁷ and R¹⁸ together with the nitrogen atom to which they are attached form a 5- or 6-membered heterocycle which may contain a further heteroatom O or N in the ring and which may be substituted by (C₁-C₆)-alkyl, (C₁-C₆)-alkanoyl or (C₁-C₆)-alkoxycarbonyl,
or
R¹⁰ and R¹¹ together with the nitrogen atom to which they are attached form a 7- to 12-membered bicyclic or tricyclic heterocycle which is fused or spirocyclic and which may have one or two further heteroatoms from the group consisting of N and O in the ring and which may be substituted by fluorine, (C₁-C₄)-alkyl, (C₁₋C₄)-alkoxycarbonyl, (C₁-C₄)-alkanoyl or benzyl,
and C(=O)R¹⁴,
in which
R¹⁴ represents (C₁-C₆)-alkoxy, (C₁-C₆)-alkylamino or a 5-to 10-membered mono- or bicyclic heterocycle which is attached via a nitrogen atom, which is fused or spirocyclic and which may have one or two further heteroatoms from the group consisting of N and O in the ring,
where alkylamino for its part may be substituted by a 5- or 6-membered heterocycle,
or a salt, a hydrate, a hydrate of a salt or a solvate thereof.

2. Compounds according to Claim 1
in which
A represents a radical 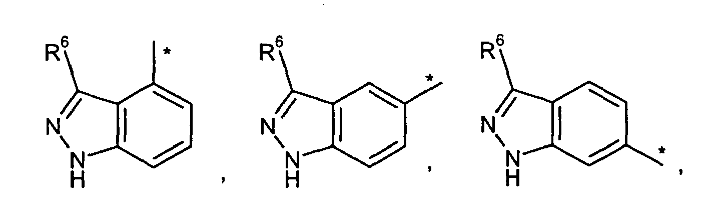 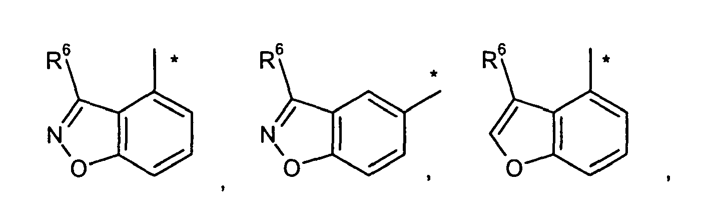 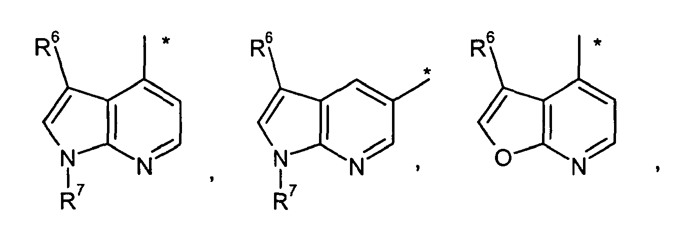 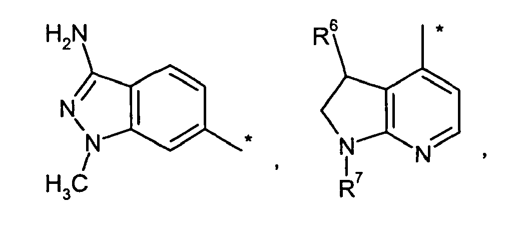 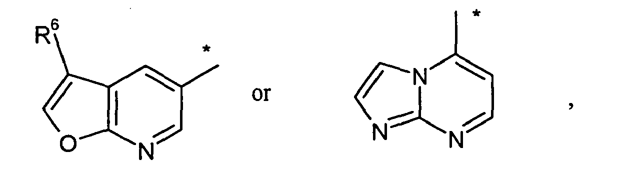 in which
R⁶ represents hydrogen, (C₁-C₄)-alkyl or NH-R⁷,
R⁷ represents hydrogen or (C₁-C₄)-alkyl
and
* denotes the point of attachment to the phenolic oxygen,
R¹ and R² independently of one another represent hydrogen, fluorine or chlorine,
R³ and R⁴ independently of one another represent hydrogen or fluorine,
R⁵ represents a radical selected from the group consisting of:
hydrogen, chlorine, (C₃-C₈)-cycloalkyl, (C₁-C₆)-alkyl, (C₁-C₆)-alkoxy,
where alkyl and alkoxy for their part may be substituted by hydroxyl, carboxyl, (C₁-C₄)-alkoxy, (C₁-C₄)-alkoxycarbonyl, NR⁸R⁹ or C(=O)NR⁸R⁹,
in which
R⁸ and R⁹ independently of one another represent hydrogen, (C₁-C₈)-alkyl, optionally (C₁-C₄)-alkyl-substituted (C₃-C₆)-cycloalkyl, optionally halogen-substituted phenyl or 5- or 6-membered heteroaryl
or
R⁸ and R⁹ together with the nitrogen atom to which they are attached form a morpholine, piperazine, piperidine or pyrrolidine ring, where the rings for their part may be substituted by (C₁-C₄)-alkyl,
(C₆-C₁₀)-aryl, 5- or 6-membered heteroaryl, 5- or 6-membered heterocyclyl which is attached via a carbon atom,
where aryl, heteroaryl and heterocyclyl for their part may be substituted by halogen, cyano, nitro, carboxyl, amino, trifluoromethyl, optionally hydroxyl-substituted (C₁-C₄)-alkyl, (C₁-C₄)-alkoxy, (C₁-C₄)-alkylamino, (C₁-C₄)-alkanoyl, (C₁-C₄)-alkoxycarbonyl, (C₁-C₄)-alkanoylamino, (C₁-C₄)-alkoxycarbonylamino or 6-membered heterocyclyl,
NR¹⁰R¹¹
in which
R¹⁰ and R¹¹ independently of one another represent hydrogen, (C₁-C₆)-alkyl, (C₃-C₈)-cycloalkyl, phenyl or 5- or 6-membered heteroaryl,
where alkyl and cycloalkyl for their part may be substituted by hydroxyl, (C₁-C₄)-alkoxy, phenyl, 5-or 6-membered heteroaryl or NR¹⁵R¹⁶,
in which
R¹⁵ and R¹⁶ independently of one another represent hydrogen, (C₁-C₄)-alkyl, (C₃₋C₆)-cycloalkyl, phenyl or 5- or 6-membered heteroaryl
or
R¹⁵ and R¹⁶ together with the nitrogen atom to which they are attached form a morpholine, piperazine, piperidine or pyrrolidine ring, where the rings for their part may be substituted by (C₁₋C₄)-alkyl,
and
phenyl and heteroaryl for their part may be substituted by fluorine, chlorine, hydroxyl, amino, cyano, trifluoromethyl, (C₁-C₄)-alkyl, (C₁-C₄)-alkoxy, (C₁-C₄)-alkylamino or (C₁-C₄)-alkanoylamino,
or
R¹⁰ and R¹¹ together with the nitrogen atom to which they are attached form a 4- to 6-membered heterocycle which may contain a further heteroatom O or N in the ring and which may be substituted by fluorine, hydroxyl, carboxyl, 5- to 7-membered heterocyclyl which may contain one or two further heteroatoms N and/or O in the ring and which for its part may be substituted by (C₁-C₄)-alkyl or (C₁-C₄)-alkoxycarbonyl, (C₁-C₄)-alkoxy, optionally hydroxyl-, (C₁-C₄)-alkoxy- or NR¹⁷R¹⁸-substituted (C₁-C₄)-alkyl, (C₁-C₄)-alkanoyl, (C₁-C₄)-alkoxycarbonyl or NR¹²R¹³,
where
R¹² and R¹³ independently of one another represent hydrogen or (C₁-C₄)-alkyl
or
R¹² and R¹³ together with the nitrogen atom to which they are attached form a 5- or 6-membered heterocycle which may contain a further heteroatom O or N in the ring and which may be substituted by (C₁-C₆)-alkyl, (C₁-C₆)-alkanoyl or (C₁-C₆)-alkoxycarbonyl,
and
R¹⁷ and R¹⁸ independently of one another represent hydrogen, optionally hydroxyl-substituted (C₁-C₄)-alkyl or phenyl
or
R¹⁷ and R¹⁸ together with the nitrogen atom to which they are attached form a pyrrolidine ring,
or
R¹⁰ and R¹¹ together with the nitrogen atom to which they are attached form a 7- to 12-membered bicyclic or tricyclic heterocycle which is fused or spirocyclic, which may have one or two further heteroatoms from the group consisting of N and O in the ring and which may be substituted by (C₁-C₄)-alkyl, (C₁-C₄)-alkoxycarbonyl, (C₁-C₄)-alkanoyl or benzyl,
and C(=O)R¹⁴
in which
R¹⁴ represents (C₁-C₆)-alkoxy, (C₁-C₆)-alkylamino or a 5-to 10-membered mono- or bicyclic heterocycle which is attached via a nitrogen atom, which is fused or spirocyclic and which may have one or two further heteroatoms from the group consisting of N and O in the ring,
where alkylamino for its part may be substituted by a 5- or 6-membered heterocyclyl,
or a salt, a hydrate, a hydrate of a salt or a solvate thereof.

3. Compounds according to Claim 1 or 2
in which
A represents a radical 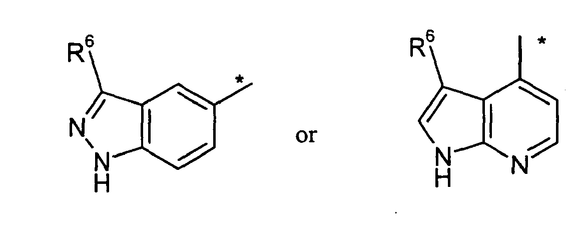 in which
R⁶ represents hydrogen or methyl
and
* denotes the point of attachment to the phenolic oxygen,
R¹ and R² independently of one another represent hydrogen, fluorine or chlorine,
R³ and R⁴ represent hydrogen,
R⁵ represents a radical selected from the group consisting of:
hydrogen, chlorine, cyclohexyl, (C₁-C₄)-alkyl, (C₁-C₄)-alkoxy,
where alkyl and alkoxy for their part may be substituted by hydroxyl, carboxyl, (C₁-C₄)-alkoxy, methyloxycarbonyl, ethyloxycarbonyl, NR⁸R⁹ or C(=O)NR⁸R⁹,
in which
R⁸ and R⁹ independently of one another represent hydrogen, (C₁-C₈)-alkyl, cyclopropyl, optionally methyl-substituted cyclopentyl or optionally fluorine-substituted phenyl
or
R⁸ and R⁹ together with the nitrogen atom to which they are attached form a piperidine, 2-methyl-piperidine or 2,6-dimethylpiperidine ring,
phenyl, pyridyl, pyrrolyl, piperidin-3-yl, piperidin-4-yl, pyrrolidin-2-yl,
where phenyl, pyridyl and pyrrolyl for their part may be substituted by fluorine, chlorine, bromine, cyano, nitro, trifluoromethyl, methyl, hydroxymethyl, methoxy, dimethylamino or morpholinyl,
and
piperidin-3-yl, piperidin-4-yl and pyrrolidin-2-yl for their part may be substituted by methyl, ethyl, n-propyl, isopropyl, methylcarbonyl or ethylcarbonyl,
NR¹⁰R¹¹
in which
R¹⁰ and R¹¹ independently of one another represent hydrogen, (C₁-C₄)-alkyl, 3-hydroxypropyl, 2-hydroxycyclohexyl, 2-aminocyclohexyl, phenyl, pyridyl or pyrazolyl,
where phenyl and pyridyl for their part may be substituted by chlorine, hydroxyl, amino, cyano, methyl or methoxy,
or
R¹⁰ and R¹¹ together with the nitrogen atom to which they are attached form a piperazine, 3-methylpiperazine, 3,5-dimethylpiperazine, 4-isobutylpiperazine, morpholine, pyrrolidine, 3-aminopyrrolidine, 3-methylaminopyrrolidine, 3-(*N,N-*dimethylamino)pyrrolidine, 2-amino-methylpyrrolidine, 3-hydroxypyrrolidine, 2-hydroxymethylpyrrolidine or 2-methoxymethyl-pyrrolidine ring or a radical 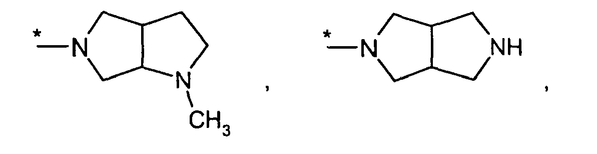 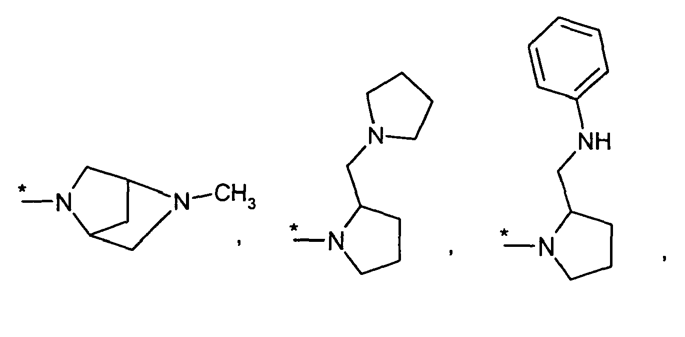 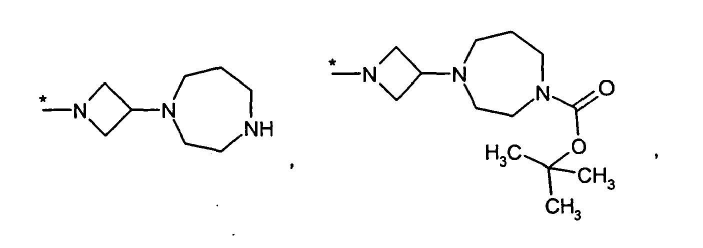 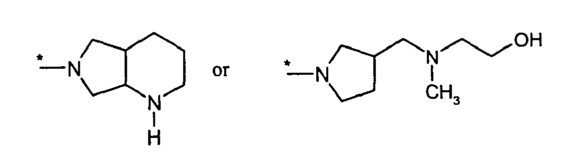
in which
* denotes the point of attachment to the pyrimidine ring,
and C(=O)R¹⁴
in which
R¹⁴ represents methoxy, piperidinyl-N-ethylamino, piperidinyl or piperazinyl,
or a salt, a hydrate, a hydrate of a salt or a solvate thereof.

4. Process for preparing compounds as defined in Claim 1, **characterized in that** either
**[A]** compounds of the formula (II) 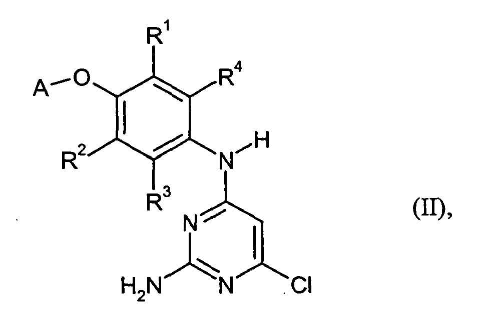 in which
A, R¹, R², R³ and R⁴ are as defined in Claim 1
are reacted with compounds of the formula (III)
R⁵―X¹ (III),
in which
R⁵ is as defined in Claim 1 and
X¹ represents hydrogen, B(OH)₂ or a boronic acid ester such as 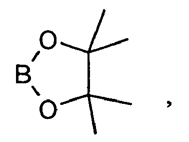
or
[B] compounds of the formula (IV) 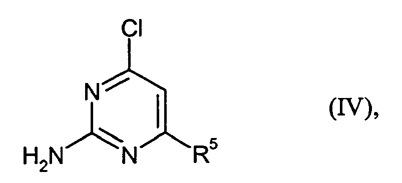 in which
R⁵ is as defined in Claim 1
are reacted with compounds of the formula (V) 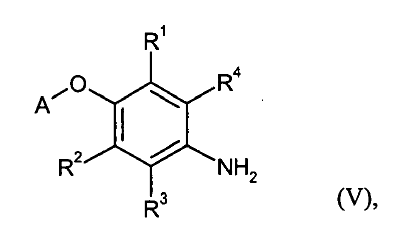 in which
A, R¹, R², R³ and R⁴ are as defined in Claim 1.

5. Compound as defined in any of Claims 1 to 3 for the treatment and/or prophylaxis of disorders.

6. Use of a compound as defined in any of Claims 1 to 3 for preparing medicaments for the treatment and/or prophylaxis of cardiovascular disorders.

7. Use of a compound as defined in any of Claims 1 to 3 for preparing medicaments for the treatment and/or prophylaxis of erectile dysfunction.

8. Medicament, comprising a compound as defined in any of Claims 1 to 3 and a further active compound.

9. Medicament comprising a compound as defined in any of Claims 1 to 3 in combination with an inert nontoxic pharmaceutically acceptable auxiliary.

## Revendications

1. Composés de formule 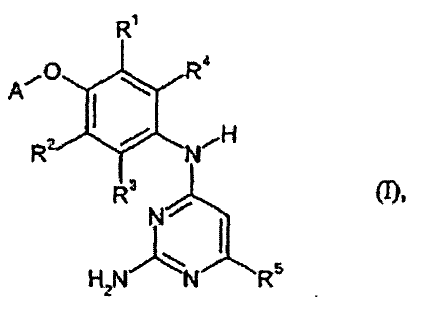 dans laquelle
A désigne un reste 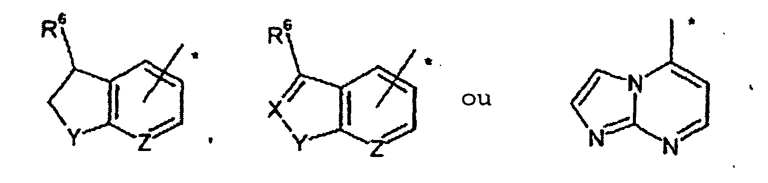 dans lequel
X représente N ou C-H,
Y représente N-R⁷, O ou S,
où
R⁷ représente l'hydrogène, un reste benzyle, phényle, alkyle en C₁ à C₆ ou cycloalkyle en C₃ à C₈,
alkyle et cycloalkyle pouvant eux-mêmes être substitués par un radical fluoro, hydroxy, amino, carboxyle, alkoxy en C₁ à C₆, alkylamino en C₁ à C₆ ou morpholinyle,
Z représente N ou C-H,
R⁶ représente l'hydrogène, un halogène, un reste trifluorométhyle, alkylamino en C₁ à C₆ ou un reste W-R⁷
dans lequel
W représente NH, O ou une liaison,
R⁷ a la définition indiquée ci-dessus
et
* représente le site de liaison à l'oxygène phénolique,
R¹ et R² représentent, indépendamment l'un de l'autre, l'hydrogène, un halogène ou un groupe cyano,
R³ et R⁴ représentent, indépendamment l'un de l'autre, l'hydrogène, le fluor ou le chlore,
R⁵ désigne un reste qui est choisi dans le groupe comprenant :
hydrogène, hydroxy, halogène, trifluorométhyle, cyclo-alkyle en C₃ à C₈, alkyle en C₁ à C₆, alkoxy en C₁ à C₆,
les restes cycloalkyle, alkyle et alkoxy pouvant eux-mêmes être substitués par un radical hydroxy, carboxyle, alkoxy en C₁ à C₆, (alkoxy en C₁ à C₆)-carbonyle, aryle en C₆ à C₁₀, NR⁸R⁹ ou C(=O)NR⁸R⁹, où
R⁸ et R⁹ représentent, indépendamment l'un de l'autre, l'hydrogène, un reste alkyle en C₁ à C₈, un reste cycloalkyle en C₃ à C₆ éventuellement substitué par un radical alkyle en C₁ à C₆, un reste aryle en C₆ à C₁₀ éventuellement substitué par un halogène, ou un reste hétéroaryle pentagonal à décagonal,
ou bien
R⁸ et R⁹ forment, conjointement avec l'atome d'azote auquel ils sont liés, un hétérocycle pentagonal ou hexagonal qui peut contenir un autre hétéroatome O ou N dans le noyau et qui peut être substitué par un reste alkyle en C₁ à C₆, alcanoyle en C₁ à C₆ ou (alkoxy en C₁ à C₆)-carbonyle,
un reste aryle en C₆ à C₁₀, aryloxy en C₆ à C₁₀, hétéroaryle pentagonal à décagonal, hétéroaryloxy pentagonal à décagonal, hétérocyclyle pentagonal à décagonal lié par l'intermédiaire d'un atome de carbone,
les restes aryle, aryloxy, hétéroaryle, hétéroaryloxy et hétérocyclyle pouvant eux-mêmes être substitués par un halogène, un groupe cyano, nitro, carboxyle, amino, un reste trifluoro-méthyle, alkyle en C₁ à C₆ éventuellement substitué par un radical hydroxy, un reste alkoxy en C₁ à C₆, alkylamino en C₁ à C₆, alcanoyle en C₁ à C₆, (alkoxy en C₁ à C₆)-carbonyle, alcanoylamino en C₁ à C₆, (alkoxy en C₁ à C₆)-carbonylamino ou hétérocyclyle pentagonal ou hexagonal,
un reste NR¹⁰R¹¹,
dans lequel
R¹⁰ et R¹¹ représentent, indépendamment l'un de l'autre, l'hydrogène, un reste alkyle en C₁ à C₆, cycloalkyle en C₃ à C₈, aryle en C₆ à C₁₀ ou hétéroaryle pentagonal à décagonal, alkyle et cycloalkyle pouvant eux-mêmes être substitués par un radical hydroxy, alkoxy en C₁ à C₆, aryle en C₆ à C₁₀, hétéroaryle pentagonal à décagonal ou NR¹⁵R¹⁶,
où
R¹⁵ et R¹⁶ représentent, indépendamment l'un et l'autre, l'hydrogène, un reste alkyle en C₁ à C₆, cycloalkyle en C₃ à C₆, aryle en C₆ à C₁₀ ou hétéroaryle pentagonal ou hexagonal,
ou bien
R¹⁵ et R¹⁶ forment, conjointement avec l'atome d'azote auquel ils sont liés, un hétérocycle pentagonal ou hexagonal qui peut contenir un autre hétéroatome O ou N dans le noyau et qui peut être substitué par un reste alkyle en C₁ à C₆, alcanoyle en C₁ à C₆ ou (alkoxy en C₁ à C₆)-carbonyle,
et
aryle et hétéroaryle peuvent eux-mêmes être substitués par un halogène, un groupe hydroxy, amino, cyano, un reste trifluorométhyle, alkyle en C₁ à C₆, alkoxy en C₁ à C₆, alkylamino en C₁ à C₆ ou alcanoylamino en C₁ à C₆,
ou bien
R¹⁰ et R¹¹ forment, conjointement avec l'atome d'azote auquel ils sont liés, un hétérocycle tétragonal à hexagonal qui peut contenir un autre hétéroatome O ou N dans le noyau et qui peut être substitué par du fluor, un groupe hydroxy, carboxyle, un reste hétéro-cyclyle pentagonal à heptagonal qui peut contenir dans le noyau un ou deux autres hétéroatomes N et/ou O et qui peut lui-même être substitué par un reste alkyle en C₁ à C₄ ou (alkoxy en C₁ à C₄)-carbonyle, un reste alkoxy en C₁ à C₄, alkyle en C₁ à C₄ éventuellement substitué par un radical hydroxy, alkoxy en C₁ à C₄ ou NR¹⁷R¹⁸, un reste alcanoyle en C₁ à C₄, (alkoxy en C₁ à C₄) -carbonyle ou NR¹²R¹³,
où
R¹² et R¹³ représentent, indépendamment l'un de l'autre, l'hydrogène, un reste alkyle en C₁ à C₆, (alkoxy en C₁ à C₄)-carbonyle, cycloalkyle en C₃ à C₈ ou alcanoyle en C₁ à C₄,
ou bien
R¹² et R¹³ forment, conjointement avec l'atome d'azote auquel ils sont liés, un hétérocycle pentagonal ou hexagonal qui peut contenir un autre hétéroatome O ou N dans le noyau et qui peut être substitué par un reste alkyle en C₁ à C₆, alcanoyle en C₁ à C₆ ou (alkoxy en C₁ à C₆)-carbonyle,
et
R¹⁷ et R¹⁸ représentent, indépendamment l'un de l'autre, l'hydrogène, un reste alkyle en C₁ à C₆ éventuellement substitué par un radical hydroxy, un reste cyclo-alkyle en C₃ à C₆, aryle en C₆ à C₁₀ ou hétéroaryle pentagonal ou hexagonal,
ou bien
R¹⁷ et R¹⁸ forment, conjointement avec l'atome d'azote auquel ils sont liés, un hétérocycle pentagonal ou hexagonal qui peut contenir un autre hétéroatome O ou N dans le noyau et qui peut être substitué par un reste alkyle en C₁ à C₆, alcanoyle en C₁ à C₆ ou (alkoxy en C₁ à C₆)-carbonyle,
ou bien
R¹⁰ et R¹¹ forment, conjointement avec l'atome d'azote auquel ils sont liés, un hétérocycle bicyclique ou tricyclique heptagonal à dodécagonal, qui est condensé ou spirocyclique et qui peut présenter dans le noyau un ou deux autres hétéroatomes de la série N et/ou O et qui peut être substitué par du fluor, un reste alkyle en C₁ à C₄, (alkoxy en C₁ à C₄)-carbonyle, alcanoyle en C₁ à C₄ ou benzyle,
et un groupe C(=O)R¹⁴,
dans lequel
R¹⁴ représente un reste alkoxy en C₁ à C₆, alkylamino en C₁ à C₆ ou un hétérocycle monocyclique ou bicyclique pentagonal à décagonal lié par l'intermédiaire d'un atome d'azote, qui est condensé ou spirocyclique et qui peut présenter un ou deux autres hétéro-atomes de la série N et/ou O dans le noyau, alkylamino pouvant lui-même être substitué par un reste hétérocyclyle pentagonal ou hexagonal,
et leurs sels, leurs hydrates, les hydrates de leurs sels et leurs produits de solvatation.

2. Composés suivant la revendication 1,
dans lesquels
A représente un reste 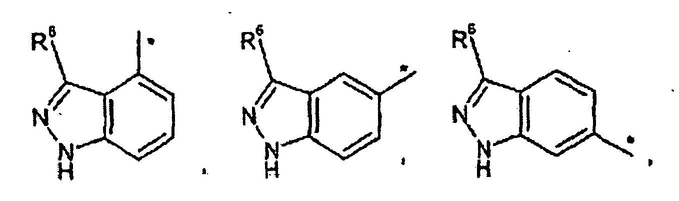 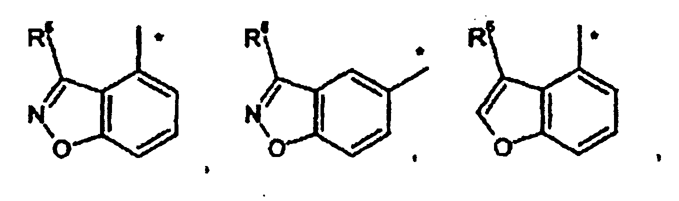 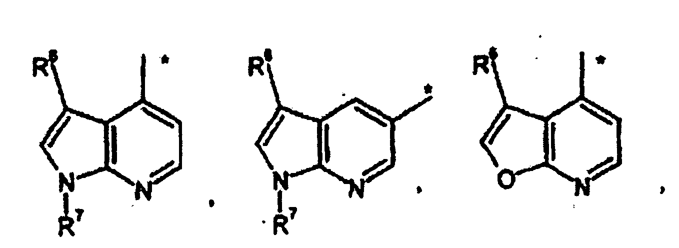 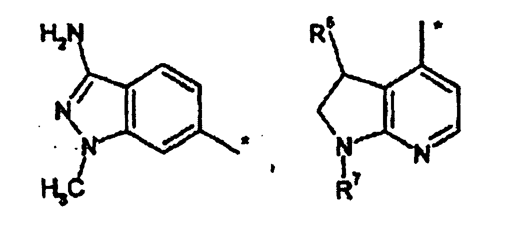 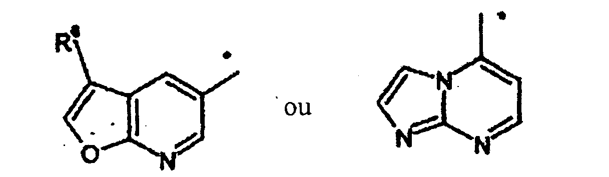 où
R⁶ représente l'hydrogène, un reste alkyle en C₁ à C₄ ou NH-R⁷,
R⁷ représente l'hydrogène ou un reste alkyle en C₁ à C₄,
et
* représente le point de liaison à l'oxygène phénolique,
R¹ et R² représentent, indépendamment l'un de l'autre, l'hydrogène, le fluor ou le chlore,
R³ et R⁴ représentent, indépendamment l'un de l'autre, l'hydrogène ou le fluor,
R⁵ désigne un reste qui est choisi dans le groupe comprenant :
l'hydrogène, le chlore, un reste cycloalkyle en C₃ à C₈, alkyle en C₁ à C₆, alkoxy en C₁ à C₆,
alkyle et alkoxy pouvant eux-mêmes être substitués par un radical hydroxy, carboxyle, alkoxy en C₁ à C₄, (alkoxy en C₁ à C₄)-carbonyle, NR⁸R⁹ ou C(=O)NR⁸R⁹ où
R⁸ et R⁹ représentent, indépendamment l'un de l'autre, l'hydrogène, un reste alkyle en C₁ à C₈, cycloalkyle en C₃ à C₆ éventuellement substitué par un radical alkyle en C₁ à C₄, phényle éventuellement substitué par un halogène ou hétéroaryle pentagonal ou hexagonal
ou bien
R⁸ et R⁹ forment, conjointement avec l'atome d'azote auquel ils sont liés, un noyau morpholine, pipérazine, pipéridine ou pyrrolidine, les noyaux pouvant eux-mêmes être substitués par un reste alkyle en C₁ à C₄,
un reste aryle en C₆ à C₁₀, hétéroaryle pentagonal ou hexagonal, hétérocyclyle pentagonal ou hexagonal lié par l'intermédiaire d'un atome de carbone, aryle, hétéroaryle et hétérocyclyle pouvant eux-mêmes être substitués par un halogène, un groupe cyano, nitro, carboxyle, amino, un reste trifluorométhyle, un reste alkyle en C₁ à C₄ éventuellement substitué par un radical hydroxy, un reste alkoxy en C₁ à C₄, alkylamino en C₁ à C₄, alcanoyle en C₁ à C₄, (alkoxy en C₁ à C₄)-carbonyle, alcanoylamino en C₁ à C₄, (alkoxy en C₁ à C₄)-carbonylamino ou hétérocyclyle hexagonal, un groupe NR¹⁰R¹¹,
dans lequel
R¹⁰ et R¹¹ représentent, indépendamment l'un de l'autre, l'hydrogène, un reste alkyle en C₁ à C₆, cycloalkyle en C₃ à C₈, phényle ou hétéroaryle pentagonal ou hexagonal, alkyle et cycloalkyle pouvant eux-mêmes être substitués par un radical hydroxy, alkoxy en C₁ à C₄, phényle, hétéroaryle pentagonal ou hexagonal ou NR¹⁵R¹⁶,
où
R¹⁵ et R¹⁶ représentent, indépendamment l'un de l'autre, l'hydrogène, un reste alkyle en C₁ à C₄, cycloalkyle en C₃ à C₆, phényle ou hétéroaryle pentagonal ou hexagonal,
ou bien
R¹⁵ et R¹⁶ forment, conjointement avec l'atome d'azote auquel ils sont liés, un noyau morpholine, pipérazine, pipéridine ou pyrrolidine, les noyaux pouvant eux-mêmes être substitués par un reste alkyle en C₁ à C₄
et
phényle et hétéroaryle peuvent eux-mêmes être substitués par du fluor, du chlore, un groupe hydroxy, amino, cyano, un reste trifluorométhyle, alkyle en C₁ à C₄, alkoxy en C₁ à C₄, alkylamino en C₁ à C₄ ou alcanoylamino en C₁ à C₄,
ou bien
R¹⁰ et R¹¹ forment, conjointement avec l'atome d'azote auquel ils sont liés, un hétérocycle tétragonal à hexagonal qui peut contenir un autre hétéro-atome O ou N dans le noyau et qui peut être substitué par du fluor, un groupe hydroxy, carboxyle, un reste hétérocyclyle pentagonal à heptagonal, pouvant contenir dans le noyau un ou deux autres hétéro-atomes N et/ou O, qui peut lui-même être substitué par un reste alkyle en C₁ à C₄ ou (alkoxy en C₁ à C₄)-carbonyle, un reste alkoxy en C₁ à C₄, un reste alkyle en C₁ à C₄ éventuellement substitué par un radical hydroxy, alkoxy en C₁ à C₄ ou NR¹⁷R¹⁸, un reste alcanoyle en C₁ à C₄, (alkoxy en C₁ à C₄)-carbonyle ou NR¹²R¹³,
où
R¹² et R¹³ représentent, indépendamment l'un de l'autre, l'hydrogène ou un reste alkyle en C₁ à C₄,
ou bien
R¹² et R¹³ forment, conjointement avec l'atome d'azote auquel ils sont liés, un hétérocycle pentagonal ou hexagonal qui peut contenir un autre hétéro-atome O ou N dans le noyau et qui peut être substitué par un radical alkyle en C₁ à C₆, alcanoyle en C₁ à C₆ ou (alkoxy en C₁ à C₆)-carbonyle,
et
R¹⁷ et R¹⁸ représentent, indépendamment l'un de l'autre, l'hydrogène, un reste alkyle en C₁ à C₄, éventuellement substitué par un radical hydroxy ou un reste phényle,
ou bien
R¹⁷ et R¹⁸ forment, conjointement avec l'atome d'azote auquel ils sont liés, un noyau pyrrolidine,
ou bien
R¹⁰ et R¹¹ forment, conjointement avec l'atome d'azote auquel ils sont liés, un hétérocycle bicyclique ou tricyclique heptagonal à dodécagonal, qui est condensé ou spiro-cyclique et qui peut présenter un ou deux autres hétéro-atomes de la série N et/ou O dans le noyau et qui peut être substitué par un radical alkyle en C₁ à C₄, (alkoxy en C₁ à C₄)-carbonyle, alcanoyle en C₁ à C₄ ou benzyle,
et un groupe C(=O)R¹⁴,
dans lequel
R¹⁴ représente un reste alkoxy en C₁ à C₆, alkylamino en C₁ à C₆ ou un hétérocycle monocyclique ou bicyclique pentagonal à décagonal lié par l'intermédiaire d'un atome d'azote, qui est condensé ou spirocyclique et qui peut présenter un ou deux autres hétéro-atomes de la série N et/ou O dans le noyau,
alkylamino pouvant lui-même être substitué par un reste hétérocyclyle pentagonal ou hexagonal,
et leurs sels, leurs hydrates, les hydrates de leurs sels et leurs produits de solvatation.

3. Composés suivant l'une des revendications 1 ou 2, dans lesquels
A représente un reste 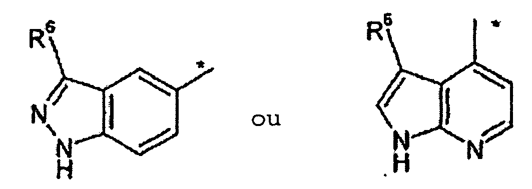 où
R⁶ représente l'hydrogène ou le groupe méthyle,
et
* représente le site de liaison à l'oxygène phénolique,
R¹ et R² représentent, indépendamment l'un de l'autre, l'hydrogène, le fluor ou le chlore,
R³ et R⁴ représentent l'hydrogène,
R⁵ désigne un reste qui est choisi dans le groupe comprenant :
l'hydrogène, le chlore, un reste cyclohexyle, alkyle en C₁ à C₄, alkoxy en C₁ à C₄,
alkyle et alkoxy pouvant eux-mêmes être substitués par un radical hydroxy, carboxyle, alkoxy en C₁ à C₄, méthyloxycarbonyle, éthyloxy-carbonyle, NR⁸R⁹ ou C(=O)NR⁸R⁹
où
R⁸ et R⁹ représentent, indépendamment l'un de l'autre, l'hydrogène, un reste alkyle en C₁ à C₈, cyclopropyle, cyclopentyle éventuellement substitué par un radical méthyle, ou phényle éventuellement substitué par du fluor,
ou bien
R⁸ et R⁹ forment, conjointement avec l'atome d'azote auquel ils sont liés, un noyau pipéridine, 2-méthylpipéridine ou 2,6-diméthylpipéridine,
un reste phényle, pyridyle, pyrrolyle, pipéridine-3-yle, pipéridine-4-yle, pyrrolidine-2-yle,
phényle, pyridyle et pyrrolyle pouvant eux-mêmes être substitués par du fluor, du chlore, du brome, un groupe cyano, nitro, un reste tri-fluorométhyle, méthyle, hydroxyméthyle, méthoxy, diméthylamino ou morpholinyle, et
pipéridine-3-yle, pipéridine-4-yle et pyrrolidine-2-yle pouvant eux-mêmes être substitués par un radical méthyle, éthyle, n-propyle, isopropyle, méthylcarbonyle ou éthylcarbonyle,
un groupe NR¹⁰R¹¹
dans lequel
R¹⁰ et R¹¹ représentent, indépendamment l'un de l'autre, l'hydrogène, un reste alkyle en C₁ à C₄, 3-hydroxypropyle, 2-hydroxy-cyclohexyle, 2-aminocyclohexyle, phényle, pyridyle ou pyrazolyle, phényle et pyridyle pouvant être eux-mêmes être substitués par du chlore, un groupe hydroxy, amino, cyano, un reste méthyle ou méthoxy,
ou bien
R¹⁰ et R¹¹ forment, conjointement avec l'atome d'azote auquel ils sont liés, un noyau pipérazine, 3-méthylpipérazine, 3,5-diméthylpipérazine, 4-isobutylpipérazine, morpholine, pyrrolidine, 3-aminopyrrolidine, 3-méthylaminopyrrolidine, 3-(N,N-diméthyl-amino)-pyrrolidine, 2-aminométhylpyrrolidine, 3-hydroxypyrrolidine, 2-hydroxyméthyl-pyrrolidine ou 2-méthoxyméthylpyrrolidine ou un reste 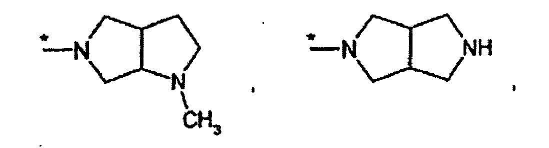 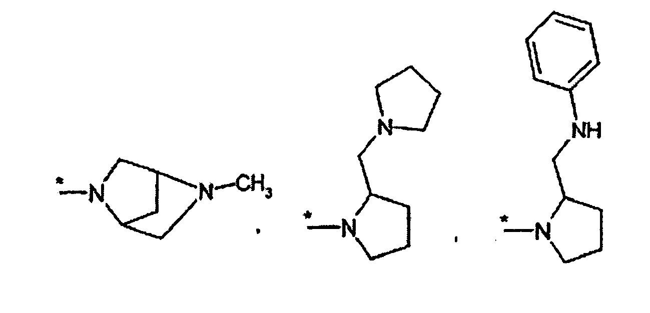 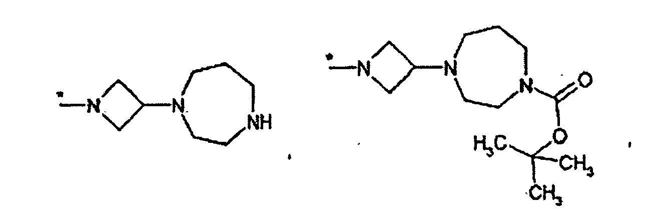 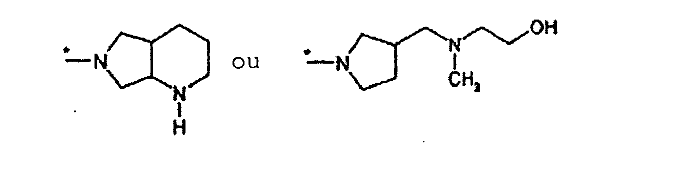
où
* représente le site de liaison au noyau pyrimidine
et un groupe C(=O)R¹⁴,
dans lequel
R¹⁴ est un reste méthoxy, pipéridinyl-N-éthylamino, pipéridinyle ou pipérazinyle
et leurs sels, leurs hydrates, les hydrates de leurs sels et leurs produits de solvatation.

4. Procédé de production de composés tels que définis dans la revendication 1,
**caractérisé en ce que**
[A] on fait réagir des composés de formule (II) 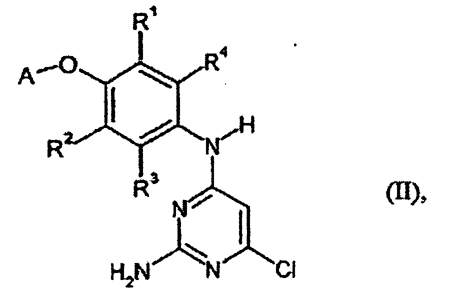 dans laquelle
A, R¹, R², R³ et R⁴ ont la définition indiquée dans la revendication 1
avec des composés de formule (III)
R⁵―X¹ (III),
dans laquelle
R⁵ a la définition indiquée dans la revendication 1 et
X¹ représente l'hydrogène, un groupe B(OH)₂ ou un ester d'acide boronique tel que 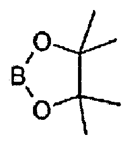
ou bien
[B] on fait réagir des composés de formule (IV) 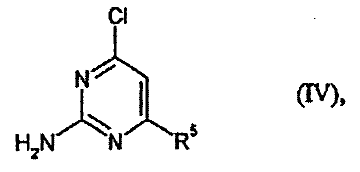 dans laquelle
R⁵ a la définition indiquée dans la revendication 1,
avec des composés de formule (V) 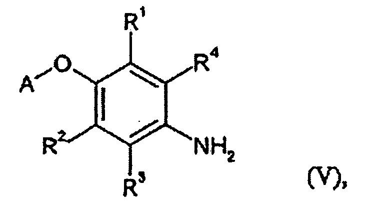 dans laquelle
A, R¹, R², R³ et R⁴ ont la définition indiquée dans la revendication 1.

5. Composé tel que défini dans l'une des revendications 1 à 3, destiné au traitement et/ou à la prophylaxie de maladies.

6. Utilisation d'un composé, tel que défini dans l'une des revendications 1 à 3, pour la préparation de médicaments destinés au traitement et/ou à la prophylaxie de maladies cardio-vasculaires.

7. Utilisation d'un composé, tel que défini dans l'une des revendications 1 à 3, pour la préparation de médicaments destinés au traitement et/ou à la prophylaxie d'un dysfonctionnement érectile.

8. Médicament contenant un composé, tel que défini dans l'une des revendications 1 à 3, et une autre substance active.

9. Médicament contenant un composé, tel que défini dans l'une des revendications 1 à 3, en combinaison avec une substance auxiliaire inerte non toxique pharmaceutiquement acceptable.
